# EUROPEAN PATENT SPECIFICATION

(11) **EP 3 668 600 B1**
(45) Date of publication and mention of the grant of the patent: **15.05.2024**
(21) Application number: 18773247.4
(22) Date of filing: 10.08.2018
(51) Int. Cl.: A61P 35/00, A61K 38/17, C07K 14/775, A61K 47/54

(54) **CARGOMERS**
CARGOMERE
CARGOMÈRES

(30) Priority: 10.08.2017 US 201762543470 P; 07.11.2017 US 201762582930 P; 07.11.2017 US 201762582924 P; 13.02.2018 US 201862630210 P
(43) Date of publication of application: 24.06.2020
(73) Proprietor: Abionyx Pharma SA, 31130 Balma (FR)
(72) Inventor: DASSEUX, Jean-Louis, 31300 Toulouse (FR)
(74) Representative: Hoffmann Eitle
(86) International application number: PCT/IB2018/001043
(87) International publication number: WO 2019/030574

(56) References cited:
- WO-A1-2006/100567
- WO-A1-2009/158678
- WO-A1-2012/109162
- WO-A1-2015/023797
- JUAN C. FRIAS ET AL: "Recombinant HDL-Like Nanoparticles:? A Specific Contrast Agent for MRI of Atherosclerotic Plaques", JOURNAL OF THE AMERICAN CHEMICAL SOCIETY, vol. 126, no. 50, 1 December 2004 (2004-12-01), pages 16316-16317, XP055163160, ISSN: 0002-7863, DOI: 10.1021/ja044911a
- CORBIN I R ET AL: "Enhanced Cancer-Targeted Delivery Using Engineered High-Density Lipoprotein-Based Nanocarriers", JOURNAL OF BIOMEDICAL NANOTECHNO, AMERICAN SCIENTIFIC PUBLISHERS, US, vol. 3, no. 4, 1 December 2007 (2007-12-01), pages 367-376, XP008137630, ISSN: 1550-7033
- KAYLIN M. MCMAHON ET AL: "Biomimetic High Density Lipoprotein Nanoparticles For Nucleic Acid Delivery", NANO LETTERS, vol. 11, no. 3, 9 March 2011 (2011-03-09), pages 1208-1214, XP055522773, US ISSN: 1530-6984, DOI: 10.1021/nl1041947
- MENGJIE RUI ET AL: "Simultaneous delivery of anti-miR21 with doxorubicin prodrug by mimetic lipoprotein nanoparticles for synergistic effect against drug resistance in cancer cells", INTERNATIONAL JOURNAL OF NANOMEDICINE, vol. Volume 12, 30 December 2016 (2016-12-30), pages 217-237, XP055522783, DOI: 10.2147/IJN.S122171

## Description

### 1. CROSS-REFERENCE TO RELATED APPLICATIONS

This application claims the priority benefit of U.S. provisional application no. 62/543,470 filed August 10, 2017, U.S. provisional application no. 62/582,924, filed November 7, 2017, U.S. provisional application no. 62/582,930, filed November 7, 2017, and U.S. provisional application no. 62/630,210, filed February 13, 2018.

### 2. SEQUENCE LISTING

The instant application contains a Sequence Listing which has been submitted electronically in ASCII format. Said ASCII copy, created on August 3, 2018 is named CRN-020WO_ST25.txt and is 10,848 bytes in size.

### 3. BACKGROUND

Drug delivery vehicles are engineered technologies for the targeted delivery and/or controlled release of therapeutic agents. Drug delivery vehicles are also useful for slowing degradation of a therapeutic agent. Various drug delivery vehicles have been described, including liposomal delivery vehicles (see, *e.g.,* Sercombe et al., 2015 Front Pharmacol. 6:286), high density lipoprotein (HDL) delivery vehicles (see, *e.g.,* Lacko et al., 2015 Front Pharmacol. 6:247), and albumin-based delivery vehicles (see, *e.g.,* Larsen et al., 2016, Mol Cell Ther. 4:3).

Liposomes are phospholipid vesicles made of one or more concentric lipid bilayers enclosing discrete aqueous spaces. Liposomes have been used as delivery vehicles for both hydrophobic molecules, which can be inserted into the lipid bilayer membrane, and hydrophilic molecules, which can be enclosed in a liposome's aqueous core. Liposomes have historically been viewed as minimally toxic, but it has been found more recently that liposomes can trigger innate immune responses, including C activation-related pseudoallergy (CARPA) (Szebeni, 2005, Toxicology 216:106-121; Szebeni and Moghimi, 2009, J Liposome Res.19(2):85-90). Many symptoms of CARPA are the same as seen in common allergic reactions, while other are unique to CARPA, for example a reaction arising from the first exposure to an allergen. Marketed liposomal drugs have been reported to cause hypersensitivity reactions (HSRs) with symptoms consistent with CARPA. The frequency of HSRs to liposomal drugs has been reported to vary between 3% and 45% (Szebeni, 2005, Toxicology 216:106-121).

HDL is one of four major classes of lipoprotein particles that are involved in the fat-transport system. Lipoprotein particles have a hydrophobic core comprised of cholesterol (cholesterol in the form of cholesterol and esterified cholesterol) and triglycerides. The core is surrounded by a surface coat comprising phospholipids, unesterified cholesterol and apolipoproteins. HDL particles usually comprises at least 1 molecule, and usually two to four molecules, of apolipoprotein A-I (ApoA-I). HDL mediates reverse cholesterol transport (RCT), the removal of cholesterol lipids, in particular from extrahepatic tissues to the liver, where it is stored, catabolized, eliminated or recycled. HDL also plays a role in the reverse transport of other lipids and apolar molecules, and in detoxification, *i.e*., the transport of lipids from cells, organs, and tissues to the liver for catabolism and excretion. The use of HDL as a drug delivery particle was suggested over 30 years ago (Counsell and Pohland, 1982, J Med Chem. 25(10):1115-20). Since that time, HDL has been studied pre-clinically as a carrier for anti-cancer drugs (see, *e*.*g*., McConathy et al., 2008, Anticancer Drugs. 19(2):183-8), cardiovascular drugs (see, *e.g*., Zhang et al., 2012 Pharmazie. 67(4):324-30), and nucleic acids (see, *e.g.,* Yang *et al.,* 2011, 7(5):568-73). However, there are not yet any marketed therapeutics which make use of HDL as a delivery vehicle.

Albumin is the most abundant plasma protein in human blood. Albumin naturally transports several different types of ligands, including endogenous ligands such as fatty acids and steroids, as well as exogenous ligands such as warfarin, penicillin and diazepam (Larsen et al., 2016, Mol Cell Ther. 4:3). The ability of albumin to bind fatty acids has been used to develop fatty acid modified drugs which bind to albumin upon administration and dissociate over time. For example, Levemir^{®} (Insulin detemir), marketed by Novo Nordisk for the treatment of diabetes, is a myristic acid modified insulin analog. Novo Nordisk also markets Victoza^{®} (liraglutide), which comprises a palmitic acid modified glucagon-like peptide-1 agonist, also for the treatment of diabetes. Abraxane^{®}, marketed by Celgene for the treatment of various cancers, contains paclitaxel within an albumin-based nanoparticle. The outer layer of the nanoparticle consists of albumin, while the inner core contains paclitaxel. Albumin based diagnostic nanoparticles have also been developed. Nanocoll^{®} and Albures^{®} are aggregated albumin particles that can be used to deliver technetium 99m, a metastable nuclear isomer, for diagnostic imaging procedures. Although albumin has proven to be useful for delivering some therapeutic and diagnostic agents in the clinic, its cargo carrying capacity is limited because it has a limited apolar pocket in which to carry cargo molecules. Albumin based nanoparticles can also have a large size due to multiple aggregated molecules of albumin. For example, Abraxane^{®} has a mean particle size of approximately 130 nanometers (www.rxlist.com/abraxane-drug.htm). Other nanolipoparticules have limiations in terms of carrying capacity, biocompatibility, safety, cost of manufacturing or targeting ability (See, Wilhelm et al., 2016, Nature Reviews Materials 1:16014).

Cargomer-based delivery complexes with apolipoprotein, cargo moieties and an amount of amphipathic molecules such as lipids have also been disclosed in the art (cf. WO 2012/109162 A1).

Thus, there is a need for new drug delivery vehicles, for example delivery vehicles with greater cargo carrying capacity, smaller size, and/or better targeting ability.

### 4. SUMMARY

This disclosure relates to delivery complexes comprising an apolipoprotein in monomeric or multimeric form and one or more cargo moieties. The cargo moieties can be amphipathic or non-amphipathic (*e*.*g*., apolar). Amphipathic cargo moieties can facilitate the solubilization of the apolipoprotein and prevent it from aggregating. Where the cargo moieties are not amphipathic or insufficient to solubilize the apolipoprotein molecule(s), the delivery complexes can also comprise one or more additional amphipathic molecules to solubilize the apolipoprotein. Thus, reference to amphipathic molecules in the context of the delivery complexes of the disclosure encompasses amphipathic molecules that are cargo moieties, amphipathic molecules that are not cargo moieties, or some combination thereof.

Accordingly, the present invention relates to a Cargomer comprising:
(a) 1-8 apolipoprotein molecules;
(b) one or more cargo moieties;
(c) an amount of amphipathic molecules sufficient to solubilize the apolipoprotein molecules, wherein one or more of the cargo moieties of (b) and one or more of the amphipathic molecules of (c) can be be the same molecule(s) in the Cargomer;
(d) optionally, one or more anchors non-covalently coupling one or more cargo moieties to the apolipoprotein molecules;

and (e) optionally, one or more linkers covalently coupling one or more cargo moieties to one or more apolipoprotein molecules, one or more amphipathic molecules or one or more anchors,
wherein (i) the amphipathic molecules, the cargo moieties and, if present, the anchors and/or linkers together contribute a net charge of at least +1 or -1 per apolipoprotein molecule in the Cargomer, and (ii), the apolipoprotein to amphipathic molecule molar ratio ranges from 8:1 to 1:15.

Other embodiments of the present invention are as defined in the appended claims.

The delivery complexes of the disclosure are referred to herein as "Cargomers". Following administration to a subject, Cargomers can interact with, and/or deliver the cargo moieties to, a tissue or organ in the subject. The cargo moieties can be the amphipathic molecules used to solubilize the apolipoprotein and/or other biologically active or diagnostically useful molecules or agents.

Cargomers are believed to provide several advantages over other delivery vehicles. Once the cargo is delivered *in vivo,* the remaining apolipoprotein can be integrated into the natural lipoprotein metabolism pathways, thereby avoiding accumulation of an empty carrier. Because the Cargomers of the disclosure contain a relatively small amount of amphipathic molecules relative to apolipoprotein, it is believed that the Cargomers of the disclosure can avoid toxicity problems such as, but not limited to, liver toxicity and C activation-related pseudoallergy (CARPA), that can potentially result from lipid based carriers such as liposomes. Without being bound by theory, it is believed that another advantage over other carriers is the flexibility of apolipoproteins to adapt to different complex sizes. Unlike the cavity of albumin, which due to its small size restrains the cargo to only few molecules, Cargomers offer a wide range of cargo carrying capacity.

Generally, Cargomers comprise one or more apolipoprotein molecules, each complexed with one or more amphipathic molecules. Cargomers also include cargo moieties that are biologically active and/or diagnostically useful following administration to a subject. The cargo moieties can be the same as the amphipathic molecules (in which case there might not be a need for separate amphipathic molecules to solubilize the apolipoprotein molecules).

Cargo moieties are biologically active molecules (*e*.*g*., drugs, biologics (for example, composed of peptides, proteins, nucleic acids, sugars, lipids, modified forms of the foregoing such as peptide nucleic acids (PNAs), or a combination of such components), immunogens, adjuvants, etc.) or other agents, for example agents used in diagnostics. As used herein, the terms "molecule" and "agent" also include complexes and conjugates (for example, antibody-drug conjugates). The terms "biologically active," "diagnostically useful" and the like are not limited to substances with direct pharmacological or biological activity, and may include substances that become active following administration, for example due to metabolism of a prodrug or cleavage of a linker. According, the terms "biologically active" and "diagnostically useful" also includes substances that become biologically active or diagnostically useful after administration, through creation or metabolites or other cleavage products that exert a pharmacological or a biological effect and/or are detectable in a diagnostic test.

Both monomeric and multimeric apolipoproteins can be used to form Cargomers, thereby making it possible to make delivery vehicles with increasing cargo carrying capacity as the number of apolipoprotein molecules increases. Cargomers comprising multimeric apolipoproteins are believed to have a cargo carrying capacity that is higher than albumin, which has a limited apolar pocket to carry cargo molecules. Cargomers also contain a relatively low number of amphipathic molecules compared to discoidal or spherical HDL, which can provide lower costs of production and a simpler manufacturing process. It is also believed that, due to their reduced size as compared to HDL-based delivery vehicles, Cargomers can penetrate the blood brain barrier and/or be taken up into the lymph, providing yet another advantage over larger drug delivery vehicles.

Optionally, Cargomers can comprise one or more anchor moieties (directly or indirectly) coupling the cargo moieties to an apolar molecule, the amphipathic molecule(s) and/or to the apolipoprotein molecule(s). The anchor moieties are typically amphipathic. Amphipathic molecules useful as anchor moieties can have a positive net charge, a negative net charge, or a net charge of zero. The use of a charged amphipathic anchor moiety may circumvent or reduce the need for additional amphipathic molecules to solubilize the apolipoprotein molecule(s). Thus, in certain embodiments, the anchor moieties and the amphipathic molecules in the Cargomers are the same.

Cargomers optionally can also comprise, in addition to or in lieu of anchor moieties, one or more linker moieties. Linker moieties covalently attach one or more cargo moieties (*e*.*g*., any of the cargo moieties described in Section 6.1.3 to one or more apolipoprotein molecules, one or more amphipathic molecules, and/or one or more anchor moieties.

The amphipathic molecules in the Cargomers serve at least the role of solubilizing the apolipoprotein and/or reducing or minimizing apolipoprotein aggregation, but can also have other functions in the Cargomer. For example, as discussed in Section 6.1.3 below, amphiphathic molecules can have therapeutic utility, and thus may be the cargo moieties intended for delivery by the Cargomer upon administration to a subject. Additionally, as discussed in Section 6.1.4 below, amphipathic molecules can be used to anchor a non-amphipathic cargo moiety to the apolipoprotein in the Cargomer. Thus, in some embodiments, the cargo moiety and the amphipathic molecule in a Cargomer are the same. In other embodiments, the anchor moiety and the amphipathic molecule in a Cargomer are the same. In yet other embodiments, the cargo moiety, the anchor moiety and the amphipathic molecule in a Cargomer are the same (for example, where the amphipathic molecule has therapeutic activity and also anchors another biologically active molecule to the apolipoprotein molecule(s)). Binding of the amphipathic molecules to the apolipoprotein is typically non-covalent but the covalent binding of amphipathic molecles to the apolipoprotein is also contemplated.

Anchor and/or linker moieties are particularly useful for Cargomers in which the cargo moiety is not an amphipathic molecule.

In certain aspects, the amphipathic molecules, cargo moieties, and optional anchors and linkers together contribute a net charge of at least +1 or -1 per apolipoprotein molecule in a Cargomer. Exemplary apolipoproteins that can be used in the Cargomers of the disclosure are described in Section 6.1.1. Exemplary amphipathic molecules are described in Section 6.1.2. Exemplary cargo moieties are described in Section 6.1.3. Exemplary anchors are described in Section 6.1.4. Exemplary linkers are described in Secion 6.1.5.

The disclosure further provides compositions comprising a Cargomer of the disclosure, including pharmaceutical compositions, vaccine compositions, and diagnostic compositions. Exemplary compositions are described in Section 6.2.

The disclosure further provides the Cargomers for use in methods of treating a subject that comprise administering a therapeutically effective amount of a Cargomer or a pharmaceutical composition of the disclosure to the subject.

The disclosure further provides the Cargomers for use in methods for diagnosing a subject comprising administering an effective amount of a Cargomer or diagnostic composition of the disclosure to the subject.

The disclosure further provides methods of using the Cargomers or diagnostic compositions of the disclosure to select a treatment for a subject (or to select subjects to be included in a clinical trial) and/or monitor treatment efficacy.

The disclosure further provides the Cargomers for use in methods of immunizing a subject comprising administering an effective amount of a Cargomer or vaccine composition of the disclosure to the subject.

The disclosure further provides the Cargomers for use in methods of desensitizing a subject to an antigen or inducing tolerance to an antigen comprising administering an effective amount of a Cargomer or vaccine composition of the disclosure to the subject.

Exemplary methods providing the Cargomers for use in: treating a subject, methods of diagnosing a subject, methods of immunizing a subject, and methods of desensitizing a subject to an antigen or inducing tolerance to an antigen are described in Section 6.3.

### 5. BRIEF DESCRIPTION OF THE FIGURES

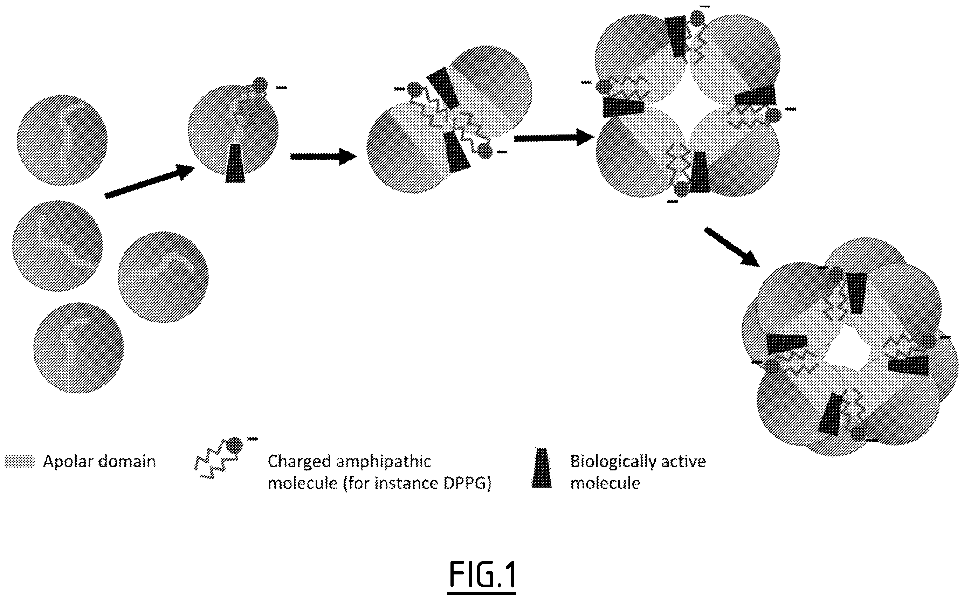

**FIG. 1** schematically shows a process by which the inventor believes Cargomers comprising 8 apolipoprotein molecules, negatively charged amphipathic molecules, and biologically active molecules as cargo moieties (*e*.*g*., as described in Section 6.1.3) that are not covalently attached to the amphipathic molecules are formed. Without being bound by theory, it is believed that Cargomers having 1 apolipoprotein molecule are formed first from apolipoprotein monomers, amphipathic molecules, and cargo moieties. Cargomers with 2 apolipoprotein molecules (*i*.*e*., Cargomers with apolipoprotein dimers) are then formed by dimerization of Cargomers having 1 apolipoprotein molecule, and Cargomers having 4 apolipoprotein molecules (*i*.*e*., Cargomers with apolipoprotein tetramers) are then formed by dimerization of Cargomers with 2 apolipoprotein molecules. Finally, Cargomers with 8 lipoprotein molecules (*i*.*e*., Cargomers with apolipoprotein octamers) are formed by dimerization of the Cargomers having 4 apolipoprotein molecules. Although the arrows shown in Fig. 1 are depicted in one direction, the formation of Cargomers are believed to result in the formation of different species that are present in an equilibrium. The equilibrium can be influenced by conditions such as pH, concentration, ionic strength, and temperature, *e*.*g*., as described in Section 6.1. It should be understood that the numbers of the component molecules shown in Fig. 1 are merely illustrative and that Cargomers having different ratios of component molecules are contemplated, and that Cargomers can be formed directly starting from an apolipoprotein in a solution, in a suspension, in a precipitate, or an aggregate.

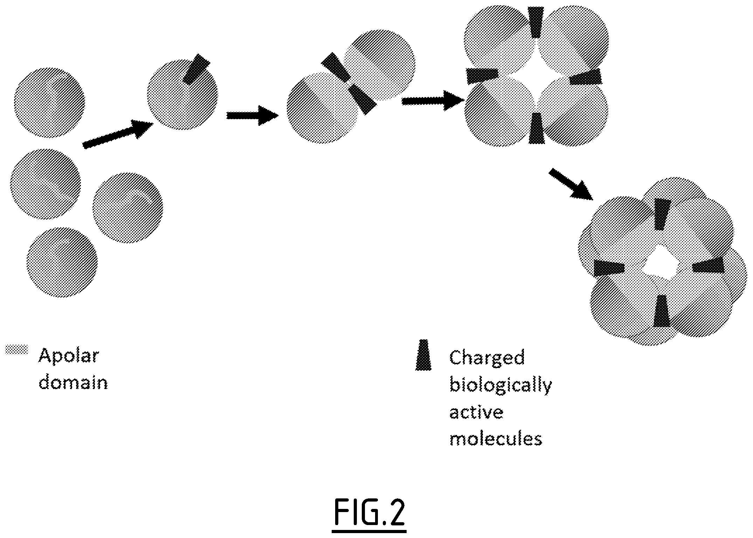

**FIG. 2** schematically shows a process by which the inventor believes Cargomers comprising 8 apolipoprotein molecules and charged, biologically active molecules that function as both the amphipathic molecules and cargo moieties are formed. Without being bound by theory, it is believed that Cargomers having 1 apolipoprotein molecule are formed first from apolipoprotein monomers and the charged biologically active molecules. Cargomers with 2 apolipoprotein molecules (*i*.*e*., Cargomers with apolipoprotein dimers) are then formed by dimerization of Cargomers having 1 apolipoprotein molecule, and Cargomers having 4 apolipoprotein molecules (*i*.*e*., Cargomers with apolipoprotein tetramers) are then formed by dimerization of Cargomers with 2 apolipoprotein molecules. Finally, Cargomers with 8 lipoprotein molecules (*i*.*e*., Cargomers with apolipoprotein octamers) are formed by dimerization of the Cargomers having 4 apolipoprotein molecules. Although the arrows shown in Fig. 2 are depicted in one direction, the formation of Cargomers is believed to result in the formation of different species that are present in an equilibrium. The equilibrium can be influenced by conditions such as pH, concentration, ionic strength, and temperature, *e*.*g*., as described in Section 6.1. It should be understood that the numbers of the component molecules shown in Fig. 2 are merely illustrative and that Cargomers having different ratios of component molecules are contemplated.

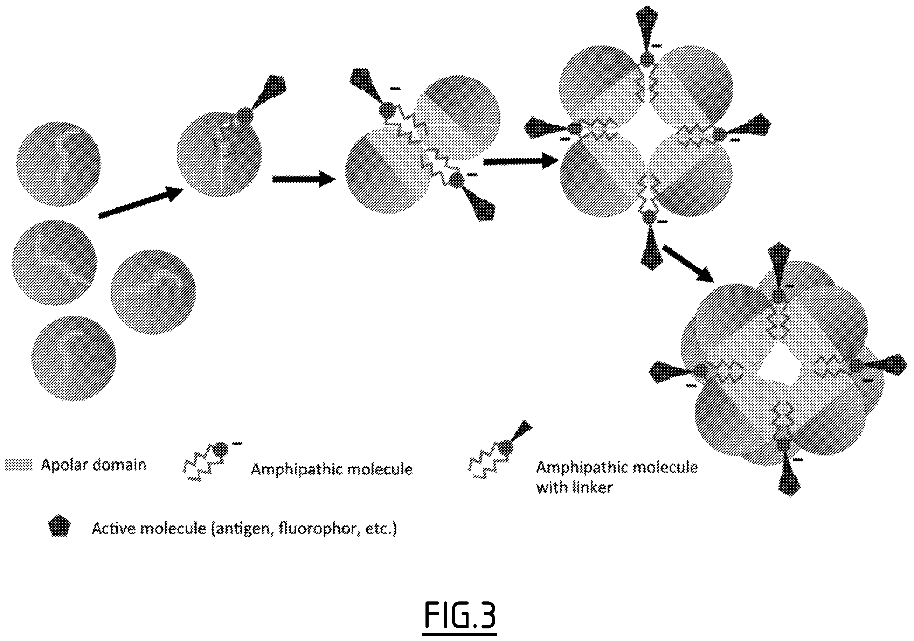

**FIG. 3** schematically shows a process by which the inventor believes Cargomers comprising 8 apolipoprotein molecules and amphipathic molecules having cargo moieties covalently coupled thereto via a linker are formed. Without being bound by theory, it is believed that Cargomers having 1 apolipoprotein molecule are formed first from apolipoprotein monomers and the amphipathic molecules having cargo moieties covalently linked thereto. Cargomers with 2 apolipoprotein molecules (*i*.*e*., Cargomers with apolipoprotein dimers) are then formed by dimerization of Cargomers having 1 apolipoprotein molecule, and Cargomers having 4 apolipoprotein molecules (*i*.*e*., Cargomers with apolipoprotein tetramers) are then formed by dimerization of Cargomers with 2 apolipoprotein molecules. Finally, Cargomers with 8 lipoprotein molecules (*i*.*e*., Cargomers with apolipoprotein octamers) are formed by dimerization of the Cargomers having 4 apolipoprotein molecules. Although the arrows shown in Fig. 3 are depicted in one direction, the formation of Cargomers is believed to result in the formation of different species that are present in an equilibrium. The equilibrium can be influenced by conditions such as pH, concentration, ionic strength, and temperature, *e*.*g*., as described in Section 6.1. It should be understood that the numbers of the component molecules shown in Fig. 3 are merely illustrative and that Cargomers having different ratios of component molecules are contemplated.

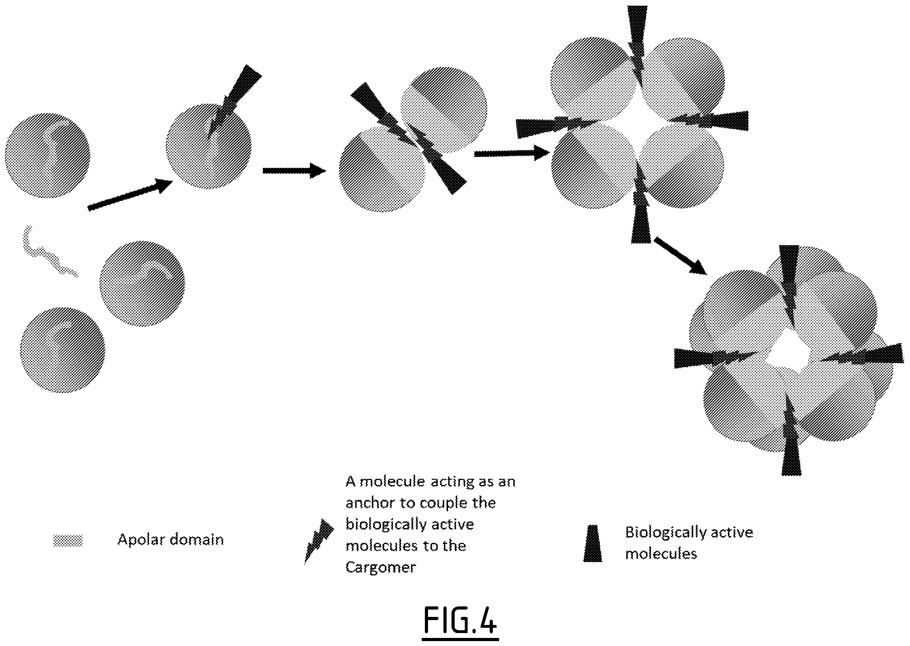

**FIG. 4** schematically shows a process by which the inventor believes Cargomers comprising 8 apolipoprotein molecules and anchor molecules having cargo moieties directly covalently coupled thereto are formed. Without being bound by theory, it is believed that Cargomers having 1 apolipoprotein molecule are formed first from apolipoprotein monomers and the amphipathic molecules having cargo moieties covalently attached thereto. Cargomers with 2 apolipoprotein molecules (*i*.*e*., Cargomers with apolipoprotein dimers) are then formed by dimerization of Cargomers having 1 apolipoprotein molecule, and Cargomers having 4 apolipoprotein molecules (*i*.*e*., Cargomers with apolipoprotein tetramers) are then formed by dimerization of Cargomers with 2 apolipoprotein molecules. Finally, Cargomers with 8 lipoprotein molecules (*i*.*e*., Cargomers with apolipoprotein octamers) are formed by dimerization of the Cargomers having 4 apolipoprotein molecules. Although the arrows shown in Fig. 4 are depicted in one direction, the formation of Cargomers is believed to result in the formation of different species that are present in an equilibrium. The equilibrium can be influenced by conditions such as pH, concentration, ionic strength, and temperature, *e*.*g*., as described in Section 6.1. It should be understood that the numbers of the component molecules shown in Fig. 4 are merely illustrative and that Cargomers having different ratios of component molecules are contemplated.
**FIG. 5** shows the scheme of the study described in Example 2.
FIGS. 6A-C show B16F10 tumor volumes in C57BL/6 mice from tumor induction to study completion for the 8 treatment groups described in Example 2. Fig. 6A: excludes data for animals which did not survive to study completion; Fig. 6B: includes the last measurement for animals which did not survive to study completion; Fig. 6C: includes data for each group until death of first animal in group.
**FIGS. 7A-7B** show bodyweights (Fig. 7A) and baseline corrected bodyweights (Fig. 7B) for the animals in the 8 treatment groups described in Example 2.
**FIGS. 8A-8J** shows individual tumour growth curves with fraction of complete tumour regression (CR) for the 8 treatment groups described in Example 2 (Fig. 8A: vehicle; Fig. 8B ApoA-I; Fig. 8C: TAs +Chol CpG; Fig. 8D: Cargomers 1:2; Fig. 8E: Cargomers 1:4; Fig. 8F: Cargomers 1:2 + immunotherapy; Fig. 8G: Cargomers 1:4 + immunotherapy; Fig. 8H: immunotherapy; Fig 8I: Cargomers 1:2 + immunotherapy; Fig. 8J: Cargomers 1:4 + immunotherapy). The growth curves for groups 6 and 7 are shown expanded on the Y axis in Fig. 8I and Fig. 8J, respectively.
**FIG. 9** shows the survival proportion for each treatment group of Example 2 over time.
**FIGS. 10A-10E** shows tumor volumes for the treatment groups of Example 3 (Fig. 10A: control; Fig. 10B: Cargomers 1:2; Fig. 10C: Cargomers 1:2 + immunotherapy; Fig. 10D: Cargomers 1:4; Fig. 10E: Cargomers 1:4 + immunotherapy).
**FIGS. 11A-11B** shows bodyweights (Fig. 11A) and baseline-corrected bodyweights (Fig. 11B) for the animals of Example 4 over the course of the study.
**FIG. 12** shows tumor volumes for the different treatment groups of Example 4.
**FIG. 13** shows tumor weights for the animals of each group of Example 4.
**FIG. 14** shows tumor volume for the animals of each group of Example 5.
FIGS. 15A-I shows tumor volumes for the animals of the treatment groups of Example 5 (Fig. 15A: vehicle; Fig. 15B: ApoA-I; Fig. 15C: TAs + CpG; Fig. 15D: TAs + CpG + immunotherapy; Fig. 15E: immunotherapy; Fig. 15F: Cargomers 1:2 ; Fig. 15G: Cargomers 1:4; Fig. 15H: Cargomers 1:2 + immunotherapy; Fig. 15I: Cargomers 1:4 + immunotherapy).
FIGS. 16A-D shows size exclusion chromatograms for Cargomers containing anti-STAT3 antisense oligonucleotide and for components thereof (Fig. 16A: STAT3 oligonucleotide (larger peak shows OD 260 nm, smaller peak shows OD 280 nm); Fig. 16B: ApoA-I (larger peak shows OD 280 nm, smaller peark shows OD 260 nm); Fig. 16C: ApoA-I:STAT3 oligonucleotide at 10:1 molar ratio (larger peak shows OD 280 nm, smaller peark shows OD 260 nm); Fig. 16D: superposition of STAT3 oligonucleotide and ApoA-I:STAT3 oligonucleotide Cargomers).
FIGS. 17A-D shows size exclusion chromatograms for Cargomers containing anti-KRAS siRNA and for components thereof (Fig. 17A: KRAS siRNA; Fig. 17B: ApoA-I:KRAS siRNA Cargomers premix; Fig. 17C: ApoA-I:KRAS siRNA Cargomers final preparation; Fig. 17D: superposition of ApoA-I:KRas siRNA premix and final preparations).
FIGS. 18A-D shows size exclusion chromatograms for Cargomers containing anti-EGFR siRNA and for components thereof (Fig. 18A: EGFR siRNA; Fig. 18B: ApoA-I:EGFR siRNA Cargomers premix; Fig. 18C: ApoA-I:EGFR siRNA Cargomers final preparation; Fig. 18D: superposition of ApoA-I:EGFR siRNA premix and final preparations).
FIGS. 19A-D shows size exclusion chromatograms for Cargomers containing CpG oligonucleotides and for components thereof (Fig. 19A: Chol-CpG; Fig. 19B: ApoA-I; Fig. 19C: ApoA-I:Chol-CpG Cargomers; Fig. 19D: superposition of ApoA-I:Chol-CpG Cargomers and Chol-CpG).
FIGS. 20A-D show the results of a western blot assay to measure the silencing efficacy of various exemplary Cargomers (Fig. 20A and Fig. 20B show the blot membranes; Fig. 20C and Fig. 20D show relative intensities of the bands).
FIGS. 21A-D show size exclusion chromatograms for ApoA-I (Fig.21A), M27 (Fig. 21B), TRP2 (Fig. 21C) and M30 (Fig. 21D).
FIGS. 22A-D show size exclusion chromatograms for the M27 peptide (Fig. 22A) and Cargomers containing M27 peptide (Figs. 22B-22D).
FIGS. 23A-D show size exclusion chromatograms for the M30 peptide (Fig. 23A) and Cargomers containing M30 peptide (Figs. 23B: -23D).
FIGS. 24A-F shows size exclusion chromatograms for the TRP2 peptide (Fig. 24A), ApoA-I (Fig. 24B) and Cargomers containing TRP2 peptide (Figs. 24C-24F).
FIGS. 25A-D show electron micrographs of ApoA-I (Fig. 25A), CER-001 (Fig. 25B), exemplary ApoA-I:M27 (1:2) Cargomers (Fig. 25C), and exemplary ApoA-I:M27 (1:2) Cargomers and CER-001 (Fig. 25D), showing that the exemplary Cargomers are small and not discoidal.

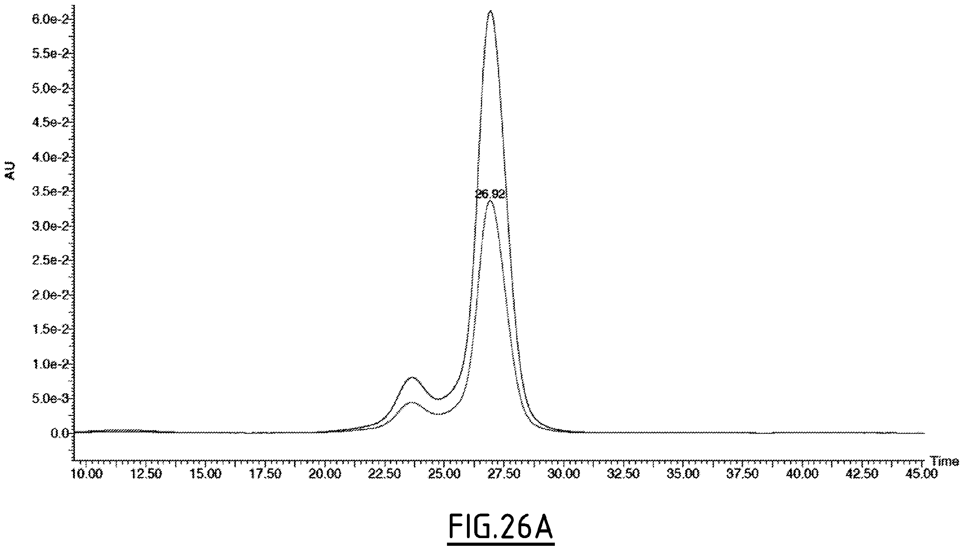

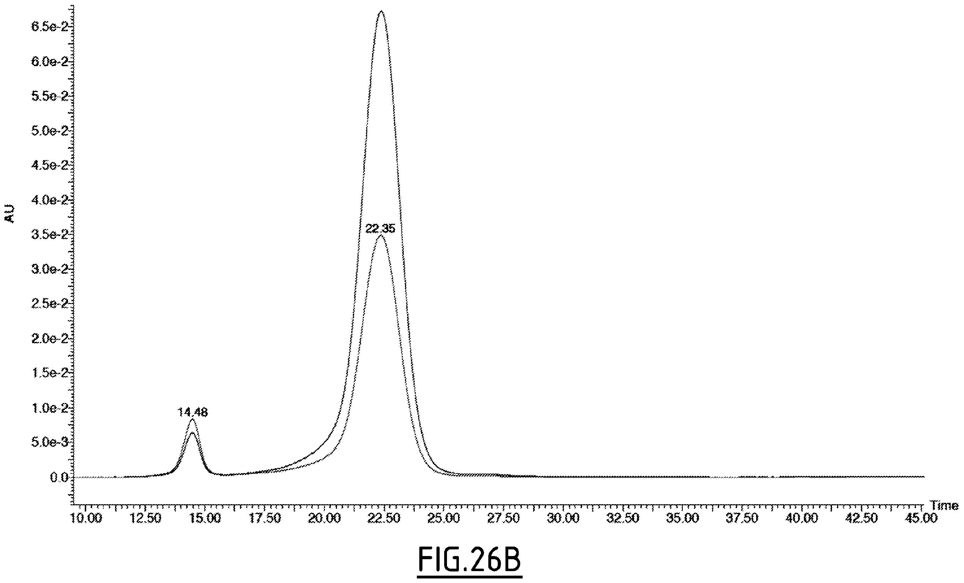

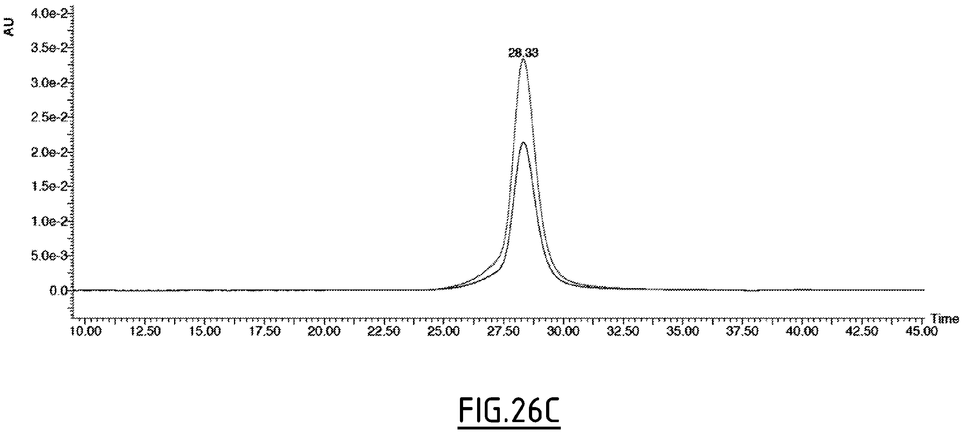

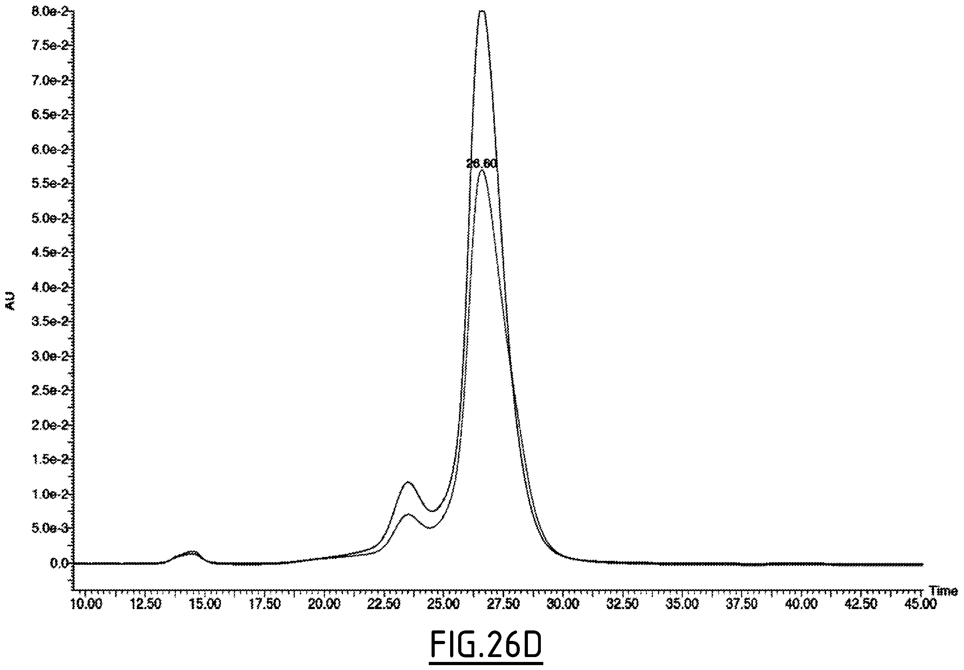

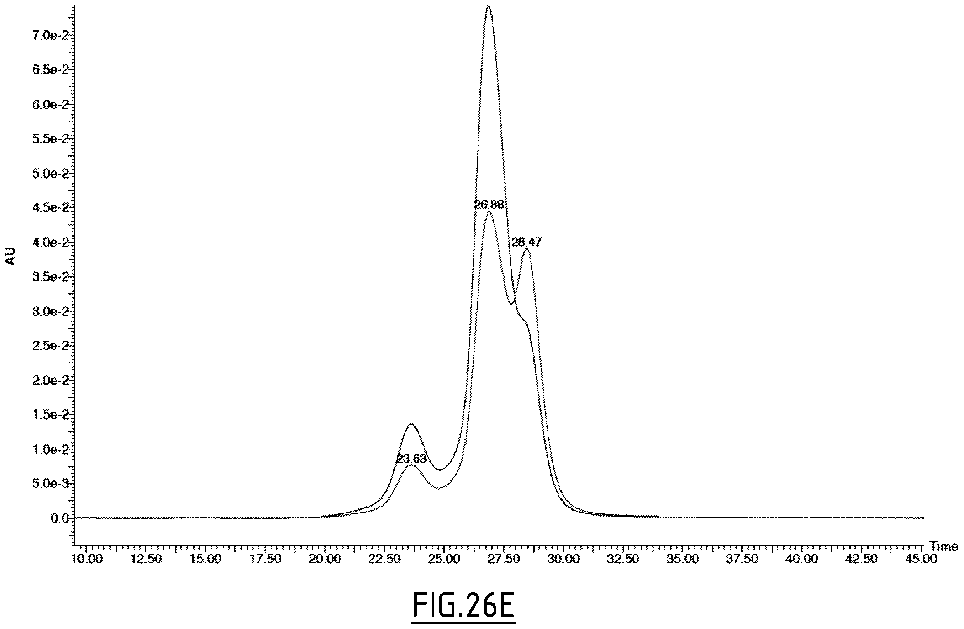

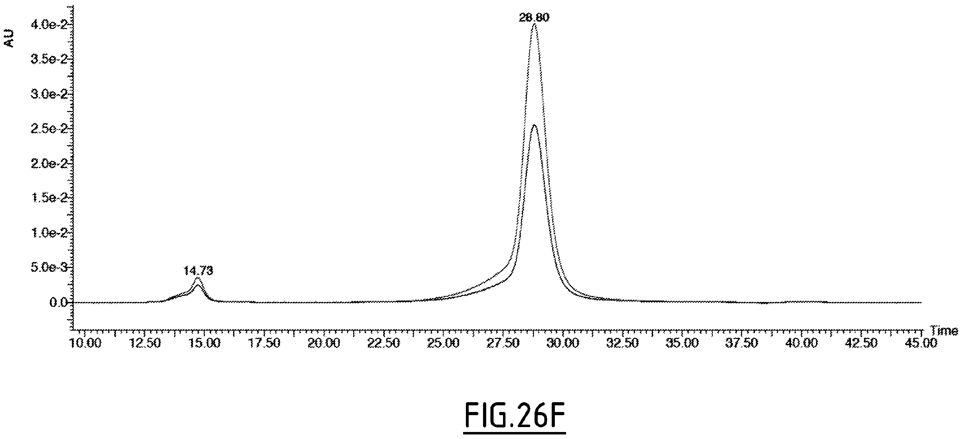

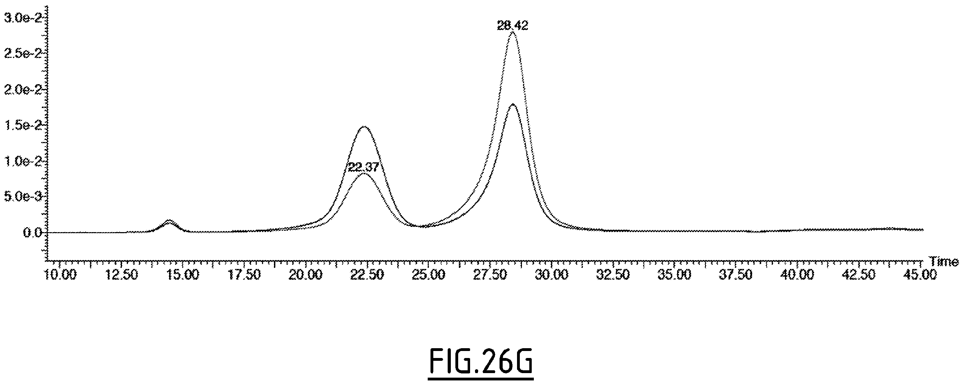

FIGS. 26A-G show chromatograms for chol-CpG containing Cargomers, components thereof, CER-001 and CER-001 with chol-CpG. Fig. 26A: ApoA-I (upper trace 280 nm; lower trace 260 nm); Fig. 26B: CER-001 (main peak upper trace 280 nm; lower trace 260 nm); Fig. 26C: Chol-CpG (upper trace 260 nm; lower trace 280 nm); Fig. 26D: ApoA-I:SM:Chol-CpG (1:2:0.1 molar ratio) Cargomers (upper trace 280 nm; lower trace 260 nm); Fig. 26E: ApoA-I:Chol-CpG (1:0.1 molar ratio) Cargomers (upper trace 280 nm; lower trace 260 nm); Fig. 26F: SM:Chol-CpG (2:0.1 molar ratio) (upper trace 260 nm; lower trace 280 nm); Fig. 26G: CER-001:Chol-CpG (1:0.5 molar ratio) (22.37 minute peak upper trace 280 nm; lower trace 260 nm) (28.42 minute peak upper trace 260 nm; lower trace 280 nm).

### 6. DETAILED DESCRIPTION

### 6.1. Cargomers

The Cargomers of the disclosure comprise 1-8 apolipoprotein molecules (*e*.*g*., 1, 2, 4, or 8 lipoprotein molecules) complexed with a sufficient number of amphipathic molecules to solubilize the apolipoprotein molecules. Preferably, Cargomers of the disclosure are not discoidal, for example as determined using NMR spectroscopy, atomic force microscopy, electron microscopy, or other suitable technique known in the art.

The apolipoprotein molecules are complexed with the amphipathic molecules in an apolipoprotein:amphipathic molecule molar ratio ranging from 8:1 to 1:15 (*e.g*., from 8:1 to 1:15, from 7:1 to 1:15, from 6:1 to 1:15, from 5:1 to 1:15, from 4:1 to 1:15, from 3:1 to 1:15, from 2:1 to 1:15, from 1:1 to 1:15, from 8:1 to 1:14, from 7:1 to 1:14, from 6:1 to 1:14, from 5:1 to 1:14, from 4:1 to 1:14, from 3:1 to 1:14, from 2:1 to 1:14, from 1:1 to 1:14, from 8:1 to 1:13, from 7:1 to 1:13, from 6:1 to 1:13, from 5:1 to 1:13, from 4:1 to 1:13, from 3:1 to 1:13, from 2:1 to 1:13, from 1:1 to 1:13, from 8:1 to 1:12, from 7:1 to 1:12, from 6:1 to 1:12, from 5:1 to 1:12, from 4:1 to 1:12, from 3:1 to 1:12, from 2:1 to 1:12, from 1:1 to 1:12, from 8:1 to 1:11, from 7:1 to 1:11, from 6:1 to 1:11, from 5:1 to 1:11, from 4:1 to 1:11, from 3:1 to 1:11, from 2:1 to 1:11, from 1:1 to 1:11, from 8:1 to 1:10, from 7:1 to 1:10, from 6:1 to 1:10, from 5:1 to 1:10, from 4:1 to 1:10, from 3:1 to 1:10, from 2:1 to 1:10, from 1:1 to 1:10, from 8:1 to 1:9, from 7:1 to 1:9, from 6:1 to 1:9, from 5:1 to 1:9, from 4:1 to 1:9, from 3:1 to 1:9, from 2:1 to 1:9, from 1:1 to 1:9, from 8:1 to 1:8, from 7:1 to 1:8, from 6:1 to 1:8, from 5:1 to 1:8, from 4:1 to 1:8, from 3:1 to 1:8, from 2:1 to 1:8, from 1:1 to 1:8, from 8:1 to 1:7, from 7:1 to 1:7, from 6:1 to 1:7, from 5:1 to 1:7, from 4:1 to 1:7, from 3:1 to 1:7, from 2:1 to 1:7, from 1:1 to 1:7, from 8:1 to 1:6, from 7:1 to 1:6, from 6:1 to 1:6, from 5:1 to 1:6, from 4:1 to 1:6, from 3:1 to 1:6, from 2:1 to 1:6, from 1:1 to 1:6, from 8:1 to 1:5, from 7:1 to 1:5, from 6:1 to 1:5, from 5:1 to 1:5, from 4:1 to 1:5, from 3:1 to 1:5, from 2:1 to 1:5, from 1:1 to 1:5, from 8:1 to 1:4, from 7:1 to 1:4, from 6:1 to 1:4, from 5:1 to 1:4, from 4:1 to 1:4, from 3:1 to 1:4, from 2:1 to 1:4, from 1:1 to 1:4, from 8:1 to 1:3, from 7:1 to 1:3, from 6:1 to 1:3, from 5:1 to 1:3, from 4:1 to 1:3, from 3:1 to 1:3, from 2:1 to 1:3, from 1:1 to 1:3, from 8:1 to 1:2, from 7:1 to 1:2, from 6:1 to 1:2, from 5:1 to 1:2, from 4:1 to 1:2, from 3:1 to 1:2, from 2:1 to 1:2, from 1:1 to 1:2, from 8:1 to 1:1, from 7:1 to 1:1, from 6:1 to 1:1, from 5:1 to 1:1, from 4:1 to 1:1, from 3:1 to 1:1, or from 2:1 to 1:1).

In some embodiments, the apolipoprotein molecules are complexed with the amphipathic molecules in an apolipoprotein:amphipathic molecule molar ratio ranging from 6:1 to 1:6 (*e*.*g*., from 5:1 to 1:6, from 4:1 to 1:6, from 3:1 to 1:6, from 2:1 to 1:6, from 5:1 to 1:5, from 4:1 to 1:5, from 3:1 to 1:5, from 2:1 to 1:5, from 5:1 to 1:4, from 4:1 to 1:4, from 3:1 to 1:4, from 2:1 to 1:4, from 5:1 to 1:3, from 4:1 to 1:3, from 3:1 to 1:3, from 2:1 to 1:3, from 5:1 to 1:2, from 4:1 to 1:2, from 3:1 to 1:2, from 2:1 to 1:2, from 5:1 to 1:1, from 4:1 to 1:1, from 3:1 to 1:1, from 2:1 to 1:1, from 1:1 to 1:6, from 1:1 to 1:5, from 1:1 to 1:4, from 1:1 to 1:3, from 1:1 to 1:2, from 1:2 to 1:6, from 1:2 to 1:5, from 1:2 to 1:4, from 1:2 to 1:3, from 1:3 to 1:6, from 1:3 to 1:5, from 1:3 to 1:4, from 1:4 to 1:6, from 1:4 to 1:5, from 1:5 to 1:6, from 1.5:1 to 1:2, from 5:4 to 4:5, from 5:3 to 3:5, from 5:2 to 2:5, or from 3:2 to 2:3).

Cargomers include one or more cargo moieties, which are optionally coupled to the Cargomers via an anchor and/or linker. In some embodiments, at least one of the cargo moieties, a majority of the cargo moieties, or all of the cargo moieties in a Cargomer are coupled to the Cargomer via anchors. In some embodiments, at least one of the cargo moieties in a Cargomer is coupled to the Cargomer via an anchor. In some embodiments, a majority of the cargo moieties in a Cargomer are coupled to the Cargomer via anchors. In some embodiments, all of the cargo moieties in a Cargomer are coupled to the Cargomer via anchors. Each anchor in a Cargomer can be the same or, alternatively, different types of anchors can be included in a single Cargomer (*e.g*., one type of cargo moiety can be coupled to the Cargomer via one type of anchor and a second type of cargo moiety can be coupled to the Cargomer via a second type of anchor).

An "anchor" as used herein refers to an amphipathic or apolar moiety that is covalently bound to a cargo moiety and which is non-covalently coupled to the apolipoprotein in the Cargomer, either directly or, where the Cargomer includes an amphipathic molecule other than the anchor moiety, via another amphipathic molecule in the Cargomer. The use of an amphipathic anchor moiety can, in certain embodiments, contribute to the apolipoprotein: amphipathic molecule molar ratio. In other embodiments, the amphipathic anchor molecule is not used in the apolipoprotiein: amphipathic molecule molar ratio calculation.

A "linker" as used herein refers to a moiety that covalently links a cargo moiety to an apolipoprotein molecule, an amphipathic molecule, or an anchor.

The molar ratio of apolipoprotein molecules to amphipathic molecules can be but does not necessarily have to be in integers or reflect a one to one relationship between the apolipoprotein and amphipathic molecules. By way of example and not limitation, a Cargomer can have an apolipoprotein to amphipathic molecule molar ratio of 2:5, 8:7, 3:2, or 4:7.

The amphipathic molecules, cargo moieties, anchors and linkers, if present, can together contribute a net charge of at least +1 or -1 per apolipoprotein in the Cargomer (*e.g*., +1, +2, +3, -1, -2, or -3). In some embodiments, for example when a cargo moiety comprises an oligonucleotide such as a siRNA, the amphipathic molecules, cargo moieties, anchors and linkers, if present, can together contribute a net charge of more than 3 (*e.g*., 5 to 30, 5 to 20, 5 to 10, 10 to 30, 10 to 20, or 20 to 30). In some embodiments, the net charge is a negative charge. In other embodiments, the net charge is a positive charge. Unless required otherwise by context, charge is measured at physiological pH.

Cargomers of the disclosure can be made, for example, by combining and mixing a composition comprising apolipoprotein molecules (*e*.*g*., a composition comprising multimer aggregates of apolipoprotein) with a solution comprising the amphipathic molecules (*e*.*g*., a solution comprising the amphipathic molecules alone or a solution comprising the amphipathic molecules and cargo moieties).

For example, Cargomers can be prepared by mixing two organic solutions, one containing an apolipoprotein and the other one containing a charged amphipathic molecule, then removing the solvent by methods such as evaporation, freeze-drying (lyophilization), spray-drying, heating or any other method known in the art. Cargomers can also be prepared by mixing two aqueous solutions, one containing an apolipoprotein and the other one containing a charged amphipathic molecule, until an homogeneous solution is obtained. Cargomers can also be prepared by hydrating an apolipoprotein with an aqueous solution of charged amphipathic molecules, then mixing until an homogeneous solution is obtained. The solutions used to make Cargomers, *e*.*g*., aqueous solutions, can be at room temperature, at a higher temperature than room temperature, or at a lower temperature than room temperature during formation of the Cargomers. Alternatively, the solutions can be thermal cycled between a higher and lower temperature, *e*.*g*., as described in Example 1 or WO 2012/109162, preferably until Cargomers of at least 85%, at least 90%, at least 95% or at least 98% homogeneity are obtained. If the solution comprising the amphipathic molecules does not contain the cargo moieties, a solution comprising the cargo moieties can be combined and mixed with the solution containing the apolipoprotein and amphipathic molecules (*e*.*g*., before thermal cycling).

Without being bound by theory, it is believed that the process of making Cargomers results in the formation of multiple species of Cargomers having different numbers of apolipoprotein molecules in equilibrium. It is known in the art that the self-association of lipid-free ApoA-I is influenced by conditions such as pH, concentration, ionic strength, and temperature (see, *e.g.,* Gianazza et al., 1997, Biochemistry, 36:7898-7905; Jayaraman et al., Journal of Biological Chemistry, 286(41):35610-35623; Schonfeld et al., 2016 J. Phys. Chem. B, 120:1228-1235) and it is believed that the equilibrium between different Cargomer species is similarly influenced by pH, concentration, ionic strength, and temperature. For example, acidic pH promotes formation of monomeric ApoA-I whereas alkaline pH encourages formation of multimeric forms, low concentrations of ApoA-I favor monomeric ApoA-I whereas high concentrations favor multimeric forms, and monomeric forms of ApoA-I are favored as temperature increases or decreases from ApoA-I's self-association maximum of 22°C. Once the Cargomers are formed, they are believed to be relatively stable and may not be as susceptible to dissociation compared to lipid-free apolipoprotein.

In some embodiments, the ratio of the apolipoprotein molecules to amphipathic molecules is about 1:1. In other embodiments, the ratio of the apolipoprotein molecules to amphipathic molecules is about 1:2. In yet other embodiments, the ratio of the apolipoprotein molecules to amphipathic molecules is about 1:3. In yet other embodiments, the ratio of the apolipoprotein molecules to amphipathic molecules is about 1:4. In yet other embodiments, the ratio of the apolipoprotein molecules to amphipathic molecules is about 1:5. In yet other embodiments, the ratio of the apolipoprotein molecules to amphipathic molecules is about 1:6.

In some embodiments, a Cargomer comprises 1 apolipoprotein molecule.

In other embodiments, a Cargomer comprises 2 apolipoprotein molecules. Cargomers comprising 2 apolipoprotein molecules preferably have a Stokes radius of 5 nm or less (e.g., 4 nm or less or 3 nm or less). The size of a Cargomer is believed to generally depend on the size of the Cargo moiety, such that Cargomers comprising a relatively large cargo moiety are expected to generally have a larger Stokes radius than a Cargomer having a smaller cargo moiety. In some embodiments, a Cargomer can comprise 2 apolipoprotein molecules and 1, 2, or 3 negatively charged amphipathic molecules (*e*.*g*., negatively charged phospholipid molecules) per apolipoprotein molecule.

In other embodiments, a Cargomer comprises 4 apolipoprotein molecules. Cargomers comprising 4 apolipoprotein molecules preferably have a Stokes radius of 5 nm or less (*e*.*g*., 4 nm or less or 3 nm or less). In some embodiments, a Cargomer can comprise 4 apolipoprotein molecules and 1, 2, or 3 negatively charged amphipathic molecules (*e*.*g*., negatively charged phospholipid molecules) per apolipoprotein molecule.

In other embodiments, a Cargomer comprises 8 apolipoprotein molecules. Cargomers comprising 8 apolipoprotein molecules preferably have a Stokes radius of 5 nm or less (*e*.*g*., 4 nm or less or 3 nm or less). In some embodiments, a Cargomer can comprise 8 apolipoprotein molecules and 1, 2, or 3 negatively charged amphipathic molecules (*e*.*g*., negatively charged phospholipid molecules) per apolipoprotein molecule. In certain embodiments, the Cargomers of the disclosure do not contain cholesterol and/or a cholesterol derivative (*e*.*g*., a cholesterol ester).

The Cargomers of the disclosure are preferably soluble in a biological fluid, for example one or more of lymph, cerebrospinal fluid, vitreous humor, aqueous humor, and blood or a blood fraction (*e*.*g*., serum or plasma).

Cargomers may include a targeting functionality, for example to target the Cargomers to a particular cell or tissue type, or to an infectious agent. In some embodiments, the Cargomer includes a targeting moiety attached to an apolipoprotein molecule or an amphipathic molecule. In some embodiments, one or more cargo moieties that are incorporated into the Cargomer has a targeting capability.

### 6.1.1. Apolipoproteins

Suitable apolipoproteins that can be included in the Cargomers of the disclosure include apolipoproteins ApoA-I, ApoA-II, ApoA-IV, ApoA-V, ApoB, ApoC-I, ApoC-II, ApoC-III, ApoD, ApoE, ApoJ, ApoH, and any combination of two or more of the foregoing. Polymorphic forms, isoforms, variants and mutants as well as truncated forms of the foregoing apolipoproteins, the most common of which are Apolipoprotein A-I_{Milano} (ApoA-I_{M}), Apolipoprotein A-I_{Paris} (ApoA-I_{P}), and Apolipoprotein A-I_{Zaragoza} (ApoA-I_{Z}), can also be used. Apolipoproteins mutants containing cysteine residues are also known, and can also be used (see, *e*.*g*., U.S. Publication No. 2003/0181372). The apolipoproteins can be modified in their primary sequence to render them less susceptible to oxidations, for example, as described in U.S. Publication Nos. 2008/0234192 and 2013/0137628, and U.S. Patent Nos. 8,143,224 and 8,541,236. The apolipoproteins can include residues corresponding to elements that facilitate their isolation, such as His tags, or other elements designed for other purposes. Preferably, the apolipoprotein in the Cargomer is soluble in a biological fluid (*e*.*g*., lymph, cerebrospinal fluid, vitreous humor, aqueous humor, blood, or a blood fraction (*e*.*g*., serum or plasma).

In some embodiments, an additional peptide can be included in a Cargomer in addition to an apolipoprotein. Such additional peptides include apolipoprotein fragments, peptide agonists of apolipoproteins, peptide analogues of apolipoproteins, and amphipathic peptides. These peptides can be made with natural amino acids, D-amino acids or can contain natural and/or non-natural amino acids. Exemplary peptide agonists are described in U.S. Patent Nos. 6,004,925, 6,037,323, 6,046,166, and 6,265,377.

Apolipoproteins can be purified from animal sources (and in particular from human sources) or produced recombinantly as is well-known in the art, see, *e.g.,* Chung et al., 1980, J. Lipid Res. 21(3):284-91; Cheung et al., 1987, J. Lipid Res. 28(8):913-29. See also U.S. Patent Nos. 5,059,528, 5,128,318, 6,617,134; U.S. Publication Nos. 2002/0156007, 2004/0067873, 2004/0077541, and 2004/0266660; and PCT Publications Nos. WO 2008/104890 and WO 2007/023476. Other methods of purification are also possible, for example as described in PCT Publication No. WO 2012/109162.

The apolipoprotein can be in prepro- form, pro- form, or mature form. For example, a Cargomer can comprise ApoA-I (*e*.*g*., human ApoA-I) in which the ApoA-I is preproApoA-I, proApoA-I, or mature ApoA-I. In some embodiments, the Cargomer comprises ApoA-I that has at least 90% sequence identity to SEQ ID NO:2. In other embodiments, the Cargomer comprises ApoA-I that has at least 95% sequence identity to SEQ ID NO:2. In other embodiments, the Cargomer comprises ApoA-I that has at least 98% sequence identity to SEQ ID NO:2. In other embodiments, the Cargomer comprises ApoA-I that has at least 99% sequence identity to SEQ ID NO:2. In other embodiments, the Cargomer comprises ApoA-I that has 100% sequence identity to SEQ ID NO:2.

The apolipoprotein molecule(s) can comprise a chimeric apolipoprotein comprising an apolipoprotein and one or more attached functional moieties, such as for example, one or more targeting moieties, a moiety having a desired biological activity, an affinity tag to assist with purification, and/or a reporter molecule for characterization or localization studies. An attached moiety with biological activity may have an activity that is capable of augmenting and/or synergizing with the biological activity of a cargo moiety incorporated into a Cargomer. For example, a moiety with biological activity may have antimicrobial (for example, antifungal, antibacterial, anti-protozoal, bacteriostatic, fungistatic, or antiviral) activity. In one embodiment, an attached functional moiety of a chimeric apolipoprotein is not in contact with hydrophobic surfaces of the Cargomer. In another embodiment, an attached functional moiety is in contact with hydrophobic surfaces of the Cargomer. In some embodiments, a functional moiety of a chimeric apolipoprotein may be intrinsic to a natural protein. In some embodiments, a chimeric apolipoprotein includes a ligand or sequence recognized by or capable of interaction with a cell surface receptor or other cell surface moiety.

In one embodiment, a chimeric apolipoprotein includes a targeting moiety that is not intrinsic to the native apolipoprotein, such as for example, *S. cerevisiae* α-mating factor peptide, folic acid, transferrin, or lactoferrin. In another embodiment, a chimeric apolipoprotein includes a moiety with a desired biological activity that augments and/or synergizes with the activity of a cargo moiety incorporated into the Cargomer. In one embodiment, a chimeric apolipoprotein may include a functional moiety intrinsic to an apolipoprotein. One example of an apolipoprotein intrinsic functional moiety is the intrinsic targeting moiety formed approximately by amino acids 130-150 of human ApoE, which comprises the receptor binding region recognized by members of the low density lipoprotein receptor family. Other examples of apolipoprotein intrinsic functional moieties include the region of ApoB-100 that interacts with the low density lipoprotein receptor and the region of ApoA-I that interacts with scavenger receptor type B 1. In other embodiments, a functional moiety may be added synthetically or recombinantly to produce a chimeric apolipoprotein. Another example is an apolipoprotein with the prepro or pro sequence from another preproapolipoprotein (*e*.*g*., prepro sequence from preproapoA-II substituted for the prepro sequence of preproapoA-I). Another example is an apolipoprotein for which some of the amphipathic sequence segments have been substituted by other amphipathic sequence segments from another apolipoprotein.

As used herein, "chimeric" refers to two or more molecules that are capable of existing separately and are joined together to form a single molecule having the desired functionality of all of its constituent molecules. The constituent molecules of a chimeric molecule may be joined synthetically by chemical conjugation or, where the constituent molecules are all polypeptides or analogs thereof, polynucleotides encoding the polypeptides may be fused together recombinantly such that a single continuous polypeptide is expressed. Such a chimeric molecule is termed a fusion protein. A "fusion protein" is a chimeric molecule in which the constituent molecules are all polypeptides and are attached (fused) to each other such that the chimeric molecule forms a continuous single chain. The various constituents can be directly attached to each other or can be coupled through one or more linkers. One or more segments of various constituents can be, for example, inserted in the sequence of an apolipoprotein, or, as another example, can be added N-terminal or C-terminal to the sequence of an apolipoprotein. For example, a fusion protein can comprise an antibody light chain, an antibody fragment, a heavy-chain antibody, or a single-domain antibody.

In some embodiments, a chimeric apolipoprotein is prepared by chemically conjugating the apolipoprotein and the functional moiety to be attached. Means of chemically conjugating molecules are well known to those of skill in the art. Such means will vary according to the structure of the moiety to be attached, but will be readily ascertainable to those of skill in the art. Polypeptides typically contain a variety of functional groups, *e.g.,* carboxylic acid (--COOH), free amino (--NH2), or sulfhydryl (--SH) groups, that are available for reaction with a suitable functional group on the functional moiety or on a linker to bind the moiety thereto. A functional moiety may be attached at the N-terminus, the C-terminus, or to a functional group on an interior residue (*i*.*e*., a residue at a position intermediate between the N- and C-termini) of an apolipoprotein molecule. Alternatively, the apolipoprotein and/or the moiety to be tagged can be derivatized to expose or attach additional reactive functional groups.

In some embodiments, fusion proteins that include a polypeptide functional moiety are synthesized using recombinant expression systems. Typically, this involves creating a nucleic acid (*e*.*g*., DNA) sequence that encodes the apolipoprotein and the functional moiety such that the two polypeptides will be in frame when expressed, placing the DNA under the control of a promoter, expressing the protein in a host cell, and isolating the expressed protein.

A nucleic acid encoding a chimeric apolipoprotein can be incorporated into a recombinant expression vector in a form suitable for expression in a host cell. As used herein, an "expression vector" is a nucleic acid which, when introduced into an appropriate host cell, can be transcribed and translated into a polypeptide. The vector may also include regulatory sequences such as promoters, enhancers, or other expression control elements (*e*.*g*., polyadenylation signals). Such regulatory sequences are known to those skilled in the art (see, *e*.*g*., Goeddel, 1990, Gene Expression Technology: Meth. Enzymol. 185, Academic Press, San Diego, Calif.; Berger and Kimmel, Guide to Molecular Cloning Techniques, Methods in Enzymology 152 Academic Press, Inc., San Diego, Calif.; Sambrook et al., 1989, Molecular Cloning--A Laboratory Manual (2nd ed.) Vol. 1-3, Cold Spring Harbor Laboratory, Cold Spring Harbor Press, NY, etc.).

In some embodiments, an apolipoprotein has been modified such that when the apolipoprotein is incorporated into a Cargomer, the modification will increase stability of the Cargomer, confer targeting ability or increase capacity. In one embodiment, the modification includes introduction of cysteine residues into apolipoprotein molecules to permit formation of intramolecular or intermolecular disulfide bonds, e.g., by site-directed mutagenesis. In another embodiment, a chemical crosslinking agent is used to form intermolecular links between apolipoprotein molecules to enhance stability of the Cargomers. Intermolecular crosslinking prevents or reduces dissociation of apolipoprotein molecules from the Cargomers and/or prevents displacement by endogenous apolipoprotein molecules within an individual to whom the Cargomers are administered. In other embodiments, an apolipoprotein is modified either by chemical derivatization of one or more amino acid residues or by site directed mutagenesis, to confer targeting ability to or recognition by a cell surface receptor.

Cargomers can be targeted to a specific cell surface receptor by engineering receptor recognition properties into an apolipoprotein. For example, Cargomers may be targeted to a particular cell type known to harbor a particular type of infectious agent, for example by modifying the apolipoprotein to render it capable of interacting with a receptor on the surface of the cell type being targeted. For example, Cargomers may be targeted to macrophages by altering the apolipoprotein to confer recognition by the macrophage endocytic class A scavenger receptor (SR-A). SR-A binding ability can be conferred to a Cargomer by modifying the apolipoprotein by site directed mutagenesis to replace one or more positively charged amino acids with a neutral or negatively charged amino acid. SR-A recognition can also be conferred by preparing a chimeric apolipoprotein that includes an N- or C-terminal extension having a ligand recognized by SR-A or an amino acid sequence with a high concentration of negatively charged residues. Cargomers can also interact with apolipoprotein receptors such as, but not limited to, ABCA1 receptors, ABCG1 receptors, CD36, Megalin, Cubulin and HDL receptors such as SR-B1.

The SR-B1 and other HDL receptors (*e*.*g*., ABCA1) are scavenger receptors essential to cell homeostasis, proliferation, and growth that can be up-regulated in cancer cells. Therefore, Cargomers of the disclosure can be used to target delivery of therapeutic agents (*e*.*g*., as described in Section 6.1.3.2) to cancer cells and tumors via the expression of SR-B1 and other HDL receptors on the surface of cancer cells.

### 6.1.2. Amphipathic molecules

An amphipathic molecule is a molecule that possesses both hydrophobic (apolar) and hydrophilic (polar) elements. Amphipathic molecules that can be used in the Cargomers of the disclosure include lipids, detergents, fatty acids, and apolar molecules and sterols covalently attached to polar molecules such as, but not limited to, sugars or nucleic acids. The Cargomers of the disclosure can include a single class of amphipathic molecule (*e*.*g*., a single species of phospholipids or a mixture of phospholipids), or can contain a combination of classes of amphipathic molecules (*e*.*g*., phospholipids and detergents). The Cargomer can contain one species of amphipathic molecules or a combination of amphipathic molecules configured to facilitate solubilization of the apolipoprotein molecule(s).

### 6.1.2.1. Lipids

The Cargomers of the disclosure can include one or more lipids. In various embodiments, one or more lipids can be saturated and/or unsaturated, natural and/or synthetic, charged or not charged, zwitterionic or not. Phospholipids can have two acyl chains that are the same or different (for example, chains having a different number of carbon atoms, a different degree of saturation between the acyl chains, different branching of the acyl chains, or a combination thereof). The lipid can also be modified to contain a fluorescent probe (*e.g*., as described at avantilipids.com/product-category/products/fluorescent-lipids/). Preferably, the lipid comprises at least one phospholipid.

Phospholipids can have unsaturated or saturated acyl chains ranging from about 6 to about 24 carbon atoms (*e*.*g*., 6-20, 6-16, 6-12, 12-24, 12-20, 12-16, 16-24, 16-20, or 20-24). In some embodiments, a phospholipid used in a Cargomer has one or two acyl chains of 12, 14, 16, 18, 20, 22, or 24 carbons (*e*.*g*, two acyl chains of the same length or two acyl chains of different length).

Non-limiting examples of acyl chains present in commonly occurring fatty acids that can be included in phospholipids are provided in Table 1, below:

| **Table 1** | |
|---|---|
| Length:Number of Unsaturations | Common Name |
| 14:0 | myristic acid |
| 16:0 | palmitic acid |
| 18:0 | stearic acid |
| 18:1 cisΔ⁹ | oleic acid |
| 18:2 cisΔ^{9,12} | linoleic acid |

| **Table 1** | |
|---|---|
| Length:Number of Unsaturations | Common Name |
| 18:3 cisΔ^{9,12,15} | linonenic acid |
| 20:4 cisΔ^{5,8,11,14} | arachidonic acid |
| 20:5 cisΔ^{5,8,11,14,17} | eicosapentaenoic acid (an omega-3 fatty acid) |

Lipids that can be present in the Cargomers include, but are not limited to, small alkyl chain phospholipids, egg phosphatidylcholine, soybean phosphatidylcholine, dipalmitoylphosphatidylcholine, dimyristoylphosphatidylcholine, distearoylphosphatidylcholine 1-myristoyl-2-palmitoylphosphatidylcholine, 1-palmitoyl-2-myristoylphosphatidylcholine, 1-palmitoyl-2-stearoylphosphatidylcholine, 1-stearoyl-2-palmitoylphosphatidylcholine, dioleoylphosphatidylcholine dioleophosphatidylethanolamine, dilauroylphosphatidylglycerol phosphatidylcholine, phosphatidylserine, phosphatidylethanolamine, phosphatidylinositol, phosphatidylglycerols, diphosphatidylglycerols such as dimyristoylphosphatidylglycerol, dipalmitoylphosphatidylglycerol, distearoylphosphatidylglycerol, dioleoylphosphatidylglycerol, dimyristoylphosphatidic acid, dipalmitoylphosphatidic acid, dimyristoylphosphatidylethanolamine, dipalmitoylphosphatidylethanolamine, dimyristoylphosphatidylserine, dipalmitoylphosphatidylserine, brain phosphatidylserine, brain sphingomyelin, palmitoylsphingomyelin, dipalmitoylsphingomyelin, egg sphingomyelin, milk sphingomyelin, phytosphingomyelin, distearoylsphingomyelin, dipalmitoylphosphatidylglycerol salt, phosphatidic acid, galactocerebroside, gangliosides, cerebrosides, dilaurylphosphatidylcholine, (1,3)-D-mannosyl-(1,3)diglyceride, aminophenylglycoside, 3-cholesteryl-6'-(glycosylthio)hexyl ether glycolipids, and cholesterol and its derivatives. Synthetic lipids, such as synthetic palmitoylsphingomyelin or N-palmitoyl-4-hydroxysphinganine-1-phosphocholine (a form of phytosphingomyelin) can be used to minimize lipid oxidation.

In some embodiments, the Cargomer includes two types of phospholipids: a neutral lipid, *e*.*g*., lecithin and/or sphingomyelin (abbreviated SM or SPH), and a charged phospholipid (*e*.*g*., a negatively charged phospholipid). A "neutral" phospholipid has a net charge of about zero at physiological pH. In many embodiments, neutral phospholipids are zwitterions, although other types of net neutral phospholipids are known and can be used. In some embodiments, the molar ratio of the charged phospholipid (*e*.*g*., negatively charged phospholipid) to neutral phospholipid ranges from 1:1 to 1:3, for example, about 1:1, about 1:2, or about 1:3.

The neutral phospholipid can comprise, for example, one or both of the lecithin and/or SM, and can optionally include other neutral phospholipids. In some embodiments, the neutral phospholipid comprises lecithin, but not SM. In other embodiments, the neutral phospholipid comprises SM, but not lecithin. In still other embodiments, the neutral phospholipid comprises both lecithin and SM. All of these specific exemplary embodiments can include neutral phospholipids in addition to the lecithin and/or SM, but in many embodiments do not include such additional neutral phospholipids.

The identity of the SM used is not critical for success. Thus, as used herein, the expression "SM" includes sphingomyelins derived or obtained from natural sources, as well as analogs and derivatives of naturally occurring SMs that are impervious to hydrolysis by LCAT, as is naturally occurring SM. SM is a phospholipid very similar in structure to lecithin, but, unlike lecithin, it does not have a glycerol backbone, and hence does not have ester linkages attaching the acyl chains. Rather, SM has a ceramide backbone, with amide linkages connecting the acyl chains. The SM can be obtained from virtually any source. For example, the SM can be obtained from milk, egg or brain. SM analogues or derivatives can also be used. Non-limiting examples of useful SM analogues and derivatives include, but are not limited to, palmitoylsphingomyelin, N-palmitoyl-4-hydroxysphinganine-1-phosphocholine (a form of phytosphingomyelin), palmitoylsphingomyelin, stearoylsphingomyelin, D-erythro-N-16:0-sphingomyelin and its dihydro isomer, D-erythro-N-16:0-dihydro-sphingomyelin. Synthetic SM such as synthetic palmitoylsphingomyelin or N-palmitoyl-4-hydroxysphinganine-1-phosphocholine (phytosphingomyelin) can be used in order to produce more homogeneous complexes and with fewer contaminants and/or oxidation products than sphingolipids of animal origin. Methods for synthesizing SM are described in U.S. Publication No. 2016/0075634.

Sphingomyelins isolated from natural sources can be artificially enriched in one particular saturated or unsaturated acyl chain. For example, milk sphingomyelin (Avanti Phospholipid, Alabaster, Ala.) is characterized by long saturated acyl chains (*i*.*e*., acyl chains having 20 or more carbon atoms). In contrast, egg sphingomyelin is characterized by short saturated acyl chains (i.e., acyl chains having fewer than 20 carbon atoms). For example, whereas only about 20% of milk sphingomyelin comprises C16:0 (16 carbon, saturated) acyl chains, about 80% of egg sphingomyelin comprises C16:0 acyl chains. Using solvent extraction, the composition of milk sphingomyelin can be enriched to have an acyl chain composition comparable to that of egg sphingomyelin, or vice versa.

The SM can be semi-synthetic such that it has particular acyl chains. For example, milk sphingomyelin can be first purified from milk, then one particular acyl chain, *e*.*g*., the C16:0 acyl chain, can be cleaved and replaced by another acyl chain. The SM can also be entirely synthesized, by *e.g.,* large-scale synthesis. See, *e.g.,* Dong et al., U.S. Pat. No. 5,220,043, entitled Synthesis of D-erythro-sphingomyelins, issued Jun. 15, 1993; Weis, 1999, Chem. Phys. Lipids 102 (1-2):3-12. SM can be fully synthetic, e.g., as described in U.S. Publication No. 2014/0275590..

The lengths and saturation levels of the acyl chains comprising a semi-synthetic or a synthetic SM can be selectively varied. The acyl chains can be saturated or unsaturated, and can contain from about 6 to about 24 carbon atoms. Each chain can contain the same number of carbon atoms or, alternatively each chain can contain different numbers of carbon atoms. In some embodiments, the semi-synthetic or synthetic SM comprises mixed acyl chains such that one chain is saturated and one chain is unsaturated. In such mixed acyl chain SMs, the chain lengths can be the same or different. In other embodiments, the acyl chains of the semi-synthetic or synthetic SM are either both saturated or both unsaturated. Again, the chains can contain the same or different numbers of carbon atoms. In some embodiments, both acyl chains comprising the semi-synthetic or synthetic SM are identical. In a specific embodiment, the chains correspond to the acyl chains of a naturally-occurring fatty acid, such as for example oleic, palmitic or stearic acid. In another embodiment, SM with saturated or unsaturated functionalized chains is used. In another specific embodiment, both acyl chains are saturated and contain from 6 to 24 carbon atoms. Non-limiting examples of acyl chains present in commonly occurring fatty acids that can be included in semi-synthetic and synthetic SMs are provided in Table 1, above.

In some embodiments, the SM is palmitoyl SM, such as synthetic palmitoyl SM, which has C16:0 acyl chains, or is egg SM, which includes as a principal component palmitoyl SM.

In a specific embodiment, functionalized SM, such as phytosphingomyelin, is used.

Lecithin can be derived or isolated from natural sources, or it can be obtained synthetically. Examples of suitable lecithins isolated from natural sources include, but are not limited to, egg phosphatidylcholine and soybean phosphatidylcholine. Additional non-limiting examples of suitable lecithins include, dipalmitoylphosphatidylcholine, dimyristoylphosphatidylcholine, distearoylphosphatidylcholine 1-myristoyl-2-palmitoylphosphatidylcholine, 1-palmitoyl-2-myristoylphosphatidylcholine, 1-palmitoyl-2-stearoylphosphatidylcholine, 1-stearoyl-2-palmitoylphosphatidylcholine, 1-palmitoyl-2-oleoylphosphatidylcholine, 1-oleoy1-2-palmitylphosphatidylcholine, dioleoylphosphatidylcholine and the ether derivatives or analogs thereof.

Lecithins derived or isolated from natural sources can be enriched to include specified acyl chains. In embodiments employing semi-synthetic or synthetic lecithins, the identity(ies) of the acyl chains can be selectively varied, as discussed above in connection with SM. In some embodiments of the Cargomers described herein, both acyl chains on the lecithin are identical. In some embodiments of Cargomers that include both SM and lecithin, the acyl chains of the SM and lecithin are all identical. In a specific embodiment, the acyl chains correspond to the acyl chains of myristitic, palmitic, oleic or stearic acid.

The Cargomers preferably include one or more negatively charged phospholipids (e.g., alone or in combination with one or more neutral phospholipids). As used herein, "negatively charged phospholipids" are phospholipids that have a net negative charge at physiological pH. The negatively charged phospholipid can comprise a single type of negatively charged phospholipid, or a mixture of two or more different, negatively charged, phospholipids. In some embodiments, the charged phospholipids are negatively charged glycerophospholipids. Specific examples of suitable negatively charged phospholipids include, but are not limited to, a 1,2-dipalmitoyl-*sn*-glycero-3-[phospho-*rac*-(1-glycerol)], a phosphatidylglycerol, a phospatidylinositol, a phosphatidylserine, a phosphatidic acid, and salts thereof (*e*.*g*., sodium salts or potassium salts). In some embodiments, the negatively charged phospholipid comprises one or more of phosphatidylinositol, phosphatidylserine, phosphatidylglycerol and/or phosphatidic acid. In a specific embodiment, the negatively charged phospholipid comprises or consists of a salt of a phosphatidylglycerol or a salt of a phosphatidylinositol. In another specific embodiment, the negatively charged phospholipid comprises or consists of 1,2-dipalmitoyl-*sn-*glycero-3-[phospho-*rac*-(1-glycerol)], or DPPG, or a salt thereof.

The negatively charged phospholipids can be obtained from natural sources or prepared by chemical synthesis. In embodiments employing synthetic negatively charged phospholipids, the identities of the acyl chains can be selectively varied, as discussed above in connection with SM. In some embodiments of the Cargomers described herein, both acyl chains on the negatively charged phospholipids are identical. In some embodiments, the acyl chains all types of phospholipids included in a Cargomer are all identical. In a specific embodiment, the Cargomer comprises negatively charged phospholipid(s), and/or SM all having C16:0 or C16:1 acyl chains. In a specific embodiment the fatty acid moiety of the SM is predominantly C16:1 palmitoyl. In one specific embodiment, the acyl chains of the charged phospholipid(s), lecithin and/or SM correspond to the acyl chain of palmitic acid. In yet another specific embodiment, the acyl chains of the charged phospholipid(s), lecithin and/or SM correspond to the acyl chain of oleic acid.

Cargomers can include one or more positively charged lipids (*e*.*g*., alone or in combination with one or more neutral phospholipids). Examples of positively charged phospholipids that can be included in the Cargomers of the disclosure include N1-[2-((1S)-1-[(3-aminopropyl)amino]-4-[di(3-amino-propyl)amino]butylcarboxamido)ethyl]-3,4-di[oleyloxy]-benzamide, 1,2-di-O-octadecenyl-3-trimethylammonium propane, 1,2-dimyristoleoyl-sn-glycero-3-ethylphosphocholine, 1-palmitoyl-2-oleoyl-sn-glycero-3-ethylphosphocholine, 1,2-dioleoyl-sn-glycero-3-ethylphosphocholine, 1,2-distearoyl-sn-glycero-3-ethylphosphocholine, 1,2-dipalmitoyl-sn-glycero-3-ethylphosphocholine, 1,2-dimyristoyl-sn-glycero-3-ethylphosphocholine, 1,2-dilauroyl-sn-glycero-3-ethylphosphocholine, 1,2-dilauroyl-sn-glycero-3-ethylphosphocholine, 1,2-dioleoyl-3-dimethylammonium-propane1,2-dimyristoyl-3-dimethylammonium-propane, 1,2-dipalmitoyl-3-dimethylammonium-propane, N-(4-carboxybenzyl)-N,N-dimethyl-2,3-bis(oleoyloxy)propan-1-aminium, 1,2-dioleoyl-3-trimethylammonium-propane, 1,2-dioleoyl-3-trimethylammonium-propane, 1,2-stearoyl-3-trimethylammonium-propane, 1,2-dipalmitoyl-3-trimethylammonium-propane, 1,2-dimyristoyl-3-trimethylammonium-propane, N-[1-(2,3-dimyristyloxy)propyl]-N, N-dimethyl-N-(2-hydroxyethyl) ammonium bromide, N,N,N-trimethyl-2-bis[(1-oxo-9-octadecenyl)oxy]-(Z,Z)- 1propanaminium methyl sulfate, and salts thereof (*e*.*g*., chloride or bromide salts). Other positively charged lipids such as stearylamine can also be used.

The lipids used are preferably at least 95% pure, and/or have reduced levels of oxidative agents (such as but not limited to peroxides). Lipids obtained from natural sources preferably have fewer polyunsaturated fatty acid moieties and/or fatty acid moieties that are not susceptible to oxidation. The level of oxidation in a sample can be determined using an iodometric method, which provides a peroxide value, expressed in milli-equivalent number of isolated iodines per kg of sample, abbreviated meq O/kg. *See, e*.*g*., Gray, 1978, Measurement of Lipid Oxidation: A Review, Journal of the American Oil Chemists Society 55:539-545; Heaton, F.W. and Ur, Improved iodometric Methods for the Determination of Lipid Peroxides, 1958, Journal of the Science of Food and Agriculture 9:781-786. Preferably, the level of oxidation, or peroxide level, is low, e.g., less than 5 meq O/kg, less than 4 meq O/kg, less than 3 meq O/kg, or less than 2 meq O/kg.

Cargomers can in some embodiments include small quantities of additional lipids. Virtually any type of lipids can be used, including, but not limited to, lysophospholipids, galactocerebroside, gangliosides, cerebrosides, glycerides, triglycerides, and sterols and sterol derivatives (*e*.*g*., a plant sterol, an animal sterol, such as cholesterol, or a sterol derivative, such as a cholesterol derivative). For example, a Cargomer can contain cholesterol or a cholesterol derivative, *e*.*g*., a cholesterol ester. The cholesterol derivative can also be a substituted cholesterol or a substituted cholesterol ester. The Cargomers of the disclosure can also contain an oxidized sterol such as, but not limited to, oxidized cholesterol or an oxidized sterol derivative (such as, but not limited to, an oxidized cholesterol ester). In some embodiments, the Cargomers do not include cholesterol and/or its derivatives (such as a cholesterol ester or an oxidized cholesterol ester).

The lipid molecules (*e*.*g*., phospholipid molecules) can together contribute a net charge of 1-3 (*e.g*., 1-3, 1-2, 2-3, 1, 2, or 3) per apolipoprotein molecule in the Cargomer. In some embodiments, the net charge is negative. In other embodiments, the net charge is positive.

### 6.1.2.2. Detergents

The Cargomers of the disclosure can contain one or more detergents. The detergent can be zwitterionic, nonionic, cationic, anionic, or a combination thereof. Exemplary zwitterionic detergents include 3-[(3-Cholamidopropyl)dimethylammonio]-1-propanesulfonate (CHAPS), 3-[(3-Cholamidopropyl)dimethylammonio]-2-hydroxy-1-propanesulfonate (CHAPSO), and N,N-dimethyldodecylamine N-oxide (LDAO). Exemplary nonionic detergents include D-(+)-trehalose 6-monooleate, N-octanoyl-N-methylglucamine, N-nonanoyl-N-methylglucamine, N-decanoyl-N-methylglucamine, 1-(7Z-hexadecenoyl)-*rac*-glycerol, 1-(8Z-hexadecenoyl)-*rac*-glycerol, 1-(8Z-heptadecenoyl)-*rac-*glycerol, 1-(9Z-hexadecenoyl)-rac-glycerol, 1-decanoyl-*rac*-glycerol. Exemplary cationic detergents include (S)-O-methyl-serine dodecylamide hydrochloride, dodecylammonium chloride, decyltrimethylammonium bromide, and cetyltrimethylammonium sulfate. Exemplary anionic detergents include cholesteryl hemisuccinate, cholate, alkyl sulfates, and alkyl sulfonates.

In some embodiments, the Cargomers of the disclosure lack detergents.

### 6.1.2.3. Fatty acids

The Cargomers can contain one or more fatty acids. The one or more fatty acids can include short-chain fatty acids having aliphatic tails of five or fewer carbons (*e*.*g*. butyric acid, isobutyric acid, valeric acid, or isovaleric acid), medium-chain fatty acids having aliphatic tails of 6 to 12 carbons (*e*.*g*., caproic acid, caprylic acid, capric acid, or lauric acid), long-chain fatty acids having aliphatic tails of 13 to 21 carbons (*e*.*g*., myristic acid, palmitic acid, stearic acid, or arachidic acid) , very long chain fatty acids having aliphatic tails of 22 or more carbons (*e*.*g*., behenic acid, lignoceric acid, or cerotic acid), or a combination thereof. The one or more fatty acids can be saturated (*e*.*g*., caprylic acid, capric acid, lauric acid, myristic acid, palmitic acid, stearic acid, arachidic acid, behenic acid, lignoceric acid, or cerotic acid), unsaturated (*e*.*g*., myristoleic acid, palmitoleic acid, sapienic acid, oleic acid, elaidic acid, vaccenic acid, linoleic acid, linoelaidic acid, α-linolenic acid, arachidonic acid, eicosapentaenoic acid, erucic acid, or docosahexaenoic acid) or a combination thereof. Unsaturated fatty acids can be cis or trans fatty acids. In some embodiments, unsaturated fatty acids used in the Cargomers of the disclosure are cis fatty acids.

### 6.1.2.4. Apolar molecules and sterols attached to a sugar

The Cargomers can contain one or more amphipathic molecules that comprise an apolar molecule or moiety (*e*.*g*., a hydrocarbon chain, an acyl or diacyl chain) or a sterol (*e*.*g*., cholesterol) attached to a sugar (*e*.*g*., a monosaccharide such as glucose or galactose, or a disaccharide such as maltose or trehalose). The sugar can be a modified sugar or a substituted sugar. Exemplary amphipathic molecules comprising an apolar molecule attached to a sugar include dodecan-2-yloxy-β-D-maltoside, tridecan-3-yloxy-β-D-maltoside, tridecan-2-yloxy-β-D-maltoside, n-dodecyl-β-D-maltoside (DDM), n-octyl-β-D-glucoside, n-nonyl-β-D-glucoside, n-decyl-β-D-maltoside, n-dodecyl-β-D-maltopyranoside, 4-n-Dodecyl-α,α-trehalose, 6-n-dodecyl-α,α-trehalose, and 3-n-dodecyl-α,α-trehalose.

### 6.1.3. Cargo moieties

The Cargomers of the disclosure comprise one or more cargo moieties (*e*.*g*., one, two, or three cargo moieties per Cargomer). In some embodiments, a Cargomer has 1 to 25 cargo moieties (*e*.*g*., 1 to 5, 1 to 10, 1 to 15, 1 to 20, 5 to 10, 5 to 15, 5 to 20, 10 to 25, 10 to 20, 10 to 15, 15 to 25, 15 to 20, or 20 to 25 cargo moieties). A Cargomer can have, for example, 1, 2, 3, 4, 5, 6, 7, 8, 9, 10, 11, 12, 13, 14, 15, 16, 17, 18, 19, 20, 21, 22, 23, 24, or 25 cargo moieties.

A cargo moiety can be, for example, a molecule or molecular assembly that is biologically active or has diagnostic utility (*e*.*g*., as described in Sections 6.1.3.1 to 6.1.3.10).

The cargo moiety can be an amphipathic molecule, for example, a ganglioside. Where the cargo moiety is amphipathic, the Cargomer need not include other amphipathic molecules, for example to solubilize the apolipoprotein, although Cargomers that include amphipathic cargo moieties and additional amphipathic molecules are within the scope of the present invention.

The cargo moiety need not be amphipathic. In such instances, the cargo moiety can be non-covalently or covalently attached to another component in the Cargomer. By way of example but not limitation, the cargo moeity can be (a) non-covalently bound to an apolar region of the Cargomer (*e*.*g*., an apolar core of a Cargomer formed by apolar regions of apolipoprotein molecules), (b) coupled to the Cargomer by being grafted to an amphipathic or apolar anchor that can non-covalently bind to an apolar region of the Cargomer (directly or via a linker) or (c) covalently coupled to an apolipoprotein molecule, for example, via a direct bond or via a linker.

The cargo moiety can be charged or uncharged. Charged cargo moieties can contribute a net charge (either positive or negative) of 1, 2, 3, or more than 3 per apolipoprotein in the Cargomer. In some embodiments the cargo moiety contributes a net charge of 1 (positive or negative) per apolipoprotein molecule in the Cargomer. In some embodiments the cargo moiety contributes a net charge of 2 (positive or negative) per apolipoprotein molecule in the Cargomer. In some embodiments the cargo moiety contributes a net charge of 3 (positive or negative) per apolipoprotein molecule in the Cargomer. In some embodiments the cargo moiety contributes a net charge of more than 3 (positive or negative) per apolipoprotein molecule in the Cargomer. In other embodiments, charged cargo moieties can contribute a net charge (either positive or negative) of 1, 2, 3, or more than 3 to the Cargomer. In some embodiments, the cargo moiety contributes a net charge of 1 (positive or negative) to the Cargomer. In some embodiments, the cargo moiety contributes a net charge of 2 (positive or negative) to the Cargomer. In some embodiments, the cargo moiety contributes a net charge of 3 (positive or negative) to the Cargomer. In some embodiments, the cargo moiety contributes a net charge of more than 3 (positive or negative) to the Cargomer. For example, a nucleic acid cargo moiety can have a relatively large charge.

Suitable cargo moieties include therapeutic agents, diagnostic agents, immunogens and adjuvants. Diagnostic agents that can be included are labeled agents such as gold labeled agents, labeled agents with stable isotopes, labeled agents with radioactive isotopes and labeled agents with fluorescent probes. Therapeutic agents that can be included in the Cargomer include immunoinhibitory agents, immunostimulatory agents, anti-cancer agents, anti-infective agents, nucleic acid drugs, anti-inflammatory agents, agents for treating cardiovascular disorders, caspase inhibitors and bioactive molecules. Unless required otherwise by context, identification of a specific agent encompasses salts thereof. Thus, for example, recitation of "warfarin" encompasses "warfarin sodium," recitation of "clopidogrel" encompasses "clopidogrel bisulfate," *etc.*

Cargomers can comprise a cargo moiety which targets the Cargomer to a desired body region (*e*.*g*., a target organ). The targeting feature can be part of, and or separate from, the therapeutic agent, diagnostic agent, immunogen or adjuvant. Such cargo moieties can assist in delivery of the Cargomers to desired body regions (*e*.*g*., bodily regions affected by a cardiovascular related disorder) and, in some instances, altogether deliver a biological (*e*.*g*., therapeutic) effect to the desired body region. Examples of targeting agents which can be included in a Cargomer include an antibody or antibody fragment (*e*.*g*. an antibody composed of two heavy chains and two light chains, an Fab fragment, a heavy chain antibody, or a single domain antibody), receptor ligand, hormone, vitamin, and antigen. In some embodiments, the antibody or antibody fragment is specific for a disease-specific antigen. In some embodiments, the receptor ligand includes, but is not limited to, a ligand for CFTR, EGFR, estrogen receptor, FGR2, folate receptor, IL-2 receptor, glycoprotein, and VEGFR. In some embodiments, the receptor ligand is folic acid. In some embodiments, the apolipoprotein moiety is the ligand to a receptor.

### 6.1.3.1. Immunoinhibitory and immunostimulatory agents

A Cargomer can include one or more immunoinhibitory agents. Immunoinhibitory agents that can be included in the Cargomers include steroids, retinoic acid, dexamethasone, cyclophosphamide, and combinations thereof.

A Cargomer can include one or more immunostimulatory agents. Immunostimulatory agents that can be included in the Cargomers include CpG oligonucleotides (ODNs), polyinosinic:polycytidylic acid (poly-I:poly-C), and other adjuvants (*e*.*g*., as described in Section 6.2). CpG ODNs are species-specific synthetic single stranded DNA incorporating unmethylated CpG dinucleotides. The optimal motif CpG motif in humans is GTCGTT and GACGTT in mouse. CpG ODNs mimic the immune stimulatory effects of unmethylated bacterial or viral sequences and activate pattern recognition transmembrane receptors, promote cytokine secretion and mount rapid responses to microbial pathogens. CpG ODNs mimic the natural Toll-like receptor (TLR) 9 ligand for the production of signaling factors and trigger a cascade of immune responses against cancer cells. These molecules can have a partially or completely phosphorothioated (PS) backbone as opposed to the phosphodiester (PO) backbone found in genomic bacterial DNA. There are three major classes (A, B, C) of stimulatory CpG ODNs based on structural characteristics and activity on human peripheral blood mononuclear cells (PBMCs) such as B cells and plasmacytoid dendritic cells (pDCs).

Class A CpG ODNs contain a central palindromic CpG-containing phosphodiester (PO) sequence and a PS-modified 3' poly-G string. The poly G tails form intermolecular tetrads to enhance stability and increase endosomal uptake which can then promote the maturation of pDCs and therefore production of large amounts of IFN-α. This class strongly activate Natural Killer (NK) cells through indirect cytokine signaling while weakly stimulating NF-κB signaling and pro-inflammatory cytokine (*e*.*g*. IL-6) production. Class B molecules are structurally linear and contain a fully PS backbone with one or more 6mer CpG motifs. CpG-B ODNs strongly stimulate B cell proliferation and activation along with NK cell activation through NF-κB signaling to display anti-tumor activity. These molecules are potent Type 1 T-helper cell (Th1) vaccine adjuvants, but are weak activators of IFN-α secretion. CpG-C ODNs are an amalgam of classes A and B with a complete PS backbone and a palindromic CpG-containing motif. These molecules are strong stimulators of B cells, type I IFN secretion and Th1-inducing adjuvants. All the CpG ODNs contain one or more unmethylated CpG dinucleotides in specific sequence contexts, which are readily recognized by mammalian cells as an indication of microbial invasion, due to the rarity of this structure in mammalian genomes.

In some embodiments, the Cargomer comprises a CpG oligonucleotide which is a class A CpG oligonucleotide, for example, a CpG oligonucleotide having a nucleotide sequence comprising or consisting of the nucleotide sequence of SEQ ID NO:3 or SEQ ID NO:4. In some embodiments, the Cargomer comprises a CpG oligonucleotide which is a class B CpG oligonucleotide, for example, a CpG oligonucleotide having a nucleotide sequence comprising or consisting of the nucleotide sequence of SEQ ID NO:5, SEQ ID NO:6 or SEQ ID NO:7. In yet other embodiments, the Cargomer comprises a CpG oligonucleotide which is a class C CpG oligonucleotide, for example, a CpG oligonucleotide having a nucleotide sequence comprising or consisting of the nucleotide sequence of SEQ ID NO:8, SEQ ID NO:9, or SEQ ID NQ:10.

A lipid moiety (*e*.*g*., having characteristics like that of cholesterol or other sterols) can be conjugated to a terminus of a CpG ODN to be used as an anchor to couple the CpG ODN to the Cargomer. A cholesterol tag can be added at the 3' or 5' of an ODN using a C4- to C8-linker or a polyethylene glycol linker to improve transduction of the molecule and improve nuclease resistance with greater target anti-viral activity. Another method of conjugating a CpG ODN to a lipid consists of conjugation of ODNs with alkyl chains (greater than 12 carbons), fatty acids, or lipid substituted crown ethers. Lipophilic dendrimers can be conjugated to either the 5' or 3' ends of ODNs to increase their cellular uptake, but increasing the size of the dendrimer can be a detriment to binding activity for their target inside the cell. Phosphatidyl groups are amenable to conjugation to ODNs and provide a high similarity in molecular structure to many lipid constituents of cell membranes. 1,2-Ditetra, 1,2-dihexa- and 1,2-dioctadecanoylglycerol having the *S* configuration at the stereogenic carbon can be used. The resulting phosphoramidites have the same stereochemistry at the chiral center as in naturally occurring phospholipids *(R)* to allow passage into cells. In addition, 5'-*O-*phosphatidyloligodeoxynucleotides with varying phosphatidyl tails and/or the sequence and length of the ODN moiety can be used.

In some embodiments, the Cargomer comprises a poly-I:poly-C oligonucleotide linked to a cholesterol, a phospholipid, or a fatty acid.

### 6.1.3.2. Anti-cancer agents

A Cargomer can include one or more anti-cancer agents. Anti-cancer agents that can be included in the Cargomer include topoisomerase inhibitors, DNA alkylating agents, DNA strand break inducing agents, anti-microtubule agents, an anti-metabolic agents, anthracyclines, vinca alkaloids, epipodophyllotoxins, tyrosine kinase inhibitors, CDK inhibitors, MAP kinase inhibitors, EGFR inhibitors, and VEGFR inhibitors.

In certain aspects, the anti-cancer agent is a chemotherapeutic agent, *e*.*g*., a cytotoxic or cytostatic agent. Examples of chemotherapeutic agents include alkylating agents such as thiotepa and CYTOXAN^{®} cyclosphosphamide; alkyl sulfonates such as busulfan, improsulfan, piposulfan and treosulfan; decarbazine; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; TLK 286 (TELCYTA^{™}); acetogenins (especially bullatacin and bullatacinone); delta-9-tetrahydrocannabinol (dronabinol, MARINOL^{®}); beta-lapachone; lapachol; colchicines; betulinic acid; a camptothecin (including the synthetic analogue topotecan (HYCAMTIN^{®}), CPT-11 (irinotecan, CAMPTOSAR^{®}), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; bisphosphonates, such as clodronate; antibiotics such as the enediyne antibiotics (e.g., calicheamicin, especially calicheamicin gamma1I and calicheamicin omegall (see, e.g., Agnew, Chem. Intl. Ed. Engl. 33:183-186 (1994)) and anthracyclines such as annamycin, AD 32, alcarubicin, daunorubicin, dexrazoxane, DX-52-1, epirubicin, GPX-100, idarubicin, KRN5500, menogaril, dynemicin, including dynemicin A, an esperamicin, neocarzinostatin chromophore and related chromoprotein enediyne antibiotic chromophores, aclacinomysins, actinomycin, authramycin, azaserine, bleomycins (e.g., bleomycin A2, bleomycin B2 and peplomycin), cactinomycin, carabicin, carminomycin, carzinophilin, chromomycinis, dactinomycin, detorubicin, 6-diazo-5-oxo-L-norleucine, ADRIAMYCIN^{®} doxorubicin (including morpholino-doxorubicin, cyanomorpholino-doxorubicin, 2-pyrrolino-doxorubicin, liposomal doxorubicin, and deoxydoxorubicin), esorubicin, marcellomycin, mitomycins such as mitomycin C, mycophenolic acid, tiazofurin, ribavarin, EICAR, nogalamycin, olivomycins, peplomycin, potfiromycin, puromycin, quelamycin, rodorubicin, streptonigrin, streptozocin, tubercidin, ubenimex, zinostatin, and zorubicin; folic acid analogues such as denopterin, pteropterin, and trimetrexate; purine analogs such as fludarabine, 6-mercaptopurine, thiamiprine, and thioguanine; pyrimidine analogs such as ancitabine, azacitidine, 6-azauridine, carmofur, cytarabine, dideoxyuridine, doxifluridine, enocitabine, and floxuridine; androgens such as calusterone, dromostanolone propionate, epitiostanol, mepitiostane, and testolactone; anti-adrenals such as aminoglutethimide, mitotane, and trilostane; folic acid replenisher such as folinic acid (leucovorin); aceglatone; anti-folate anti-neoplastic agents such as ALlMTA^{®}, LY231514 pemetrexed, dihydrofolate reductase inhibitors such as methotrexate and trimetrexate, anti-metabolites such as 5-fluorouracil (5-FU) and its prodrugs such as UFT, S-1 and capecitabine, and thymidylate synthase inhibitors and glycinamide ribonucleotide formyltransferase inhibitors such as raltitrexed (TOMUDEXRM, TDX); inhibitors of dihydropyrimidine dehydrogenase such as eniluracil; aldophosphamide glycoside; aminolevulinic acid; amsacrine; bestrabucil; bisantrene; edatraxate; defofamine; demecolcine; diaziquone; elfornithine; elliptinium acetate; an epothilone; etoglucid; gallium nitrate; hydroxyurea; deferoxamine; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK^{®} polysaccharide complex (JHS Natural Products, Eugene, Oreg.); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE^{®}, FILDESIN^{®}); dacarbazine; mannomustine; mitobronitol; mitolactol; pipobroman; gacytosine; cytosine arabinoside ("Ara-C"); cyclophosphamide; thiotepa; taxoids and taxanes, e.g., TAXOL.^{®} paclitaxel (Bristol-Myers Squibb Oncology, Princeton, N.J.), ABRAXANE^{™} Cremophor-free, albumin-engineered nanoparticle formulation of paclitaxel (American Pharmaceutical Partners, Schaumberg, III.), and TAXOTERE^{®} doxetaxel (Rhone-Poulenc Rorer, Antony, France); chloranbucil; gemcitabine (GEMZAR^{®}); 6-thioguanine; mercaptopurine; platinum; platinum analogs or platinum-based analogs such as cisplatin, oxaliplatin and carboplatin; vinblastine (VELBAN^{®}); epipodophyllins such as etoposide (VP-16), teniposide, tepotecan, 9-aminocamptothecin, camptothecin and crisnatol; ifosfamide; mitoxantrone; vinca alkaloids such as vincristine (ONCOVIN^{®}), vindesine, vinca alkaloid, and vinorelbine (NAVELBINE^{®}); novantrone; edatrexate; daunomycin; aminopterin; xeloda; ibandronate; topoisomerase inhibitor RFS 2000; difluorometlhylornithine (DMFO); retinoids such as retinoic acid; pharmaceutically acceptable salts, acids or derivatives of any of the above.

In some embodiments, the anti-cancer agent is methotrexate, taxol, L-asparaginase, mercaptopurine, thioguanine, hydroxyurea, cytarabine, cyclophosphamide, ifosfamide, nitrosoureas, cisplatin, carboplatin, mitomycin, dacarbazine, procarbizine, topotecan, nitrogen mustards, cytoxan, etoposide, 5-fluorouracil, BCNU, irinotecan, camptothecins, bleomycin, doxorubicin, idarubicin, daunorubicin, dactinomycin, plicamycin, mitoxantrone, asparaginase, vinblastine, vincristine, vinorelbine, paclitaxel, or docetaxel.

In some embodiments, the anti-cancer agent is an antibody. In certain embodiments, the anti-cancer antibody is an anti-CD20 antibody, *e*.*g*., rituximab or tositumomab (which are useful for treating, *inter alia,* B-cell non-Hodgkin's lymphoma), an anti-CD52 antibody, *e*.*g*., alemtuzumab (which is useful for treating B-cell chronic lymphocytic leukemia), an-anti EGF receptor antibody, *e*.*g*., cetuximab or panitumumab (which are useful for treating head and neck cancer and colorectal cancer), an anti-VEGF antibody, *e*.*g*., bevacizumab (which is useful for treating, inter alia, colorectal cancer), or an anti-HER2 antibody, *e*.*g*., trastuzumab (which is useful for treating HER2-positive metastatic breast cancer).

In some embodiments, the anti-cancer agent is in the form of an antibody-drug conjugate (ADC). Exemplary ADCs include gemtuzumab ozogamicin (approved to treat acute myeloid leukemia), brentuximab vedotin (approved to treat Hodgkin lymphoma), trastuzumab emtansine (approved to treat HER2-positive metastatic breast cancer), and Inotuzumab ozogamicin (approved to treat acute lymphoblastic leukemia).

Exemplary topoisomerase inhibitors include type I inhibitors such as irinotecan, topotecan, camptothecin and lamellarin D and type II inhibitor such as etoposide, teniposide, doxorubicin, daunorubicin, mitoxantrone, amsacrine, ellipticines, aurintricarboxylic acid, and HU-331. Exemplary DNA alkylating agents include classical DNA alkylating agents such as nitrogen mustards (*e*.*g*., cyclophosphamide, mechlorethamine, uramustine, melphalan, chlorambucil, ifosfamide, and bendamustine) nitrosoureas (*e.g*., carmustine, lomustine, and streptozocin) and alkyl sulfonates (*e.g*., busulfan), alkylating-like agents such as cisplatin, carboplatin, nedaplatin, oxaliplatin, satraplatin, and triplatin tetranitrate, and non-classical alkylating agents such as procarbazine and altretamine. Exemplary DNA strand break inducing agents include calicheamicin, etoposide, doxorubicin, ginsenoside Rg3, Bleomycin A5, Raltitrexed, and SCH-900776. Exemplary anti-microtubule agents include paclitaxel, docetaxel, and cabazitaxel. Exemplary anti-metabolic agents include 5-fluorouracil (5-FU), 6-mercaptopurine (6-MP), capecitabine, cytarabine, floxuridine, fludarabine, gemcitabine, hydroxycarbamide, methotrexate, and pemetrexed. Exemplary anthracyclines include daunorubicin and doxorubicin. Exemplary vinca alkaloids include vinblastine, vincristine, vindesine, vinorelbine, vincaminol, vineridine, and vinburnine. Exemplary epipodophyllotoxins include etoposide and teniposide. Exemplary tyrosine kinase inhibitors include imatinib, gefitinib, and erlotinib. Exemplary CDK inhibitors include palbociclib, abemaciclib, and ribociclib. Exemplary EGFR inhibitors include gefitinib, erlotinib, afatinib, brigatinib, icotinib, and cetuximab. Exemplary VEGFR inhibitors include pazopanib, vatalanib, sunitinib, and sorafenib.

In certain embodiments, a Cargomer of the disclosure comprises an anti-cancer agent that is useful in the treatment of melanoma. It has been demonstrated that melanomas that express high levels of SR-B1 are associated with poorer outcomes than melanomas that express low levels of SR-B1 (Mikula et al., 2017, Mol. Cancer Res., doi:10.1158/1541-7786). Without being bound by theory, it is expected that Cargomers carrying anti-melanoma agents are able to target melanoma cells that are more resistant to therapy. Accordingly, in certain aspects, the disclosure provides Cargomers comprising an anti-melanoma agent, such as, but not limited to, aldesleukin, cobimetinib, dabrafenib, dacarbazine, talimogene laherparepvec, ipilimumab, pembrolizumab, trametinib, nivolumab, or orvemurafenib). Such Cargomers can be administered to subjects with melanoma as monotherapy or as part of combination therapy regimens, for example with chemotherapeutic agents or interferon-based therapies (*e*.*g*., recombinant interferon alfa-2b, peginterferon alfa-2a, or peginterferon alfa-2b).

### 6.1.3.3. Anti-infective agents

A Cargomer can include one or more anti-infective agents. Anti-infective agents that can be included in the Cargomers of the disclosure include anti-bacterial agents, anti-viral agents, anti-parasitic agents, anti-fungal agents, and anti-mycobacterial agents.

Exemplary anti-bacterial agents include β-lactam antibiotics, penicillins (*e*.*g*., penicillin, methicillin, ampicillin, amoxicillin), cephalosporins (*e*.*g*., avibactam, cephalexin, cefepime, ceftaroline), β-lactamase inhibitors (*e*.*g*., tebipenem, clavulanate, sulbactam and tazobactam), vancomycin, aminoglycosides (*e*.*g*., gentamycin, neomycin B, neomycin C, neomycin E, streptomycin), tetracyclines (*e*.*g*., tetracycline, lymecycline, methacycline, and doxycycline), chloramphenicol, erythromycin, lincomycin, clindamycin, rifampin, metronidazole, polymyxins *(e.g.,* polymyxin B and polymyxin E), sulfonamides (*e.g*., sulfisoxazole and sulfaisodimidine), and quinolones (*e.g*., cinoxacin, ciprofloxacin, balofloxacin, and gatifloxacin).

Exemplary anti-viral agents include amantadine, rimantadine, ribivarin, acyclovir, vidarabine, trifluorothymidine, ganciclovir, zidovudine, retinovir, and interferons.

Exemplary anti-fungal agents include imidazoles (*e*.*g*., bifonazole, sertaconazole), triazoles (*e.g*., albaconazole, isavuconazole), polyene macrolide antibiotics (*e.g*., amphotericin B, nystatin, and natamycin), griseofulvin, amphotericin B, and flucytosine.

Anti-parasitic agents include anthelmintics and antiprotozoal agents.

### 6.1.3.4. Nucleic acid drugs

A Cargomer can include one or more nucleic acid drugs (which term includes nucleic acids intended for gene therapy). Nucleic acid drugs that can be included in the Cargomer of the disclosure include naturally or non-naturally occurring DNA, naturally or non-naturally occurring RNA, oligonucleotides, triple-helix forming molecules, immunostimulatory nucleic acids, small interfering RNAs (siRNA), microRNAs (miRNA), antisense oligonucleotides, aptamers, ribozymes, gene or gene fragments, and regulatory sequences. The nucleic acid drugs can include modified nucleotides and/or a modified backbone, *e.g.,* a nucleic acid drug can comprise peptide nucleic acids (see, *e.g*., Pansuwan et al., 2017, Bioconjug Chem. 28(9):2284-2292). The nucleic acid can be complexed to a moiety to facilitate binding to or uptake by a target cell. The nucleic acid can be covently bound to a molecule interacting with the Cargomer. For example, a nucleic acid can be covalently linked to a sterol, a fatty acid or a phospholipid such as those described in Section 6.1.2.1.

In some embodiments, the nucleic acid drug is a siRNA or antisense oligonucleotide. The antisense oligonucleotide can be, for example, a double-stranded oligonucleotide (*e*.*g*., as described in US 2017/0137816). Such embodiments are not limited to a particular size or type of molecule. The length of the region of the siRNA or antisense oligonucleotide complementary to the target, for example, can be from 15 to 100 nucleotides, 18 to 25 nucleotides, 20 to 23 nucleotides, or more than 15, 16, 17 or 18 nucleotides. Where there are mismatches to the corresponding target region, the length of the complementary region is generally required to be somewhat longer.

In certain embodiments, it is contemplated that delivering siRNA or antisense oligonucleotides using Cargomers disclosed herein can be used to inhibit any gene of interest.

Loading of a nucleic acid drug into Cargomers can be facilitated through cholesterol modification of the nucleic acid drug. For example, the siRNA can be modified with cholesterol at the 3' sense strand and an intermediate level of chemical modification can be used to stabilize siRNA in the serum without significantly compromising its silencing effect.

Nucleic acid drugs can be labeled with an imaging agent (*e*.*g*., fluorescent dye Cy3) to permit visualization of the biodistribution of the nucleic acid drug at the organ level and also the intracellular delivery profile. In some embodiments, RT-PCR and western blot are used to analyze the target protein at the mRNA level and protein level, respectively.

In some embodiments, the Cargomers comprise one or more siRNAs specific for proprotein convertase subtilisin/kexin 9 (PCSK9). In some embodiments, the PCSK9 siRNA sequence is cross-reactive to murine, rat, nonhuman primate and human PCSK9 mRNA (see, *e.g.,* Frank-Kamenetsky, et al., 2008, Proceedings of the National Academy of Sciences of the United States of America 105(33):11915-11920).

In some embodiments, the Cargomers comprise one or more siRNAs specific for the gene coding for apolipoprotein B, the apolipoprotein of LDL lipoproteins.

In some embodiments, the Cargomers comprise one or more targeted gene silencing molecules (*e*.*g*., siRNAs) to block production of the dysfunctional huntingtin (Htt) protein, the cause of Huntington's disease, a fatal, inherited neurodegenerative disorder (see, *e.g.,* www.scbt.com/scbt/product/huntingtin-sirna-h-shrna-and-lentiviral-particle-gene-silencers).

In some embodiments, the Cargomers comprise one or more targeted gene silencing molecules (*e*.*g*., siRNAs) to block production of the amyloid precursor protein (APP) products, which cause Alzheimer's disease.

In some embodiments, the Cargomers comprise one or more targeted gene silencing molecules, such as those described in WO 2014076195 A1, to block production of proteins involved in pathologic processes.

In some embodiments, the Cargomers comprise one or more targeted gene silencing molecules (*e*.*g*., antisense oligonucleotides or siRNAs) to block production of STAT3, which can be used, for example, for treating pancreatic cancer.

In some embodiments, the Cargomers comprise one or more targeted gene silencing molecules (*e*.*g*., antisense oligonucleotides or siRNAs) to block production of KRAS, which can be used, for example, for treating pancreatic cancer.

In some embodiments, the Cargomers comprise one or more targeted gene silencing molecules (*e*.*g*., antisense oligonucleotides or siRNAs) to block production of EGFR, which can be used, for example, for treating pancreatic cancer.

### 6.1.3.5. Anti-inflammatory agents

A Cargomer can include one or more anti-inflammatory agents. Anti-inflammatory agents that can be included in the Cargomers of the disclosure include one or more of Alclofenac; Alclometasone Dipropionate; Algestone Acetonide; Alpha Amylase; Amcinafal; Amcinafide; Amfenac Sodium; Amiprilose Hydrochloride; Anakinra; Anirolac; Anitrazafen; Apazone; Aspirin; Balsalazide Disodium; Bendazac; Benoxaprofen; Benzydamine Hydrochloride; Bromelains; Broperamole; Budesonide; Carprofen; Cicloprofen; Cintazone; Cliprofen; Clobetasol Propionate; Clobetasone Butyrate; Clopirac; Cloticasone Propionate; Cormethasone Acetate; Cortodoxone; Deflazacort; Desonide; Desoximetasone; Dexamethasone Dipropionate; Diclofenac Potassium; Diclofenac Sodium; Diflorasone Diacetate; Diflumidone Sodium; Diflunisal; Difluprednate; Diftalone; Dimethyl Sulfoxide; Drocinonide; Endrysone; Enlimomab; Enolicam Sodium; Epirizole; Etodolac; Etofenamate; Felbinac; Fenamole; Fenbufen; Fenclofenac; Fenclorac; Fendosal; Fenpipalone; Fentiazac; Flazalone; Fluazacort; Flufenamic Acid; Flumizole; Flunisolide Acetate; Flunixin; Flunixin Meglumine; Fluocortin Butyl; Fluorometholone Acetate; Fluquazone; Flurbiprofen; Fluretofen; Fluticasone Propionate; Furaprofen; Furobufen; Halcinonide; Halobetasol Propionate; Halopredone Acetate; Ibufenac; Ibuprofen; Ibuprofen Aluminum; Ibuprofen Piconol; Ilonidap; Indomethacin; Indomethacin Sodium; Indoprofen; Indoxole; Intrazole; Isoflupredone Acetate; Isoxepac; Isoxicam; Ketoprofen; Lofemizole Hydrochloride; Lornoxicam; Loteprednol Etabonate; Meclofenamate Sodium; Meclofenamic Acid; Meclorisone Dibutyrate; Mefenamic Acid; Mesalamine; Meseclazone; Methylprednisolone Suleptanate; Morniflumate; Nabumetone; Naproxen; Naproxen Sodium; Naproxol; Nimazone; Olsalazine Sodium; Orgotein; Orpanoxin; Oxaprozin; Oxyphenbutazone; Paranyline Hydrochloride; Pentosan Polysulfate Sodium; Phenbutazone Sodium Glycerate; Pirfenidone; Piroxicam; Piroxicam Cinnamate; Piroxicam Olamine; Pirprofen; Prednazate; Prifelone; Prodolic Acid; Proquazone; Proxazole; Proxazole Citrate; Rimexolone; Romazarit; Salcolex; Salnacedin; Salsalate; Salycilates; Sanguinarium Chloride; Seclazone; Sermetacin; Sudoxicam; Sulindac; Suprofen; Talmetacin; Talniflumate; Talosalate; Tebufelone; Tenidap; Tenidap Sodium; Tenoxicam; Tesicam; Tesimide; Tetrydamine; Tiopinac; Tixocortol Pivalate; Tolmetin; Tolmetin Sodium; Triclonide; Triflumidate; Zidometacin; Glucocorticoids; Gemcabene, Bempedoic acid and Zomepirac Sodium.

### 6.1.3.6. Agents for treating cardiovascular related disorders

The Cargomers of the disclosure can include one or more agents for treating cardiovascular related disorders, such as atherosclerosis, heart failure, arrhythmia, atrial fibrillation, hypertension, coronary artery disease, and angina pectoris). Examples of therapeutic agents known to be useful in treating and/or preventing cardiovascular related disorders include angiotensin-converting enzyme (ACE) inhibitors (*e*.*g*., benazepril, enalapril, Lisinopril, perindopril, Ramipril), adenosine, alpha blockers (alpha adrenergic antagonist medications) (*e*.*g*., clonidine, guanabenz, labetalol, phenoxybenzamine, terazosin, doxazosin, guanfacine, methyldopa, prazosin), angtiotensin II receptor blockers (ARBs) (*e*.*g*., candesartan, irbesartan, olmesartan medoxomil, telmisartan, eprosartan, losartan, tasosartan, valsartan), antiocoagulants (*e*.*g*., heparin fondaparinux, warfarin, ardeparin, enoxaparin, reviparin, dalteparin, nadroparin, tinzaparin), antiplatelet agents *(e.g.,* abciximab, clopidogrel, eptifibatide, ticlopidine, cilostazol, dipyridamole, sulfinpyrazone, tirofiban), beta blockers (*e*.*g*., acebutolol, betaxolol, carteolol, metoprolol, penbutolol, propranolol, atenolol, bisoprolol, esmolol, nadolol, pindolol, timolol), calcium channel blockers (*e.g.,* amlopidine, felodipine, isradipine, nifedipine, verapamil, diltiazem, nicardipine, nimodipine, nisoldipine), diuretics, aldosterone blockers, loop diuretics (*e*.*g*., bumetanide, furosemide, ethacrynic acid, torsemide), potassium-sparing diuretics, thiazide diuretics (*e.g.,* chlorothiazide, chlorthalidone, hydrochlorothiazide, hydroflumethiazide, methyclothiazide, metolazone, polythiazide, quinethazone, trichlormethiazide), inoptropics, bile acid sequestrants (*e.g*., cholestyramine, coletipol, colesevelam), fibrates (*e.g*., clofibrate, gemfibrozil, fenofibrate), statins (*e.g*., atorvastatin, lovastatin, simvastatin, fluvastatin, pravastatin), selective cholesterol absorption inhibitors (*e.g.,* ezetimibe), gemcabene, bempedoic acid , potassium channel blockers (*e.g.,* amidarone, ibutilide, dofetilide), sodium channel blockers (*e*.*g*., disopyramide, mexiletine, procainamide, quinidine, flecainide, moricizine, propafenone), thrombolytic agents (*e*.*g*., alteplase, reteplase, tenecteplase, anistreplase, streptokinase, urokinase), vasoconstrictors, vasodilators (*e*.*g*., hydralazine, minoxidil, mecamylamine, isorbide dintrate, isorbide mononitrate, nitroglycerin), cholesteryl ester transfer protein inhibitors (e.g., anacetrapib, evacetrapib), PPAR agonists (e.g., K-877, CER-002, DSP-8658, INT131 , GFT505), apoA-I activators (*e*.*g*., RVX-208), sphingosine-1-phosphate, retinoid X receptor (RXR) agonists (*e*.*g*., bexarotene, CD3254, docosahexaenoic acid, fluorobexarotene, isotretinoin, retinoic acid, SRI 1237, fenretinide, HX630, liarozole dihydrochloride, LG100754 and LG101506), liver X receptor (LXR) agonists (*e*.*g*., TO901317, ATI-111, LXR-623, XL-652, hypocholamide, GW3965, N,N-dimethyl-3beta-hydroxy-cholenamide (DMHCA), 22(R)-hydroxy cholesterol, 24(S)-hydroxy cholesterol, (- )anthrabenzoxocinone and (-) bischloroanthrabenzoxocinone ((-)-BABX)).

### 6.1.3.7. Caspase inhibitors

The Cargomers of the disclosure can include one or more caspase inhibitors (*e*.*g*., a caspase-1 inhibitor, a caspase-3 inhibitor, or a caspase-8 inhibitor). Caspase inhibitors may be useful for treating non-alcoholic fatty liver disease, epilepsy, ischemic disorders, Huntington's disease, amyotrophic lateral sclerosis (ALS), autoimmune diseases such as rheumatoid arthritis, osteoarthritis, inflammatory bowel disease, viral infections (*e*.*g*., hepatitis C) and sepsis. Exemplary caspase inhibitors that have been studied clinically and which can be included in Cargomers of the disclosure include emricasan, pralnacasan, and VX-765. Other caspase inhibitors that can be included in Cargomers are described in US 2010/0041661, WO/2001/021600, and US 9,365,612.

### 6.1.3.8. Bioactive agents

The Cargomers of the disclosure can include one or more bioactive agents, which are optionally deuterated. Highly deuterated bioactive agents can be useful for inhibiting biological processes. Exemplary bioactive agents include polyphenols, such as flavonoids (*e*.*g*., anthoxanthins such as luteolin, apigenin, tangeritin, quercetin, kaempferol, myricetin, fisetin, galangin, isorhamnetin, pachypodol, rhamnazin, pyranoflavonols, and furanoflavonols; flavanones such as hesperetin, naringenin, eriodictyol, and homoeriodictyol; flavanonols such as dihydroquercetin and dihydrokaempferol, flavans such as catechin, gallocatechin, catechin 3-gallate, gallocatechin 3-gallate, epicatechins, epigallocatechin, epicatechin 3-gallate, epigallocatechin 3-gallate, theaflavin, and leucoanthocyanidin), carotenoids (*e*.*g*., beta-carotene, alpha-carotene, beta-cryptoxanthin and gamma-carotene, lutein, lycopene, astaxanthin, zeaxanthin) and phytosterols.

### 6.1.3.9. Diagnostic agents

The Cargomers of the disclosure can include one or more imaging agents such as a fluorescent moiety, a phosphorescent moiety, gold, a radioactive moiety, a beta emitter, or a combination thereof. Suitable imaging agents include, but are not limited to, fluorescent molecules such as those described by Molecular Probes (Handbook of fluorescent probes and research products), such as Rhodamine, fluorescein, Texas red, Acridine Orange, Alexa Fluor (various), Allophycocyanin, 7-aminoactinomycin D, BOBO-1, BODIPY (various), Calcien, Calcium Crimson, Calcium green, Calcium Orange, 6-carboxyrhodamine 6G, Cascade blue, Cascade yellow, DAPI, DiA, DID, Di1, DiO, DiR, ELF 97, Eosin, ER Tracker Blue-White, EthD-1, Ethidium bromide, Fluo-3, Fluo4, FM1-43, FM4-64, Fura-2, Fura Red, Hoechst 33258, Hoechst 33342, 7-hydroxy-4-methylcoumarin, Indo-1, JC-1, JC-9, JOE dye, Lissamine rhodamine B, Lucifer Yellow CH, LysoSensor Blue DND-167, LysoSensor Green, LysoSensor Yellow/Blu, Lysotracker Green FM, Magnesium Green, Marina Blue, Mitotracker Green FM, Mitotracker Orange CMTMRos, MitoTracker Red CMXRos, Monobromobimane, NBD amines, NeruoTrace 500/525 green, Nile red, Oregon Green, Pacific Blue. POP-1, Propidium iodide, Rhodamine 110, Rhodamine Red, R-Phycoerythrin, Resorfin, RH414, Rhod-2, Rhodamine Green, Rhodamine 123, ROX dye, Sodium Green, SYTO blue (various), SYTO green (Various), SYTO orange (various), SYTOX blue, SYTOX green, SYTOX orange, Tetramethylrhodamine B, TOT-1, TOT-3, X-rhod-1, YOYO-1, YOYO-3. In some embodiments, ceramides are provided as imaging agents. In some embodiments, S1P agonists are provided as imaging agents. Additionally, radionuclides can be used as imaging agents. Suitable radionuclides include, but are not limited to radioactive species of Fe(III), Fe(II), Cu(II), Mg(II), Ca(II), and Zn(I1) Indium, Gallium and Technetium. For example, the imaging agent can be a radioactive species of iron, copper, magnesium, calcium, zinc, indium, gallium (*e.g.,* gallium-67), technetium, fluorine (*e.g.,* fluorine-18), krypton (*e.g.,* krypton-81), rubidium (*e.g.,* rubidium-82), nitrogen (*e.g.,* nitrogen-13), iodine (*e.g.* iodine-123), xenon (*e.g.* xenon-133), thallium (*e.g.* thallium-201), zirconium (*e.g.,* zirconium-89), or a combination thereof. In some embodiments, the imaging agent comprises zirconium-89.

Other suitable contrast agents include metal ions generally used for chelation in paramagnetic T1-type MIR contrast agents, and include di-and tri-valent cations such as copper, chromium, iron, gadolinium, manganese, erbium, europium, dysprosium and holmium. Metal ions that can be chelated and used for radionuclide imaging, include, but are not limited to metals such as gallium, germanium, cobalt, calcium, indium, iridium, rubidium, yttrium, ruthenium, yttrium, technetium, rhenium, platinum, thallium and samarium. Additionally metal ions known to be useful in neutron-capture radiation therapy include boron and other metals with large nuclear cross-sections. Also suitable are metal ions useful in ultrasound contrast, and X-ray contrast compositions. Examples of other suitable contrast agents include gases or gas emitting compounds, which are radioopaque.

Metal imaging agents (*e*.*g*., radioactive metals) can be attached to a Cargomer via a metal chelator, such as the bifunctional chelator p-isothiocyanatobenzyl desferrioxamine (Df-Bz-NCS). See, Zheng et al., 2016, Atherosclerosis 251 :381-388; Perez-Medina et al., 2015, Journal of Nuclear Medicine, 56(8):1272-1277; and Vosjan et al., 2010, Nat Protoc. 5(4):739-43. In some embodiments, the chelator is covalently attached to an amphipathic molecule (directly or through a linker). In other embodiments, the chelator is covalently attached to an apolipoprotein molecule (directly or through a linker).

### 6.1.3.10. Immunogens

The Cargomers can include one or more immunogens such as an antigen or an antigen-encoding nucleic acid. The antigen can be an antigen associated with an allergic reaction, for example, a pollen, a venom, animal dander, a fungal spore, a drug allergen or a food allergen. The antigen can be an autoantigen, for example, a lupus antigen, a multiple sclerosis antigen, a rheumatoid arthritis antigen, a diabetes mellitus type I antigen, an inflammatory bowel disease antigen, a thyroiditis antigen, or a celiac disease antigen.

The antigen can be, for example, a peptide based antigen, a protein based antigen, a polysaccharide based antigen, a saccharide based antigen, a lipid based antigen, a glycolipid based antigen, a nucleic acid based antigen, an inactivated organism based antigen, an attenuated organism based antigen, a viral antigen, a bacterial antigen, a parasite antigen, an antigen derived from an allergen, or a tumor antigen.

A peptide based antigen can be, for example, a retro-inverso peptide (e.g., as described in van Regenmortel et al., 1998, Dev Biol Stand. 92:139-43).

In some embodiments, the antigen is a self antigen, which is an immunogenic antigen or epitope native to a mammal and which may be involved in the pathogenesis of an autoimmune disease.

In some embodiments, the antigen is a viral antigen. Viral antigens can be isolated from any virus including, but not limited to, a virus from any of the following viral families: *Arenaviridae, Arterivirus, Astroviridae, Baculoviridae, Badnavirus, Barnaviridae, Birnaviridae, Bromoviridae, Bunyaviridae, Caliciviridae, Capillovirus*, *Carlavirus, Caulimovirus, Circoviridae, Closterovirus, Comoviridae, Coronaviridae* (*e.g*., Coronavirus, such as severe acute respiratory syndrome (SARS) virus), *Corticoviridae*, *Cystoviridae, Deltavirus, Dianthovirus, Enamovirus, Filoviridae* (*e.g*., Marburg virus and Ebola virus *(e.g.,* Zaire, Reston, Ivory Coast, or Sudan strain)), *Flaviviridae,* (*e.g*., Hepatitis C virus, Dengue virus 1, Dengue virus 2, Dengue virus 3, and Dengue virus 4), *Hepadnaviridae, Herpesviridae* (*e.g*., Human herpesvirus 1, 3, 4, 5, and 6, and Cytomegalovirus), *Hypoviridae, Iridoviridae, Leviviridae, Lipothrixviridae, Microviridae, Orthomyxoviridae* (*e.g.,* Influenzavirus A and B and C), *Papovaviridae, Paramyxoviridae* (*e.g*., measles, mumps, and human respiratory syncytial virus), *Parvoviridae, Picornaviridae* (*e.g*., poliovirus, rhinovirus, hepatovirus, and aphthovirus), *Poxviridae* (*e.g.*, vaccinia and smallpox virus), *Reoviridae* (*e.g*., rotavirus), *Retroviridae* (*e.g*., lentivirus, such as human immunodeficiency virus (HIV) 1 and HIV 2), *Rhabdoviridae* (for example, rabies virus, measles virus, respiratory syncytial virus, etc.), *Togaviridae* (for example, rubella virus, dengue virus, etc.), and *Totiviridae.* Suitable viral antigens also include all or part of Dengue protein M, Dengue protein E, Dengue D1NS1, Dengue D1NS2, and Dengue D1NS3.

Viral antigens may be derived from a particular strain such as a papilloma virus, a herpes virus, *i.e.* herpes simplex 1 and 2; a hepatitis virus, for example, hepatitis A virus (HAV), hepatitis B virus (HBV), hepatitis C virus (HCV), the delta hepatitis D virus (HDV), hepatitis E virus (HEV) and hepatitis G virus (HGV), the tick-borne encephalitis viruses; parainfluenza, varicella-zoster, cytomeglavirus, Epstein-Barr, rotavirus, rhinovirus, adenovirus, coxsackieviruses, equine encephalitis, Japanese encephalitis, yellow fever, Rift Valley fever, and lymphocytic choriomeningitis.

In some embodiments, the antigen is a bacterial antigen. Bacterial antigens can originate from any bacteria including, but not limited to, *Actinomyces, Anabaena, Bacillus*, *Bacteroides, Bdellovibrio, Bordetella, Borrelia, Campylobacter, Caulobacter, Chlamydia, Chlorobium, Chromatium, Clostridium, Corynebacterium, Cytophaga, Deinococcus, Escherichia, Francisella, Halobacterium, Heliobacter, Haemophilus, Hemophilus influenza* type B (HIB), *Hyphomicrobium, Legionella, Leptspirosis, Listeria, Meningococcus* A, B and C, *Methanobacterium, Micrococcus, Myobacterium, Mycoplasma, Myxococcus, Neisseria, Nitrobacter, Oscillatoria, Prochloron, Proteus, Pseudomonas, Phodospirillum, Rickettsia, Salmonella, Shigella, Spirillum, Spirochaeta, Staphylococcus, Streptococcus, Streptomyces, Sulfolobus, Thermoplasma, Thiobacillus*, and *Treponema, Vibrio,* and *Yersinia.*

In some embodiments, the antigen is a parasite antigen. Parasite antigens can be obtained from parasites such Cryptococcus neoformans, Histoplasma capsulatum, Candida albicans, Candida tropicalis, Nocardia asteroides, Rickettsia ricketsii, Rickettsia typhi, Mycoplasma pneumoniae, Chlamydial psittaci, Chlamydial trachomatis, Plasmodium falciparum, Trypanosoma brucei, Entamoeba histolytica, Toxoplasma gondii, Trichomonas vaginalis and Schistosoma mansoni. These include Sporozoan antigens, Plasmodian antigens, such as all or part of a Circumsporozoite protein, a Sporozoite surface protein, a liver stage antigen, an apical membrane associated protein, or a Merozoite surface protein.

In some embodiments, the antigen is an allergen and environmental antigen, such as, but not limited to, an antigen derived from naturally occurring allergens such as pollen allergens (tree-, herb, weed-, and grass pollen allergens), insect allergens (inhalant, saliva and venom allergens), animal hair and dandruff allergens, and food allergens. Important pollen allergens from trees, grasses and herbs originate from the taxonomic orders of Fagales, Oleales, Pinales and platanaceae including birch (*Betula*), alder (*Alnus*), hazel (*Corylus*), hornbeam (*Carpinus*) and olive (*Olea*), cedar (*Cryptomeria* and *Juniperus*), Plane tree (*Platanus*), the order of Poales including i.e. grasses of the genera *Lolium, Phleum, Poa, Cynodon, Dactylis, Holcus, Phalaris, Secale,* and *Sorghum,* the orders of Asterales and Urticales including i.a. herbs of the genera *Ambrosia, Artemisia,* and *Parietaria.* Other allergen antigens that may be used include allergens from house dust mites of the genus *Dermatophagoides* and *Euroglyphus,* storage mite *e.g Lepidoglyphys, Glycyphagus* and *Tyrophagus,* those from cockroaches, midges and fleas *e.g. Blatella, Periplaneta, Chironomus* and *Ctenocepphalides,* those from mammals such as cat, dog and horse, birds, venom allergens including such originating from stinging or biting insects such as those from the taxonomic order of Hymenoptera including bees (superfamily Apidae), wasps (superfamily Vespidea), and ants (superfamily Formicoidae). Still other allergen antigens that may be used include inhalation allergens from fungi such as from the genera *Alternaria* and *Cladosporium.*

In some embodiments, the antigen is a tumor antigen. The antigen can be a tumor antigen, including a tumor-associated or tumor-specific antigen or a peptide fragment that elicits an immune response against a tumor-associated or tumor-specific antigen. Examples of tumor-associated or tumor-specific antigens include, but are not limited to, alpha-actinin-4, Bcr-Abl fusion protein, Casp-8, beta-catenin, cdc27, cdk4, cdkn2a, coa-1, dek-can fusion protein, EF2, ETV6-AML1 fusion protein, LDLR-fucosyltransferaseAS fusion protein, HLA-A2, HLA-A11, hsp70-2, KIAAO205, Mart2, Mum-1, 2, and 3, neo-PAP, myosin class I, OS-9, pml-RARα fusion protein, PTPRK, K-ras, N-ras, Triosephosphate isomeras, Bage-1, Gage 3,4,5,6,7, GnTV, Herv-K-mel, Lage-1, Mage-A1,2,3,4,6,10,12, Mage-C2, NA-88, NY-Eso-1/Lage-2, SP17, SSX-2, and TRP2-Int2, MelanA (MART-I), gp100 (Pmel 17), tyrosinase, TRP-1, TRP-2, MAGE-1, MAGE-3, BAGE, GAGE-1, GAGE-2, p15(58), CEA, RAGE, NY-ESO (LAGS), SCP-1, Hom/Mel-40, PRAME, p53, H-Ras, HER-2/neu, BCR-ABL, E2A-PRL, H4-RET, IGH-IGK, MYL-RAR, Epstein Barr virus antigens, EBNA, human papillomavirus (HPV) antigens E6 and E7, TSP-180, MAGE-4, MAGE-5, MAGE-6, p185erbB2, p180erbB-3, c-met, nm-23H1, PSA, TAG-72-4, CA 19-9, CA 72-4, CAM 17.1, NuMa, K-ras, β-Catenin, CDK4, Mum-1, p16, TAGE, PSMA, PSCA, CT7, telomerase, 43-9F, 5T4, 791Tgp72, α-fetoprotein, 13HCG, BCA225, BTAA, CA 125, CA 15-3 (CA 27.29\BCAA), CA 195, CA 242, CA-50, CAM43, CD68\KP1, CO-029, FGF-5, G250, Ga733 (EpCAM), HTgp-175, M344, MA-50, MG7-Ag, MOV18, NB\70K, NY-CO-1, RCAS1, SDCCAG16, TA-90 (Mac-2 binding protein\cyclophilin C-associated protein), TAAL6, TAG72, TLP, and TPS.

In some embodiments, the antigen is PCSK9.

In some embodiments, the antigen is gp100 melanoma.

In some embodiments, the antigen is a neo-antigen. The term neo-antigen is used herein to define any newly expressed antigenic determinant. Neo-antigens may arise upon conformational change in a protein, as newly expressed determinants (especially on the surfaces of transformed or infected cells), as the result of complex formation of one or more molecules or as the result of cleavage of a molecule with a resultant display of new antigenic determinants. Thus, as used herein, the term neo-antigen covers antigens expressed upon infection (*e*.*g*. viral infection, protozoal infection or bacterial infection), in prion-mediated diseases, an on cell transformation (cancer), in which latter case the neo-antigen may be termed a tumor-associated antigen.

Identification of neo-antigens can involve identifying all, or nearly all, mutations in the neoplasia/tumor at the DNA level using whole genome sequencing, whole exome (*e.g*., only captured exons) sequencing, or RNA sequencing of tumor versus matched germline samples from each patient. In some embodiments, identification of neo-antigens involves analyzing the identified mutations with one or more peptide-MHC binding prediction algorithms to generate a plurality of candidate neo-antigen T cell epitopes that are expressed within the neoplasia/tumor and may bind patient HLA alleles. In some embodiments, identification of neo-antigens involves synthesizing the plurality of candidate neo-antigen peptides selected from the sets of all neo open reading frame peptides and predicted binding peptides for use in a cancer vaccine. Several contract research organizations offer neo-antigen identification services, including Personalis (www.personalis.com), BGI (www.bgi.com), and Cancer Genetics Incorporated (www.cancergenetics.com).

In some embodiments, the antigen is a shared antigen, *i.e*., an antigen overexpressed in malignant cell but that also exists in non-malignant cells.

In certain embodiments the size of a neo-antigenic peptide molecule or shared antigen peptide molecule can be, for example, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 60, about 70, about 80, about 90, about 100, about 110, about 120 or greater amino molecule residues, and any range derivable therein. In specific embodiments the neo-antigenic or shared antigenic peptide molecules are equal to or less than 50 amino acids. In some embodiments, the neo-antigenic peptide or shared antigenic peptide molecules are equal to about 10 to 30 amino acids. In some embodiments, the neo-antigenic peptide molecules or shared antigenic peptide molecules are equal to about 10 to about 20 amino acids. In a preferred embodiment, the neo-antigenic peptide molecules or shared antigenic peptide molecules are equal to about 20 to about 30 amino acids.

Cargomers can include one or more neo-antigenic peptides. In some embodiments, a Cargomer comprises one neo-antigenic peptide. In some embodiments, a Cargomer comprises two neo-antigenic peptides. In some embodiments, a Cargomer comprises at least 5 or more neo-antigenic peptides. In some embodiments, a Cargomer comprises at least about 6, about 8, about 10, about 12, about 14, about 16, about 18, or about 20 distinct peptides. In some embodiments, a Cargomer comprises at least 20 distinct peptides.

Cargomers can include one or more shared antigen peptides. In some embodiments, a Cargomer comprises one shared antigen peptide. In some embodiments, a Cargomer comprises two shared antigen peptides. In some embodiments, a Cargomer comprises at least 5 or more shared antigen peptides. In some embodiments, a Cargomer comprises at least about 6, about 8, about 10, about 12, about 14, about 16, about 18, or about 20 distinct peptides. In some embodiments, a Cargomer comprises at least 20 distinct peptides.

Neo-antigenic and shared antigenic peptides, polypeptides, and analogs can be further modified to contain additional chemical moieties not normally part of the protein. Those derivatized moieties can improve the solubility, the biological half-life, absorption of the protein, or binding affinity. The moieties can also reduce or eliminate any desirable side effects of the proteins and the like. An overview for those moieties can be found in Allen et al., eds., 2012, Remington: The Science and Practice of Pharmacy, 22nd Edition, Pharmaceutical Press, London, UK. For example, neo-antigenic and shared peptides and polypeptides having the desired activity may be modified as necessary to provide certain desired attributes, *e*.*g*. improved pharmacological characteristics, while increasing or at least retaining substantially all of the biological activity of the unmodified peptide to bind the desired MHC molecule and activate the appropriate T cell. For instance, the neo-antigenic or shared antigenic peptides and polypeptides may be subject to various changes, such as substitutions, either conservative or non-conservative, where such changes might provide for certain advantages in their use, such as improved MHC binding. Such conservative substitutions may encompass replacing an amino acid residue with another amino acid residue that is biologically and/or chemically similar, *e*.*g*., one hydrophobic residue for another, or one polar residue for another. The effect of single amino acid substitutions may also be probed using D- amino acids. Such modifications may be made using well known peptide synthesis procedures.

In some embodiments, the neo-antigenic or shared antigenic peptides and polypeptides may be modified with linking agents for purposes of facilitating complexing with the Cargomer. The disclosure is not limited to a particular type or kind of linking agent. In some embodiments, the linking agent is a cysteine-serine-serine (CSS) molecule.

In some embodiments wherein the neo-antigenic or shared antigenic peptide or polypeptide is modified with CSS, the Cargomer is further modified with dioleoyl-sn-glycero-3-phosphoethanolamine-N-[3-(2-pyridyldithio) propionate] (DOPE-PDP) wherein upon mixing, the DOPE-PDP and CSS engage thereby resulting in a complexing (linking) of the CSS-Ag with the Cargomer.

The neo-antigenic or shared antigenic peptides and polypeptides may also be modified by extending or decreasing the compound's amino acid sequence, *e*.*g*., by the addition or deletion of amino acids. The neo-antigenic or shared antigenic peptides, polypeptides, or analogs can also be modified by altering the order or composition of certain residues. It will be appreciated by the skilled artisan that certain amino acid residues essential for biological activity, *e*.*g*., those at critical contact sites or conserved residues, may generally not be altered without an adverse effect on biological activity. The non-critical amino acids need not be limited to those naturally occurring in proteins, such as L-a- amino acids, or their D-isomers, but may include non-natural amino acids as well, such as β-γ-δ- amino acids, as well as many derivatives of L-a-amino acids. The non-critical amino acids can be engineered so that the peptide is a substrate for a protease, which can promote liberation of the peptide within cells. Cysteine residues at non-critcal amino acids (e.g., added to the N-terminus or C-terminus) can facilitate the coupling to an anchor (*e*.*g*., a sterol, phospholipid, or fatty acid). Such peptides can be liberated in a reducing environment.

Typically, a neo-antigen or shared antigen polypeptide or peptide may be optimized by using a series of peptides with single amino acid substitutions to determine the effect of electrostatic charge, hydrophobicity, etc. on MHC binding. For instance, a series of positively charged (*e.g*., Lys or Arg) or negatively charged (*e.g*., Glu) amino acid substitutions may be made along the length of the peptide revealing different patterns of sensitivity towards various MHC molecules and T cell receptors. In addition, multiple substitutions using small, relatively neutral moieties such as Ala, Gly, Pro, or similar residues may be employed. The substitutions may be homo-oligomers or hetero-oligomers. The number and types of residues which are substituted or added depend on the spacing necessary between essential contact points and certain functional attributes which are sought (e.g., hydrophobicity versus hydrophilicity). Increased binding affinity for an MHC molecule or T cell receptor may also be achieved by such substitutions, compared to the affinity of the parent peptide. In any event, such substitutions should employ amino acid residues or other molecular fragments chosen to avoid, for example, steric and charge interference which might disrupt binding. Amino acid substitutions are typically of single residues. Substitutions, deletions, insertions or any combination thereof may be combined to arrive at a final peptide.

One of skill in the art will appreciate that there are a variety of ways in which to produce such tumor specific neo-antigens or shared antigens. In general, such tumor specific neo-antigens and shared antigens may be produced either *in vitro* or *in vivo.* Tumor specific neo-antigens and shared antigens may be produced *in vitro* as peptides or polypeptides, which may then be formulated into a personalized neoplasia vaccine and administered to a subject. Such in vitro production may occur by a variety of methods known to one of skill in the art such as, for example, peptide synthesis or expression of a peptide/polypeptide from a DNA or RNA molecule in any of a variety of bacterial, eukaryotic, or viral recombinant expression systems, followed by purification of the expressed peptide/polypeptide.

Alternatively, tumor specific neo-antigens may be produced *in vivo* by introducing molecules (*e*.*g*., DNA, RNA, viral expression systems, and the like) that encode tumor specific neo- antigens into a subject, whereupon the encoded tumor specific neo-antigens are expressed.

In some embodiments, Cargomers can comprise one or more neoantigens (*e*.*g*., a peptide of 10 to 30 amino acid residues) identified in subjects having esophageal cancer attached to an anchor (*e*.*g*., a sterol, a phospholipid or a fatty acid) which can be used, for example, for treating esophageal cancer (*e*.*g*., adenocarcinoma, squamous cell carcinoma or others). The one or more neoantigens can be directly attached to an anchor or through a linker, *e*.*g*., as described in Section 6.1.5.

In some embodiments, Cargomers can comprise one or more neoantigens (*e*.*g*., a peptide of 10 to 30 amino acid residues) identified in subjects having pancreatic cancer attached to an anchor (*e*.*g*., a sterol, a phospholipid or a fatty acid), which can be used, for example, for treating pancreatic cancer. The one or more neoantigens can be directly attached to an anchor or through a linker, *e*.*g*., as described in Section 6.1.5.

In some embodiments, Cargomers can comprise one or more shared antigens (*e.g*., a peptide of 10 to 30 amino acid residues) identified in subjects having cancer (*e.g.* pancreatic cancer) attached to an anchor (*e*.*g*., a sterol, a phospholipid or a fatty acid), which can be used, for example, for treating subjects having cancer (*e*.*g*., pancreatic cancer). The one or more shared antigens can be directly attached to an anchor or through a linker, *e*.*g*., as described in Section 6.1.5.

In some embodiments, Cargomers can comprise one or more neoantigens (*e.g*., a peptide of 10 to 30 amino acid residues) identified in subjects having pancreatic cancer attached to an anchor (*e*.*g*., a sterol, a phospholipid or a fatty acid), which can be used, for example, for treating pancreatic cancer. The one or more neoantigens can be directly attached to an anchor or through a linker, *e*.*g*., as described in Section 6.1.5.

In some embodiments, Cargomers can comprise one or antigens (*e*.*g*., a peptide of 10 to 30 amino acid residues) from a mutated RAS protein (e.g., KRAS, HRAS, or NRAS) linked to an anchor (*e*.*g*., a sterol, a phospholipid or a fatty acid), which can be used, for example, for treating pancreatic cancer. The one or more antigens can be directly attached to an anchor or through a linker, *e*.*g*., as described in Section 6.1.5.

Proteins or peptides may be made by any technique known to those of skill in the art, including the expression of proteins, polypeptides or peptides through standard molecular biological techniques, the isolation of proteins or peptides from natural sources, or the chemical synthesis of proteins or peptides. The nucleotide and protein, polypeptide and peptide sequences corresponding to various genes have been previously disclosed, and may be found at computerized databases known to those of ordinary skill in the art. One such database is the National Center for Biotechnology Information's Genbank and GenPept databases located at the National Institutes of Health website. The coding regions for known genes may be amplified and/or expressed using the techniques disclosed herein or as would be known to those of ordinary skill in the art. Alternatively, various commercial preparations of proteins, polypeptides and peptides are known to those of skill in the art.

Antigens can be provided as single antigens or can be provided in combination. Antigens can also be provided as complex mixtures of polypeptides or nucleic acids.

### 6.1.4. Anchors

A cargo moiety can be covalently bound to an amphipathic or apolar moiety to facilitate coupling of the cargo moiety to a Cargomer. Amphipathic and apolar moieties can interact with apolar regions in Cargomers, thereby anchoring cargo moieties attached to amphipathic and apolar moieties to Cargomers.

Amphipathic moieties that can be used as anchors include lipids (*e*.*g*., as described in Section 6.1.2.1) and fatty acids (*e*.*g*., as described in Section 6.1.2.3). In some embodiments, the anchors comprise a sterol or a sterol derivative e.g., a plant sterol, an animal sterol, or a sterol derivative such as a vitamin). For example, sterols such as cholesterol can be covalently bound to a cargo moiety (*e*.*g*., via the hydroxyl group at the 3-position of the A-ring of the sterol) and used to anchor the cargo moiety to a Cargomer. Apolar moieties that can be used as anchors include alkyl chains, acyl chains, and diacyl chains. Cargo moieties can be covalently bound to anchor moieties directly or indirectly via a linker (*e*.*g*., via a difunctional peptide or other linker described in Section 6.1.5). Cargo moieties that are biologically active may retain their biological activity while covalently bound to the anchor (or linker attached to the anchor), while others may require cleavage of the covalent bond (*e*.*g*., by hydrolysis) attaching the cargo moiety to the anchor (or linker attached to the anchor) to regain biological activity.

### 6.1.5. Linkers

Linkers comprise a chain of atoms that covalently attach cargo moieties to other moieties in a Cargomer, including apolipoprotein molecules, amphipathic molecules, and anchors. A number of linker molecules are commercially available, for example from ThermoFisher Scientific. Suitable linkers are well known to those of skill in the art and include, but are not limited to, straight or branched-chain carbon linkers, heterocyclic carbon linkers, and peptide linkers. A linker can be a bifunctional linker, which is either homobifunctional or heterobifunctional.

Suitable linkers include cleavable and non-cleavable linkers.

A linker may be a cleavable linker, facilitating release of a cargo moiety *in vivo.* Cleavable linkers include acid-labile linkers (*e*.*g*., comprising hydrazine or cis-aconityl), protease-sensitive (*e.g*., peptidase-sensitive) linkers, photolabile linkers, or disulfide-containing linkers (Chari et al., 1992, Cancer Research 52:127-131; U.S. Patent No. 5,208,020). A cleavable linker is typically susceptible to cleavage under intracellular conditions. Suitable cleavable linkers include, for example, a peptide linker cleavable by an intracellular protease, such as lysosomal protease or an endosomal protease. In exemplary embodiments, the linker can be a dipeptide linker, such as a valine-citrulline (val-cit), a phenylalanine-lysine (phe-lys), or a serine-serine linker.

A cleavable linker can be pH-sensitive, *i.e*., sensitive to hydrolysis at certain pH values. Typically, a pH-sensitive linker is hydrolyzable under acidic conditions. For example, an acid-labile linker that is hydrolyzable in the lysosome (*e*.*g*., a hydrazone, semicarbazone, thiosemicarbazone, cis-aconitic amide, orthoester, acetal, ketal, or the like) can be used. (See, *e*.*g*., U.S. Patent Nos. 5,122,368; 5,824,805; 5,622,929; Dubowchik and Walker, 1999, Pharm. Therapeutics 83:67-123; Neville et al., 1989, Biol. Chem. 264:14653-14661). Such linkers are relatively stable under neutral pH conditions, such as those in the blood, but are unstable at below pH 5.5 or 5.0, the approximate pH of the lysosome. In certain embodiments, the hydrolyzable linker is a thioether linker (such as, *e.g.,* a thioether attached to the cargo moiety via an acylhydrazone bond (see, *e.g.,* U.S. Patent No. 5,622,929).

In some embodiments, the linker is cleavable under reducing conditions (*e.g.,* a disulfide linker). A variety of disulfide linkers are known in the art, including, for example, those that can be formed using SATA (N-succinimidyl-5-acetylthioacetate), SPDP (N-succinimidyl-3-(2-pyridyldithio)propionate), SPDB (N-succinimidyl-3-(2-pyridyldithio)butyrate) and SMPT (N-succinimidyloxycarbonyl-alpha-methyl-alpha-(2-pyridyl-dithio)toluene), SPDB and SMPT (see, *e.g.,* Thorpe et al., 1987, Cancer Res. 47:5924-5931; Wawrzynczak et al., In Immunoconjugates: Antibody Conjugates in Radioimagery and Therapy of Cancer (C.W. Vogel ed., Oxford U. Press, 1987. See also, U.S. Patent No. 4,880,935).

In some embodiments, the linker is cleavable by a cleaving agent, *e.g.,* an enzyme, that is present in the intracellular environment (*e.g.,* within a lysosome or endosome or caveolea). The linker can be, *e.g.,* a peptidyl linker that is cleaved by an intracellular peptidase or protease enzyme, including, but not limited to, a lysosomal or endosomal protease. In some embodiments, the peptidyl linker is at least two amino acids long or at least three amino acids long. Cleaving agents can include cathepsins B and D and plasmin, all of which are known to hydrolyze dipeptide drug derivatives resulting in the release of active drug inside target cells (see, *e.g.,* Dubowchik and Walker, 1999, Pharm. Therapeutics 83:67-123). In some embodiments, the peptidyl linker cleavable by an intracellular protease is a Val-Cit linker or a Phe-Lys linker.

In some embodiments, the linker is a malonate linker (Johnson et al., 1995, Anticancer Res. 15:1387-93), a maleimidobenzoyl linker (Lau et al., 1995, Bioorg-Med-Chem. 3(10):1299-1304), or a 3'-N-amide analog (Lau et al., 1995, Bioorg-Med-Chem. 3(10): 1305-12).

In other embodiments, the linker unit is not cleavable and the cargo moiety is released, for example, by Cargomer degradation. Exemplary non-cleavable linkers include maleimidocaproyl, N-succinimidyl 4-(maleimidomethyl)cyclohexanecarboxylate (SMCC) and N-succinimidyl-4-(iodoacetyl)-aminobenzoate (SlAB).

### 6.2. Compositions comprising Cargomers

The disclosure provides compositions comprising Cargomers of the disclosure. Pharmaceutical compositions can include Cargomers and one or more pharmaceutically acceptable carriers, excipients, diluents, or a combination thereof. The Cargomers of the disclosure can also be included in a vaccine composition comprising Cargomers and one or more pharmaceutically acceptable carriers, diluents, excipients, adjuvants, or a combination thereof. The Cargomers of the disclosure can also be included in a diagnostic composition comprising Cargomers of and one or more carriers, diluents, excipients, or a combination thereof which are suitable for diagnostic use.

Exemplary carriers include solvents or dispersion media containing, for example, water, ethanol, a polyol (for example, glycerol, propylene glycol, and liquid polyethylene glycol, and the like), suitable mixtures thereof, and vegetable oils. Exemplary diluents include water for injection, saline solution, buffered solutions such as phosphate buffered saline solution, and sugar solutions such as sucrose or dextran solutions. Exemplary excipients include fillers, binders, disintegrants, solvents, solubilizing agents, and coloring agents. Exemplary excipients also include positively charged molecules such as, but not limited to, lysine, arginine, ornithine, poly-lysine, poly-arginine, poly-ornithine, poly-lys-arg, and poly-lys-orn.

Exemplary adjuvants include CPG, polylC, poly-ICLC, 1018 ISS, aluminum salts, Amplivax, AS15, BCG, CP-870,893, CpG7909, CyaA, dSLIM, GM-CSF, IC30, IC31, Imiquimod, ImuFact IMP321, IS Patch, ISS, ISCOMATRIX, Juvlmmune, LipoVac, MF59, monophosphoryl lipid A, Montanide IMS 1312, Montanide ISA 206, Montanide ISA 50V, Montanide ISA-51, OK-432, OM-174, OM-197-MP-EC, ONTAK, PepTel.RTM, vector system, PLGA microparticles, imiquimod, resiquimod, gardiquimod, 3M-052, SRL172, Virosomes and other Virus-like particles, YF-17D, VEGF trap, beta-glucan, Pam3Cys, Aquila's QS21 stimulon, vadimezan, and AsA404 (DMXAA).

The compositions of the disclosure can be formulated according to techniques known in the art (*e.g.,* as described in Allen et al., eds., 2012, Remington: The Science and Practice of Pharmacy, 22nd Edition, Pharmaceutical Press, London, UK). For example, the compositions can be formulated for subcutaneous, intradermal, intravenous, or intraperitoneal injection (*e.g.,* as a solution), inhalation (intranasal or intrapulmonary inhalation), implantation *(e.g.,* as a suppository), ocular or intraocular administration (*e.g.,* via eye drops).

In some embodiments, the compositions are packaged in unit dosage amounts suitable for administration. For example, in some embodiments, the compositions comprise unit dosage amounts of dried (for example lyophilized) Cargomers packaged in sealed vials. Such compositions are suitable for reconstitution with water, physiological solution (such as saline) or buffer, and administration via injection. Such compositions may optionally include one or more anti-caking and/or anti-agglomerating agents to facilitate reconstitution of the Cargomers, or one or more buffering agents, isotonicity agents (e.g., sucrose and/or mannitol), sugars or salts (e.g., sodium chloride) designed to adjust the pH, osmolality and/or salinity of the reconstituted suspension. The compositions described above can be manufactured under conditions that minimize oxidation, thereby reducing the risk of side effects, such as liver damage, caused by oxidized products. For example, the compositions can be manufactured under an inert gas, such as nitrogen, helium, or argon.

Cargomers may also be formulated in pharmaceutical compositions for controlled release. As used herein, "controlled release" refers to release of a cargo moiety from a formulation at a rate that the blood concentration of the cargo moiety in an individual is maintained within the therapeutic range for an extended duration, over a time period on the order of hours, days, weeks, or longer. Cargomers may be formulated in a bioerodible or nonbioerodible controlled matrix, a number of which are well known in the art. A controlled release matrix may include a synthetic polymer or copolymer, for example in the form of a hydrogel. Examples of such polymers include polyesters, polyorthoesters, polyanhydrides, polysaccharides, poly(phosphoesters), polyamides, polyurethanes, poly(imidocarbonates) and poly(phosphazenes), and poly-lactide-co-glycolide (PLGA), a copolymer of poly(lactic acid) and poly(glycolic acid). Collagen, albumin, and fibrinogen containing materials may also be used.

Preferably, the compositions of the disclosure contain only a small amount of uncomplexed amphipathic molecules, apolipoprotein, and cargo moieties. In some embodiments, no more than 20% of the amphipathic molecules in the composition are in uncomplexed form. In other embodiments, no more than 10% of the amphipathic molecules are in uncomplexed form. In yet other embodiments, no more than 5% of the amphipathic molecules are uncomplexed form. In yet other embodiments, no more than 2% of the amphipathic molecules are in uncomplexed form.

The homogeneity of the Cargomers and compositions of the disclosures can be measured by gel permeation chromatography. A highly homogeneous composition will generally have a main peak corresponding to the Cargomers and, possibly, one or more secondary peaks corresponding to one or more of free protein, free amphipathic molecules, and free cargo moieties. Secondary peaks corresponding to Cargomers or complexes having a different size from the Cargomers in the main peak may also be seen. The area of the main peak on a gel permeation chromatogram relative to the total area of the main and secondary peaks determines the percent homogeneity of a composition. In some embodiments, the compositions of the disclosure are at least 75% homogeneous. In other embodiments, the compositions of the disclosure are at least 85% homogeneous. In other embodiments, the compositions of the disclosure are at least 95% homogeneous. In yet other embodiments, the compositions of the disclosure are at least 98% homogeneous.

In some embodiments, the homogeneity of the Cargomers in the compositions of the disclosure (*i.e.,* the area of the main peak relative to the total area of the main and secondary peaks corresponding to Cargomers having both apolipoprotein and amphipathic molecules) is at least 75%. In other embodiments, the Cargomers in the compositions of the disclosure are at least 85% homogeneous. In other embodiments, the Cargomers in the compositions of the disclosure are at least 95% homogeneous. In other embodiments, the Cargomers in the compositions of the disclosure are at least 98% homogeneous.

In the compositions of the disclosure, lipoprotein complexes that (a) have Stokes radii of greater than 2 nm or 3.4 nm *(e.g.,* as determined by gel permeation chromatography) and/or (b) are discoidal and/or (c) have an apolipoprotein:amphipathic molecule molar ratio of 1:8 or greater, if present, preferably represent no more than 10% of the apolipoprotein in the composition on a weight basis. In some embodiments, such complexes represent no more than 5% of the apolipoprotein in the composition on a weight basis. In some embodiments, such complexes represent no more than 2% of the apolipoprotein in the composition on a weight basis. In some embodiments, the compositions are free of detectable lipoprotein complexes that (a) have Stokes radii of greater than 3.4 nm and/or (b) are discoidal and/or (c) have an apolipoprotein:amphipathic molecule molar ratio of 1:8 or greater when the compositions are subjected to gel permeation chromatography under conditions capable of resolving such complexes. Preferably, the compositions of the disclosure are free of lipoprotein complexes having a Stokes radius of greater than 3.25 nm.

The identity and amount of lipoprotein molecules in a composition of Cargomers can be determined, for example, by mass spectrometry (see, e.g., Zhang et al., 2010, Methods Mol Biol. 673: 211-222) and in particular FT-MS or by NMR. The identity and amount of amphipathic molecules in a composition of Cargomers can be determined, for example, by thin layer chromatography (see, *e.g.,* Clogston and Patri, 2011, Methods Mol Biol. 697:109-17). The presence of discoidal particles in a composition of Cargomers can be determined, for example, using NMR spectroscopy.

### 6.3. Uses of the Cargomers

The following methods and uses are to be construed as directed to the Cargomers for use in the said methods and uses.

### 6.3.1. Therapeutic Uses of the Cargomers

The Cargomers and pharmaceutical compositions of the disclosure can be used to treat a subject for a disease or condition treatable with the one or more cargo moieties. For example, a Cargomer having a cargo moiety which is an anti-cancer agent can be used to treat a subject afflicted with cancer. The methods of treatment can comprise administering a therapeutically effective amount of a Cargomer or a pharmaceutical composition containing the Cargomer to the subject alone or in combination with a therapeutically effective amount of a second Cargomer of the disclosure or composition comprising the second Cargomer (*e.g.,* a second pharmaceutical composition or a vaccine composition). Preferably, the Cargomer and second Cargomer comprise different cargo moieties. The Cargomer and the second Cargomer (or compositions thereof) can be administered sequentially or simultaneously. When administered simultaneously, Cargomers having different cargo moieties can be formulated into a single composition or be formulated in two separate compositions.

Cargomers and Cargomer pharmaceutical compositions can be administered to a subject according to any suitable administration regimen, for example, weekly, twice a week, or daily. Cargomers and Cargomer pharmaceutical compositions can be administered for a set number of doses, (*e.g.,* 1 to 24 doses, 1 to 12 doses, or 12 to 24 doses), such as when the Cargomers contain an anti-infective agent, or can be administered until a disease or condition that the subject is afflicted with subsides. In some embodiments, administration of a Cargomer or Cargomer pharmaceutical composition can be repeated after verification that a biological marker of a disease or condition has regressed.

Cargomers comprising an immunogen and vaccine compositions of the disclosure can be used to immunize a subject (or induce tolerance to an antigen in a subject) by administering an effective amount of such a Cargomer or vaccine composition to the subject. The methods of immunizing a subject can comprise administering an effective amount of the Cargomer or vaccine composition alone or in combination with an effective amount of a second Cargomer of the disclosure or composition comprising the second Cargomer. Preferably, the Cargomer and second Cargomer comprise different cargo moieties. The Cargomer and the second Cargomer (or compositions thereof) can be administered sequentially or simultaneously. When administered simultaneously, Cargomers having different cargo moieties can be formulated into a single composition or be formulated in two separate compositions.

Cargomers configured to activate an immune response and vaccine compositions containing such Cargomers are useful for treating a subject having or being predisposed to any disease or disorder to which the subject's immune system mounts an immune response. The compositions are useful as prophylactic vaccines, which confer resistance in a subject to subsequent exposure to infectious agents. The compositions are also useful as therapeutic vaccines, which can be used to initiate or enhance a subject's immune response to a pre-existing antigen, such as a tumor antigen in a subject with cancer, or a viral antigen in a subject infected with a virus. For example, subjects having or at risk of cancer such as esophageal cancer can be treated with a Cargomer containing a neoantigen peptide. As another example, subjects having or at risk of a cancer such as pancreatic cancer can be treated with a Cargomer containing a shared antigen peptide. Exemplary shared antigen peptides include peptides from a mutated RAS protein (e.g., KRAS, HRAS, or NRAS), and peptides derived therefrom, e.g., peptides comprising a neoantigen peptide sequence and an added N-terminal or C-terminal amino acid sequence useful for coupling the peptide to an amphipathic molecule or anchor. Exemplary shared antigen based peptides that can be included in Cargomers of the disclosure are shown in SEQ ID NOs:15-28.

The compositions are also useful as desensitizing vaccines, which function to make an individual tolerant to an environmental antigen, such as an allergen.

Cargomers comprising a neo-antigenic peptide can be used to induce a neoplasia/tumor specific immune response in a subject, vaccinate against a neoplasia/tumor, treat and/or alleviate a symptom of cancer in a subject by administering to the subject a therapeutically effective amount of such a Cargomer or a vaccine composition comprising the Cargomer. Such Cargomers and vaccine compositions may be used for a subject that has been diagnosed as having cancer, or at risk of developing cancer. In some embodiments, the subject has a solid tumor such as breast, ovarian, prostate, lung, kidney, gastric, esophageal, colon, testicular, head and neck, pancreas, brain, melanoma, and other tumors of tissue organs and hematological tumors, such as lymphomas and leukemias, including acute myelogenous leukemia, chronic myelogenous leukemia, chronic lymphocytic leukemia, T cell lymphocytic leukemia, and B cell lymphomas. The Cargomer or vaccine composition can be administered in an amount sufficient to induce a CTL response, alone or in combination with other therapeutic agents (*e.g.,* a chemotherapeutic or biotherapeutic agent, radiation, immunotherapy, or an anti-immunosuppressive or immunostimulatory agent). For example, a subject having melanoma can be administered a Cargomer or vaccine composition of the disclosure in combination with another therapeutic agent used to treat melanoma (e.g., aldesleukin, cobimetinib, dabrafenib, dacarbazine, talimogene laherparepvec, recombinant interferon alfa-2b, ipilimumab, pembrolizumab, trametinib, nivolumab, peginterferon alfa-2a, peginterferon alfa-2b, or orvemurafenib).

In some embodiments, a Cargomer or vaccine composition of the disclosure is administered to a subject diagnosed as having cancer or at risk of developing cancer in combination with an immunotherapy agent. Exemplary immunotherapy agents include alemtuzumab, atezolizumab, ipilimumab, ofatumumab, nivolumab, pembrolizumab, rituximab, and rurvalumab.

In some embodiments, a Cargomer or vaccine composition of the disclosure is administered to a subject diagnosed as having cancer or at risk of developing cancer in combination with an immunotherapy agent which is a checkpoint inhibitor. Exemplary checkpoint inhibitors include atezolizumab, ipilimumab, pembrolizumab, and nivolumab.

In some embodiments, a Cargomer or vaccine composition of the disclosure is administered to a subject diagnosed as having cancer or at risk of developing cancer in combination with an antibody-drug conjugate (ADC) comprising an anti-cancer agent (e.g., as described in Section 6.1.3.2). Exemplary ADCs include gemtuzumab ozogamicin (approved to treat acute myeloid leukemia), brentuximab vedotin (approved to treat Hodgkin lymphoma), trastuzumab emtansine (approved to treat HER2-positive metastatic breast cancer), and Inotuzumab ozogamicin (approved to treat acute lymphoblastic leukemia).

Subjects with or at risk for immunosuppressed conditions can be treated therapeutically or prophylactically with Cargomers configured to activate an immune response as disclosed herein. The Cargomers disclosed herein can be used for treatment of disease conditions characterized by immunosuppression, including, but not limited to, AIDS or AIDS-related complex, idiopathic immuno suppression, drug induced immunosuppression, other virally or environmentally-induced conditions, and certain congenital immune deficiencies. Such Cargomers can also be employed to increase immune function that has been impaired by the use of radiotherapy of immunosuppressive drugs (e.g., certain chemotherapeutic agents), and therefore can be particularly useful when used in conjunction with such drugs or radiotherapy.

Subjects with or at risk for coronary heart disease and/or elevated LDL-C levels can be treated therapeutically or prophylactically with Cargomers configured to activate an immune response as disclosed herein. Embodiments of the disclosure wherein Cargomers include a PCSK9-antigen and a CpG-adjuvant address such needs. Indeed, vaccination against PCSK9 with such Cargomers can be used to inhibit interaction between PCSK9 and LDLR, while avoiding the need for repeated injections of expensive mAb.

Subjects having or at risk for coronary heart disease and/or elevated LDL-C levels can be treated with a Cargomer containing a siRNA against a PCSK9 gene and/or an apoB gene.

Subjects having or at risk for Huntington's disease can be treated with a Cargomer containing a siRNA against a dysfunctional huntingtin gene.

Subjects having or at risk of Alzheimer's disease can be treated with a Cargomer containing a siRNA against an amyloid precursor protein gene.

The Cargomers and compositions can be administered in the methods of the disclosure to a subject (which is preferably a mammal and most preferably a human) by any suitable route. For example, administration can be via injection (*e.g.,* subcutaneous, intradermal, intravenous, or intraperitoneal injection), inhalation (*e.g.*, intranasal or intrapulmonary inhalation, implantation, optionally (*e.g.,* via a suppository), or ocular or intraocular routes (*e.g.,* via eye drops). In some embodiments, the solution is administered as a depot injection. In some embodiments, a Cargomer composition is administered as a perfusion over 15 to 24 hours (*e.g.,* 15 minutes to 1 hour, 1 hour to 3 hours, 3 hours to 6 hours, 6 hours to 12 hours, or 12 hours to 24 hours).

### 6.3.2. Diagnostic Uses of the Cargomers

Cargomers of the disclosure comprising a diagnostic agent and diagnostic compositions comprising such Cargomers can be used for diagnosing a subject with a disease or condition or evaluating the effect of a treatment on the subject. For example, Cargomers of the disclosure comprising an imaging agent can be used in place of conventional imaging agents in an imaging procedure (*e.g.,* CT scan or MRI scan).

### 6.3.2.1. Methods of imaging a tumor

The disclosure provides methods of using Cargomers to image tumors. In one aspect, a method for imaging a tumor comprises administering a Cargomer comprising an imaging agent (*e.g.,* zirconium-89) to a subject and subsequently imaging the subject to detect delivery of the imaging agent to the tumor. The Cargomer can be administered by any suitable means prior to the imaging, *e.g.,* orally or intravenously. In a preferred embodiment, the Cargomer is administered intravenously. The imaging can be performed on a part of the subject's body which is known to contain a tumor (*e.g.,* an organ or region of the body, such as abdomen, head, or neck), or can comprise a full body scan (which can be useful, for example, to identify a secondary tumor).

The amount of time between administration of the Cargomer and the imaging of the subject typically ranges from less than 1 hour *(e.g.,* 15 to 45 minutes, 15 to 30 minutes, or 30 to 45 minutes) to several days (*e.g.,* 3 days to 4 days). In some embodiments, the imaging of the subject is performed from 30 minutes to 4 days after administration of the Cargomer (*e.g.,* 1 hour to 3 days, 1 hour to 1 day, or 1 day to 3 days). In specific embodiments, the imaging is performed 1 hour after administration of the Cargomer, 1 day after administration of the Cargomer, 2 days after administration of the Cargomer, 3 days after administration of the Cargomer, or 4 days after administration of the Cargomer.

The type of imaging procedure used to detect delivery of the imaging agent to the tumor will depend upon the type of imaging agent in the Cargomer. For example, nuclear imaging scans can be used to detect imaging agents comprising a radioactive moiety. Examples of nuclear imaging scans that can be used to detect delivery of a radioactive moiety to a tumor include positron emission tomography (PET), positron emission tomography-computed tomography (PET-CT), scintigraphy (scint), single-photon emission computed tomography (SPECT), and single-photon emission computed tomography-computed tomography (SPECT-CT). When the imaging agent comprises a radioactive moiety, the amount of Cargomers administered to the subject per administration can contain, for example, 5 Mbq to 20 Mbq of radioactivity (*e.g.,* 5 Mbq to 10 Mbq, 10 Mbq to 15 Mbq, or 15 Mbq to 20 Mbq). In some embodiments, the amount of labeled Cargomers administered to the subject per administration contains 10 Mbq to 18 Mbq of radioactivity.

Magnetic resonance imaging (MRI) (e.g., dynamic contrast enhanced magnetic resonance imaging (DCE-MRI)) can be used when the imaging agent comprises a contrast agent, and fluorescence imaging (FI) can be used when the imaging agent comprises a fluorescent moiety.

In some embodiments, an imaging technique for detecting delivery of the imaging agent to a tumor is performed in combination with a second imaging technique (*e.g.,* shortly before or after the first imaging technique). For example, dynamic contrast enhanced magnetic resonance imaging (DCE-MRI) can be used to evaluate tumor microcirculation.

### 6.3.2.2. Methods of monitoring tumor progression, regression, or recurrence

The disclosure further provides methods of using labeled Cargomers to monitor tumor progression, regression, or recurrence. In one aspect, the methods comprise administering a Cargomer comprising an imaging agent to the subject in a first administration and imaging the subject to detect delivery of the imaging agent to the tumor (e.g., as described in Section 6.3.2.1), and administering the Cargomer comprising an imaging agent to the subject in a second administration and imaging the subject to detect delivery of the imaging agent to the tumor (*e.g.,* as described in Section 6.3.2.1). Repeating the administration of Cargomer comprising an imaging agent and imaging can be used to monitor the progression of a tumor (*e.g.,* in the absence of any treatment or in response to a treatment), regression of a tumor (*e.g.,* in response to a treatment), or recurrence of a tumor (*e.g.,* after surgery to remove the tumor or after cessation of an anti-cancer treatment). As used herein, the expression "surgery to remove a tumor," and the like encompasses surgeries in which all or only part of a tumor are removed from the subject.

The first administration of the Cargomer can be performed, for example, prior to surgery to remove the tumor and/or prior to beginning treatment with an anti-cancer therapy, thereby providing a baseline by which to evaluate the success of the surgery and/or efficacy of treatment. Alternatively, the first administration of the Cargomer can be performed after a surgery to remove the tumor and/or after beginning treatment with an anti-cancer therapy, thereby providing a baseline by which to evaluate tumor recurrence and/or efficacy or continued efficacy of the anti-cancer therapy.

The second administration of the Cargomer can be performed, for example, after surgery to remove the tumor and/or after the subject has begun receiving treatment with an anti-cancer therapy, thereby allowing for an evaluation of the success of the surgery and/or efficacy of the anti-cancer therapy. The administration of the Cargomer comprising an imaging agent and imaging can be repeated one or more times (*e.g.,* once every week to once every month) to further monitor progression, regression, or recurrence of a tumor. The administration of the Cargomer comprising an imaging agent and imaging can be repeated, for example, for a set period of time, indefinitely, or until the occurrence of a specific event (*e.g.,* remission of the cancer or when the tumor becomes undetectable by the imaging).

The methods for monitor tumor progression, regression, or recurrence can be used to guide treatment for the subject. For example, for a patient receiving treatment with an anti-cancer therapy, if the imaging indicates a decrease in tumor size from the first administration of the Cargomer comprising an imaging agent to the second administration of the Cargomer comprising an imaging agent, it may be advantageous for the patient to continue receiving the anti-cancer therapy. However, if the imaging does not indicate a decrease in tumor size (or indicates an increase) from the first administration of the Cargomer comprising an imaging agent to the second administration of the Cargomer comprising an imaging agent, it may be advantageous to alter the treatment that the subject is receiving (*e.g.,* to discontinue treatment of the anti-cancer therapy, increase the dose of the anti-cancer therapy and/or begin treatment with a different anti-cancer therapy, for example begin treatment with a Cargomer comprising an anti-cancer agent which is different from a Cargomer comprising an anti-cancer agent which the subject has already received). After continuing or altering treatment, the administration of the Cargomer comprising an imaging agent and imaging can be repeated one or more times (*e.g.,* as described in the preceding paragraph) to further monitor the efficacy of the continued or altered treatment.

### 6.3.2.3. Methods for selecting a subject afflicted with a cancer for a treatment

The disclosure further provides methods for selecting a subject afflicted with a cancer for treatment with a Cargomer comprising an anti-cancer agent. In one aspect, a method for selecting a subject afflicted with a cancer for treatment with a Cargomer comprising an anti-cancer agent comprises administering a Cargomer comprising an imaging agent to the subject and imaging the subject to detect delivery of the imaging agent to the tumor (*e.g.,* as described in Section 6.3.2.1), and selecting the subject for treatment with a Cargomer comprising an anti-cancer agent if the imaging shows delivery of the imaging agent to the tumor. If the imaging does not show delivery of the imaging agent to the tumor, an alternative treatment can be selected for the subject (*e.g.,* with one or more of the anti-cancer therapies described in Section 6.1.3.2).

In some embodiments, the subject is selected to receive treatment with a Cargomer comprising an anti-cancer agent as monotherapy. In other embodiments, the subject is selected to receive treatment with a Cargomer comprising an anti-cancer agent in combination with a second anti-cancer therapy. The second anti-cancer therapy can be, for example, any of the anti-cancer therapies identified in Section 6.1.3.2. In some embodiments, the second anti-cancer therapy comprises a second Cargomer comprising a second anti-cancer agent. The subject can be selected to receive the treatment with a Cargomer comprising an anti-cancer agent prior to, concurrently with, or subsequent to the treatment with the second anti-cancer therapy. The term "concurrently with" is not limited to treatment regimens in which the two therapies are administered to the subject in a single administration, but encompasses regimens in which the therapeutic effect of the two therapies overlaps.

In the methods for selecting a subject afflicted with a cancer for treatment with a Cargomer comprising an anti-cancer agent described herein, the subject can be selected to receive the treatment with a Cargomer comprising an anti-cancer agent which begins before or after surgery to remove the tumor. In some embodiments, the subject can be selected to receive treatment with a Cargomer comprising an anti-cancer agent which begins before surgery to remove the tumor and continues after the surgery. In other embodiments, the subject can be selected to receive the treatment with a Cargomer comprising an anti-cancer agent in the absence of surgery to remove the tumor (*e.g.,* when the subject's course of treatment does not include a surgery to remove the tumor, for example, when the tumor is inoperable).

### 6.3.2.4. Methods of treating a subject afflicted with cancer following tumor imaging

The disclosure further provides methods of treating a subject afflicted with cancer following tumor imaging. In one aspect, the methods of treating a subject afflicted with a cancer comprise administering a Cargomer comprising an imaging agent to the subject and imaging the subject to detect delivery of the imaging agent to the tumor (*e.g.,* as described in Section 6.3.2.1), and administering a Cargomer comprising an anti-cancer agent to the subject if the imaging shows delivery of the imaging agent to the tumor. If the imaging does not show delivery of the imaging agent to the tumor, an alternative treatment can be selected for the subject (*e.g.,* with one or more of the anti-cancer therapies described in Section 6.1.3.2).

In some embodiments, the subject is administered treatment with a Cargomer an anti-cancer agent as monotherapy. In other embodiments, the subject is administered treatment with a Cargomer comprising an anti-cancer agent in combination with a second anti-cancer therapy. The second anti-cancer therapy can be, for example, any of the anti-cancer therapies identified in Section 6.1.3.2. In some embodiments, the second anti-cancer therapy comprises a second Cargomer comprising a second anti-cancer agent.

In the methods for treating a subject afflicted with a cancer described herein, the subject can be administered a Cargomer comprising an anti-cancer agent for the first time before or after surgery to remove the tumor. In some embodiments, the subject can begin receiving treatment with a Cargomer comprising an anti-cancer agent before surgery to remove the tumor and continue receiving the Cargomer comprising an anti-cancer agent after the surgery. In other embodiments, the subject can be administered a Cargomer comprising an anti-cancer agent in the absence of surgery to remove the tumor (e.g., when the subject's course of treatment does not include a surgery to remove the tumor, for example, when the tumor is inoperable).

### 7. EXEMPLARY METHODS FOR PREPARING CARGOMERS

Cargomers having a defined number of apolipoprotein molecules can be prepared in an aqueous solution by defining the appropriate pH, the concentration of apolipoprotein monomers and amphipathic molecules, the ionic strength and the temperature to make a solution of "empty" Cargomers. Cargo moieties can be added to the solution of empty Cargomers as a solution or as a powder in order to make loaded Cargomers having a defined molar ratio of apolipoprotein to cargo moieties. The loaded Cargomers can be used directly after being formed or can be purified in order to separate loaded Cargomers from unbound molecules (*e.g.,* unbound cargo moieties).

When cargo molecules are not easily soluble in aqueous solutions, the apolipoprotein, amphipathic molecules and the cargo moieties can be solubilized in an organic solvent solution in order to make a homogeneous solution and avoid any aggregates or precipitates. Then the solvent can be removed by any suitable technique known in the art for removing a solvent from a solution, such as but not limited to evaporation, freeze-drying, spray-drying, etc. in order to make a solid powder or film in which the apolipoprotein, amphipathic molecules, and cargo moieties have been intimately mixed. An aqueous solution with the appropriate pH and ionic strength can be added to the powder or film to solubilize the molecules to spontaneously form loaded Cargomers at the appropriate concentrations. Addition of the aqueous solution can be done at a fixed temperature or, right after the addition, the mixture can be heated at a temperature up to 80 degrees Celsius. The mixture can also be thermal cycled (*e.g.,* between 2 and 10 cycles) between a low temperature (*e.g.,* no lower than 10 degrees Celsius) and a higher temperature (*e.g.,* not exceeding 80 degrees Celsius) to form the Cargomers. Thermal cycling methods have been described for making discoidal lipoprotein complexes (see, WO 2012/109162), and those methods can similarly be used to make Cargomers. Among organic solvents, para-xylene, ortho-xylene or acetic acid solutions are preferred for freeze drying. In some embodiments, acetic acid solutions from 70 to 100% are used. Other solvents or mixtures of solvents can be used and be later eliminated by different techniques for removing solvents that are well-known in the art. Solvents can be selected in order to solubilize each component of the Cargomers. In the case of a mixture of solvents, they should be miscible together. After hydration (addition of the aqueous solution) the Cargomers can be purified by any appropriate technique known in the art such as but not limited to chromatography, filtration, electrophoresis, etc. in order to eliminate unbound material or used without further separation/purification. After hydration (addition of the aqueous solution), pH can be adjusted and/or ionic strength can be adjusted, and/or osmolality can be adjusted.

In one embodiment, the following steps can be followed in order to prepare Cargomers comprising multimeric apolipoprotein. 1) Select conditions that favor formation of multimeric apolipoprotein (*e.g.,* ApoA-I), *e.g.,* select a concentration of apolipoprotein, temperature, ionic strength of the aqueous solvent used, and pH that favor multimeric forms (see Section 6.1). 2) Confirm the presence of multimeric apolipoprotein by an analytical method, *e.g.,* dynamic light scattering (DLS), static light scattering, size exclusion chromatography, or gel electrophoresis. See, *e.g.,* Schonfeld et al., 2016, J. Phys. Chem. B, 120:1228-1235; Jayaraman et al., 2011, Journal of Biological Chemistry, 286(41):35610-35623; Gianazza et al., 1997, Biochemistry, 36:7898-7905. Such analysis should be performed using conditions that will not perturb the existence of the multimeric apolipoprotein. For example, significantly diluting a sample and/or adding the sample to a high salt buffer for analysis can cause dissociation of multimeric apolipoprotein. 3) To the apolipoprotein solution, add one or more cargo moieties (optionally attached to anchors) and an amount of amphipathic molecules sufficient to solubilize the apoliprotein molecules. The one or more of the cargo moieties and the one or more of the amphipathic molecules can be be the same molecules or different molecules. The addition of each ingredient can be done sequentially, in mixture, or all at once. The multimeric form of apolipoprotein can be determined for the solution of apolipoprotein alone or after the mixing of apolipoprotein with one or more ingredients. The mixture containing all Cargomer components can be incubated (*e.g.,* at a single temperature or thermal cycled) and/or mixed to promote Cargomer formation. Functional assays can be used to test and select the best loaded Cargomers from a structure point of view, an homogeneity point of view, or from an efficacy point of view.

### 8. EXAMPLES

### 8.1. Example 1: ApoA-I/DPPG/Sphingomyelin Cargomers

Equimolar amounts of 1,2-dipalmitoyl-*sn*-glycero-3-[phospho-*rac*-(1-glycerol)] (DPPG) and sphingomyelin (SM) are weighed, solubilized in CHCl₃ and dried under a stream of N₂. The lipid film is dispersed in 10 mM phosphate buffer pH 8.0 at 37°C using a high sheer Ultra-Turax T25 mixer for 5 min. The solution is kept under a nitrogen overlay during preparation.

A solution comprising a therapeutic agent is made by dissolving an amount of the therapeutic agent in water.

A solution of purified human ApoA-I is thawed at room temperature. The ApoA-I solution, DPPG:SM solution, and therapeutic agent solution are mixed to provide a mixture having ApoA-I, DPPG, and SM at a 1:2:2 molar ratio, and the therapeutic agent. The mixture is heated to 37°C for 4 hours. The mixture is then subjected to thermal cycling (three cycles of 20 minutes at 57°+/-2 °C and 5 minutes at 37° +/- 2 °C) to complete formation of the Cargomers. After completion of the thermal cycling, the Cargomer solution is cooled down and stored at 4°C.

### 8.2. Example 2: Study of Cargomers loaded with tumor antigen peptides and cholesterol-CpG oligonucleotides in a syngeneic mouse cancer model

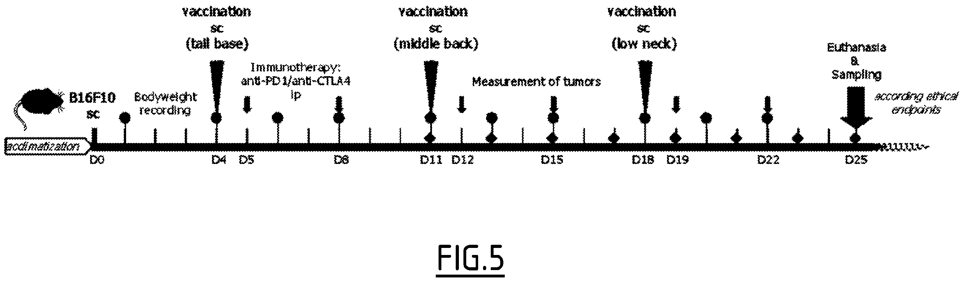

The purpose of this study is to evaluate the efficacy of Cargomers loaded with tumor antigen (TA) peptides and cholesterol-CpG oligonucleotides (chol-CpG) in a syngeneic mouse cancer model. The scheme for the study is shown in Fig. 5.

### 8.2.1. Materials and Methods

### 8.2.1.1. Cargomers

Cargomers comprising ApoA-I and chol-CpG (5'-T*C*C*A*T*G*A*C*G*T*T*C*C*T*G*A*C*G*T*T/3ChoITEG/-3' (SEQ ID NO: 11)) at a 10:1 molar ratio and Cargomers comprising ApoA-I and TA peptides M27 (LCPGNKYEM (SEQ ID NO:12)), M30 (CSSVDWENVSPELNSTDQ (SEQ ID NO:13)), and TRP2 (CSVYDFFVWL (SEQ ID NO:14)) at an ApoA-I:TA peptide molar ratio of 1:2 and 1:4 were prepared. To make the Cargomers comprising TA peptides, the TA peptides were first reacted with PDP-PE lipid (1,2-dipalmitoyl-sn-glycero-3-phosphoethanolamine-N-(3-(2-pyridyldithio) propionate), sodium salt) and then incorporated into the Cargomers. The Cargomers were stored at 4°C after preparation and were allowed to stand at room temperature prior to use. The Cargomers containing chol-CpG and the Cargomers containing the TA peptides were mixed prior to administration.

### 8.2.1.2. Mice

64 female C57BU6 mice 5 weeks of age and weighing 18-22 g at the beginning of the study were used in the study. Animals were housed in cages in 8 groups of 8 animals. Animals were allowed a minimum acclimatization period of 4 days in the housing room prior to initiation of the study. During acclimatization and for the duration of the study, feed and water were offered to the mice *ad libitum.*

### 8.2.1.3. Cell Culture and Tumor Induction

B16F10 murine melanoma cells were cultured in Dulbecco modified Eagle medium supplemented with 10% fetal bovine serum and 2 mM glutamine and maintained in a humidified atmosphere of 5 % CO₂ in air at 37°C. Tumor induction was performed by implanting 1×10⁵ B16F10 murine melanoma cells by subcutaneous injection on the right flank of each animal. Cells for implanatation were prepared in PBS and a final volume of 0.1 mL of cell suspension was injected into each mouse. Implantation was done on Day 0 after shaving and depilation of the animals at Day -1.

Tumor engraftment was monitored by daily observation and palpation. Caliper measurement was performed every other day. Tumor size in mm³ was estimated from the formula *w²* x *l x **π***/*6* where "*l*" is the longest diameter of the tumor and "*w*" is the diameter perpendicular to the longest diameter measured in millimeters.

### 8.2.1.4. Treatments

The 8 groups of mice received one of 8 treatments as shown in Table 2:

| **Group** | **Vaccination (SC)** | | | | | **Immunotherapy (IP)** |
|---|---|---|---|---|---|---|
| | **Formulation** | **Dose (nmol per animal)** | | | **Volume administered** | |
| | | **ApoA-I** | **Chol-CpG** | **TA** | | |
| 1 | Vehicle (PBS) | 0 | 0 | 0 | 0.1 mL | No |
| 2 | ApoA-I | 26 | 0 | 0 | 0.1 mL | No |
| 3 | TAs + Chol-CpG (non-Cargomer form) | 0 | 2.3 | 15 | 0.1 mL | No |
| 4 | Cargomers (1:2)¹ | 30.5 | 2.3 | 15 | 0.1 mL | No |
| 5 | Cargomers (1:4)² | 26.75 | 2.3 | 15 | 0.1 mL | No |

| | | | | | | |
|---|---|---|---|---|---|---|
| 6 | Cargomers (1:2)¹ | 30.5 | 2.3 | 15 | 0.1 mL | Yes |
| 7 | Cargomers (1:4)² | 26.75 | 2.3 | 15 | 0.1 mL | Yes |
| 8 | Vehicle (PBS) | 0 | 0 | 0 | - | Yes |
| Table 2: Treatment Groups | | | | | | |
| ¹ mixture of ApoA-I:chol-CpG (10:1) Cargomers and ApoA-I:TA (1:2) Cargomers | | | | | | |
| ² mixture of ApoA-I:chol-CpG (10:1) Cargomers and ApoA-I:TA (1:4) Cargomers | | | | | | |

Vaccination was performed once a week for 3 weeks (at days 4, 11 and 18) by subcutaneous injection. Immunotherapy consisting of anti-CTLA4 and anti-PD1 antibodies administered by intraperitoneal injection (100 µg of each antibody in a final volume of 0.2 mL in PBS per mouse) was performed twice a week for 3 weeks (at days 5, 8, 12, 15, 19 and 22).

### 8.2.1.5. Results

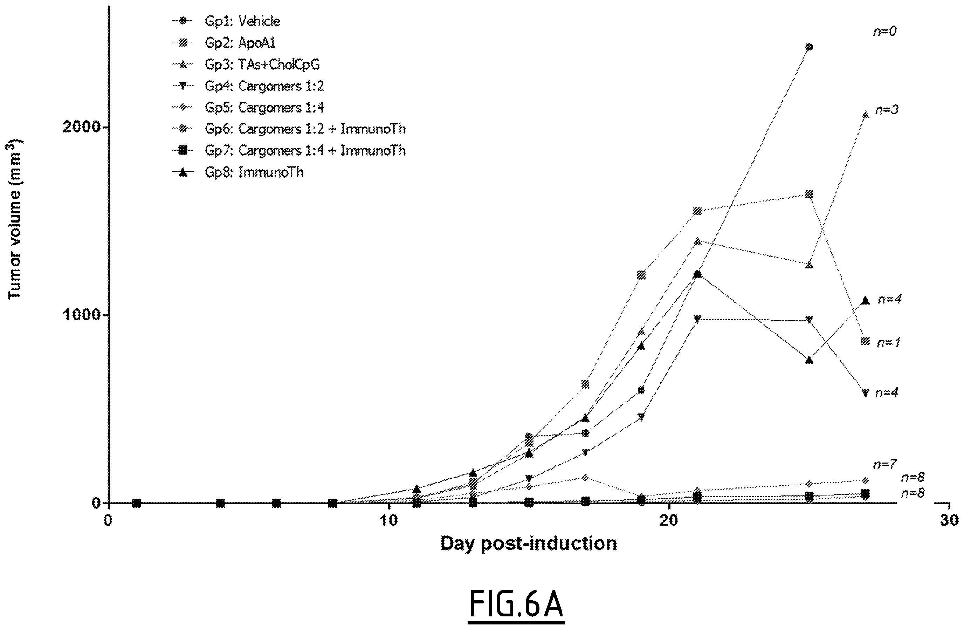

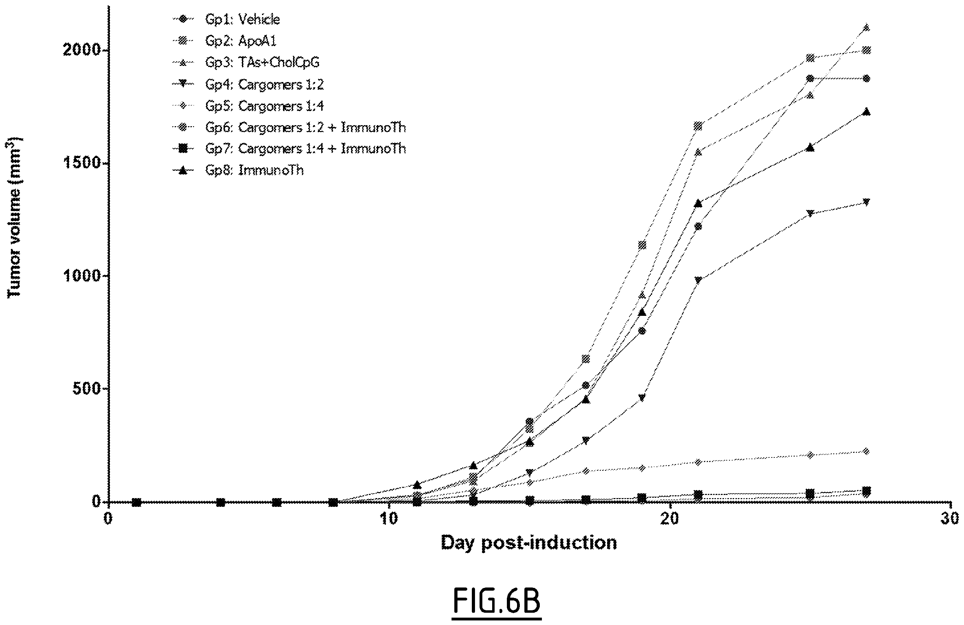

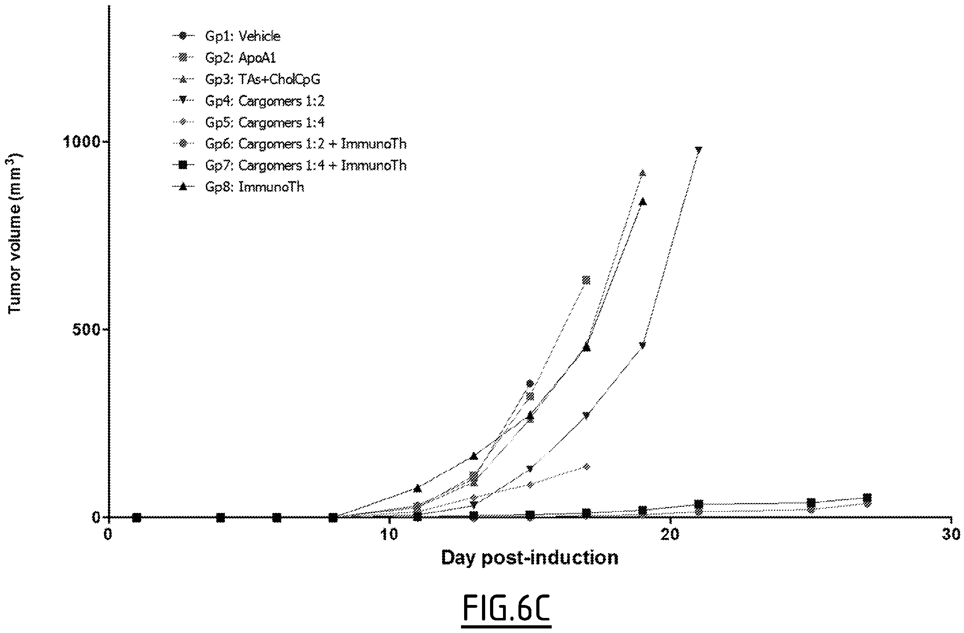

Tumor volumes for each of the 8 treatment groups during the course of the study are shown in Figs. 6A-6C. Figs. 6A-6C show that the two treatments comprising a combination of Cargomers having chol-CpG and TAs and immunotherapy greatly outperformed all other treatments.

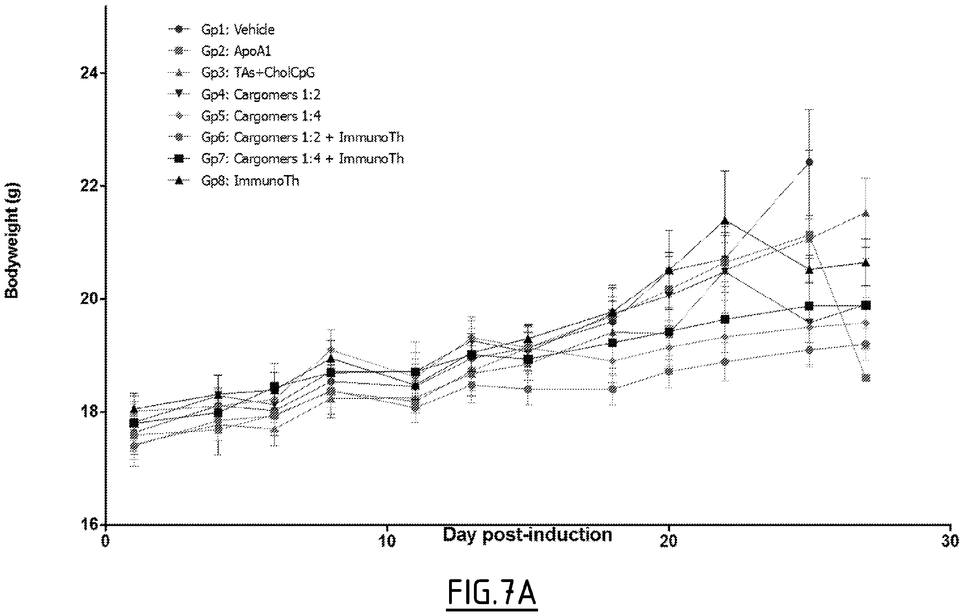

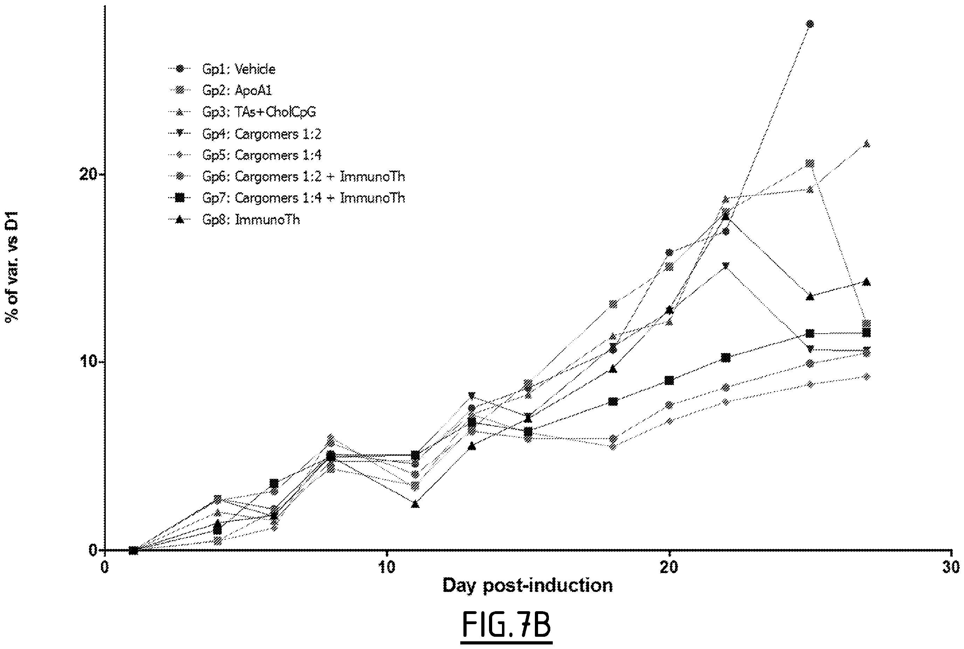

Bodyweights and baseline-corrected bodyweights for the treatment groups are shown in Fig. 7A and Fig. 7B, respectively.

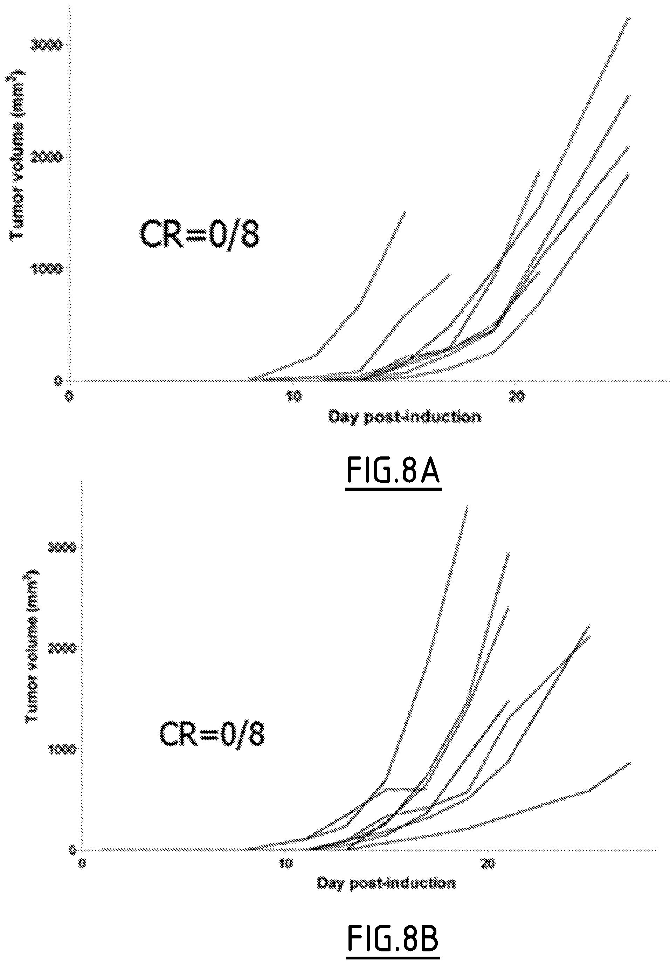

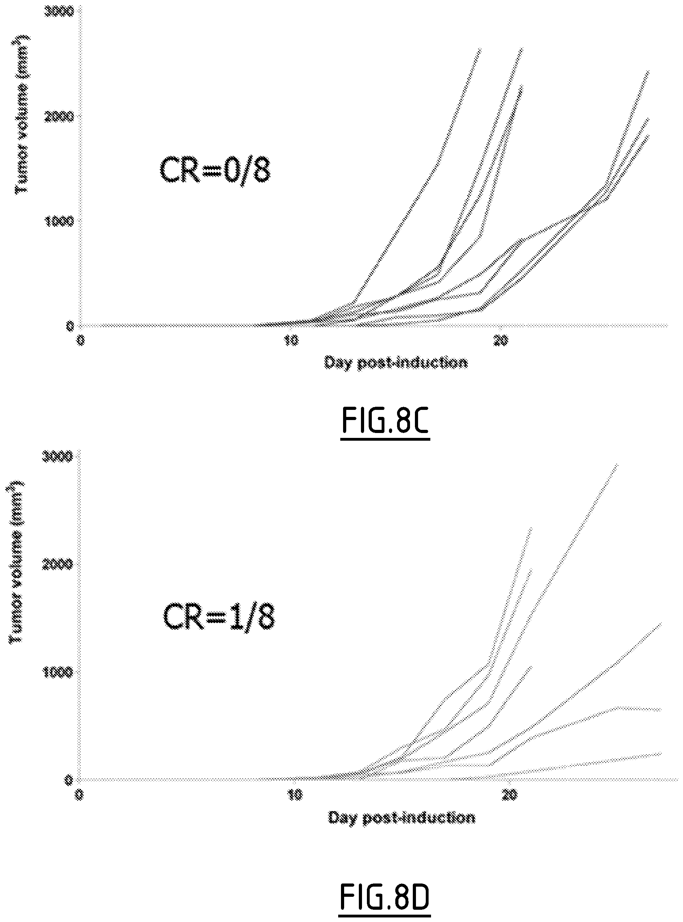

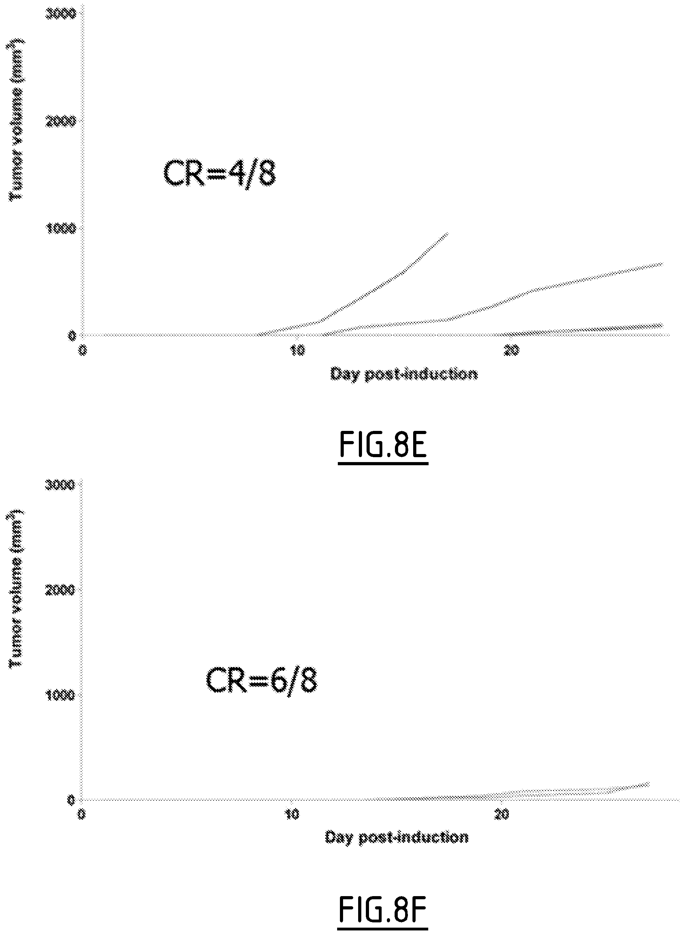

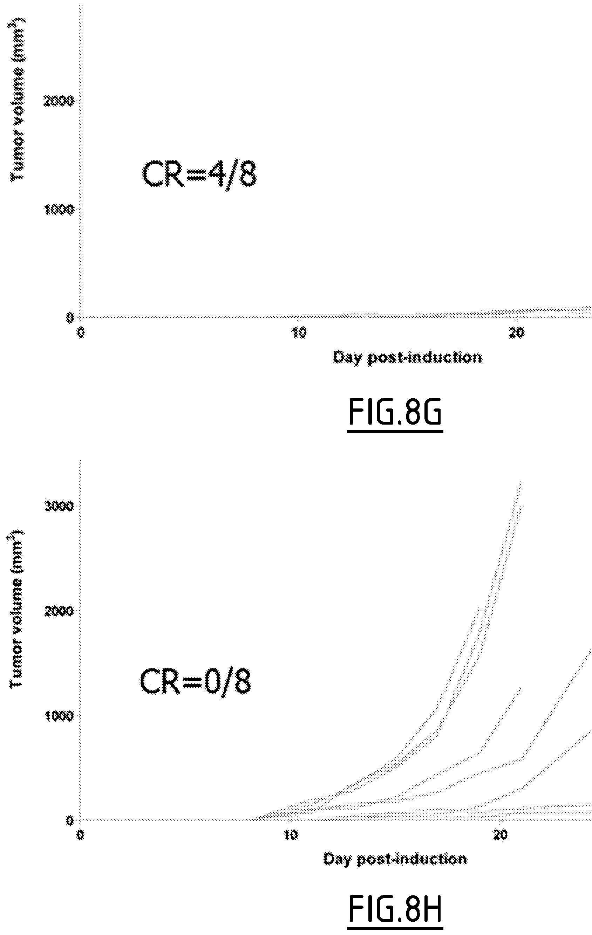

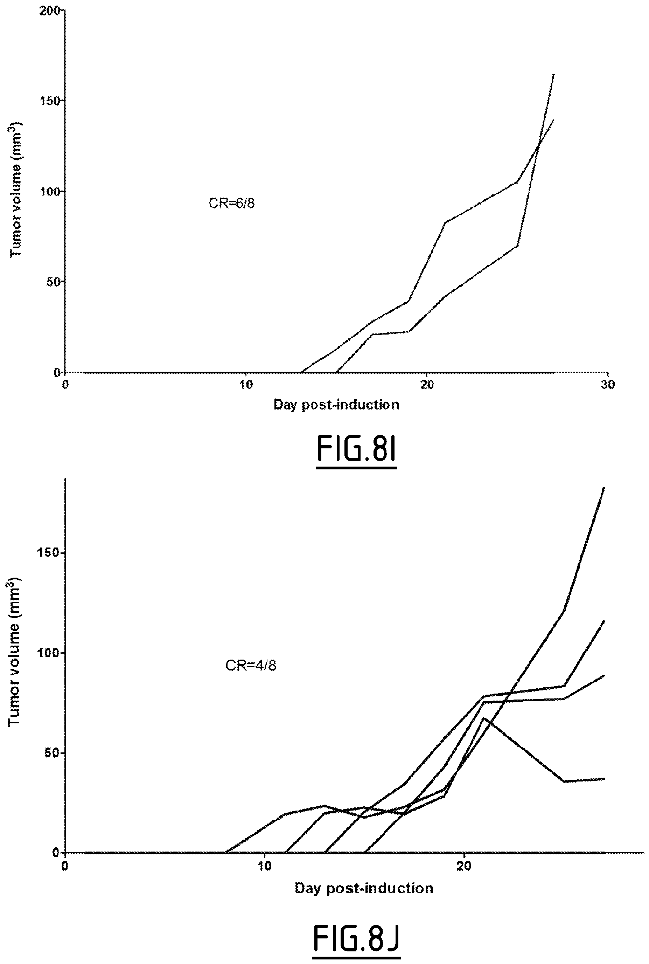

Individual tumour growth curves with fraction of complete tumour regression (CR) for the 8 treatment groups are shown in Figs. 8A-8H, with the growth curves for groups 6 and 7 expanded on the Y axis in Fig. 8I and Fig. 8J, respectively.

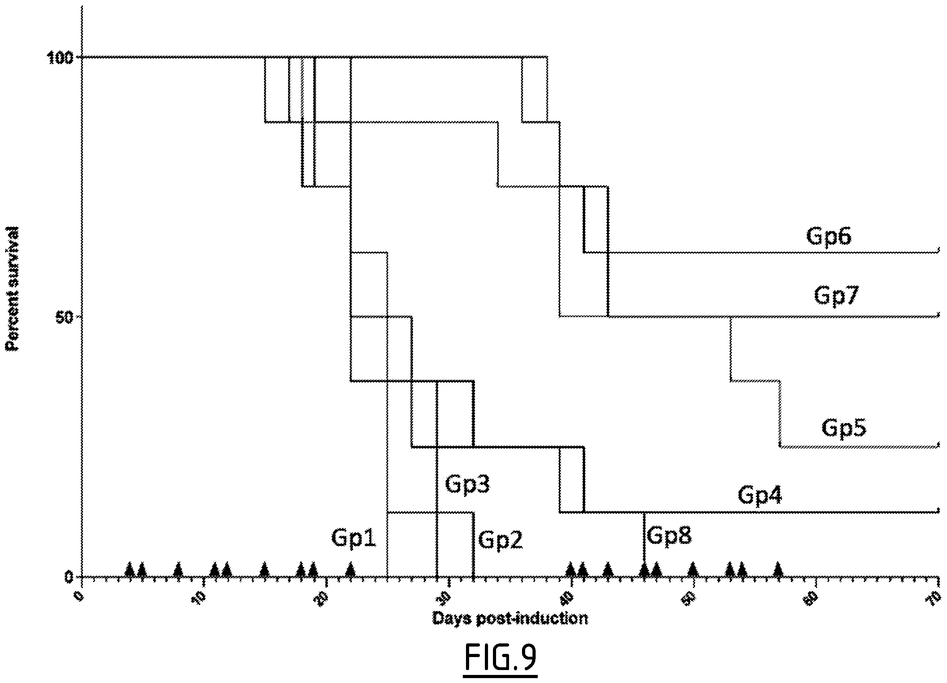

The survival proportion for each treatment group of is shown in Fig. 9.

Thus, the results show that Cargomers comprising chol-CpG and TAs can be successfully used to deliver chol-CpG and TAs for cancer immunotherapy.

### 8.3. Example 3: Follow-up study with surviving mice from Example 2

Surviving animals from Example 2 which were treated with Cargomers (with or without check point inibitor treatment) were injected with new tumor cells and followed over time. Naive mice of same age were added as a control group.

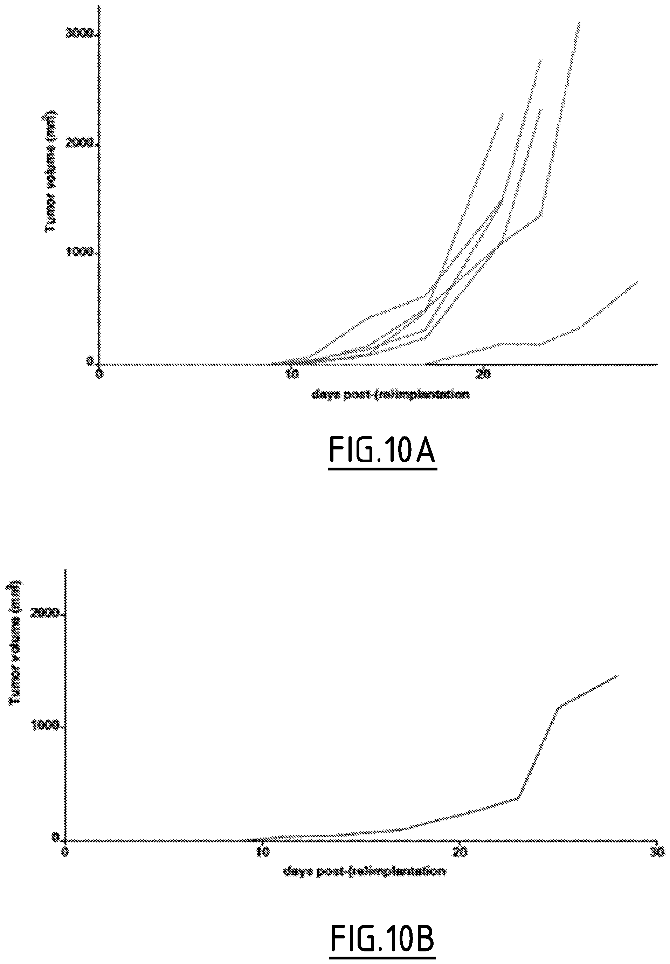

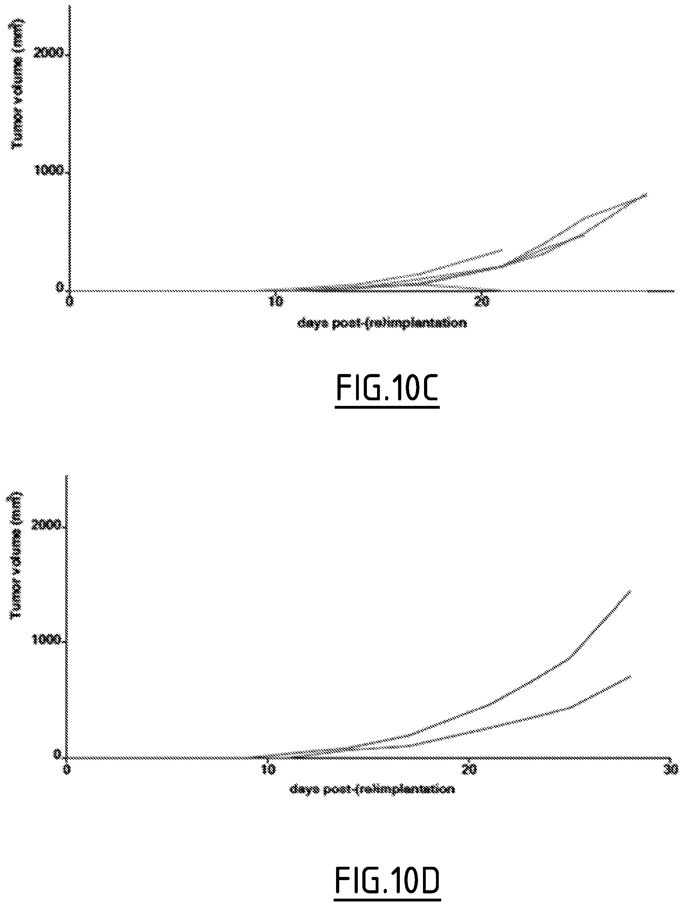

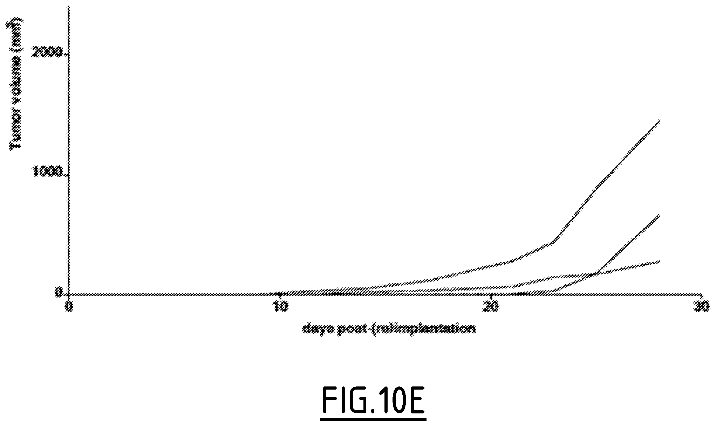

Tumors grew rapidly in most of the the control group while the formerly Cargomer treated mice had slow progression as shown in Fig. 10. Thus, vaccination with Cargomers was demonstrated to be very effective and long lasting. As mice in the survivor group received only three vaccinations with Cargomers, it is envisioned that permanent protection could be achieved with more or recurrent vaccination.

### 8.4. Example 4: Study of Cargomers loaded with tumor antigen peptides and cholesterol-CpG oligonucleotides in a syngeneic mouse cancer model II

A study similar to the study of Example 2 was performed with new preparations of Cargomers. Animals were divided into five treatment groups as follows:

| Group | Number of animals | Vaccination / SC | | | | | Immunotherapy / IP |
|---|---|---|---|---|---|---|---|
| | | Formulation ID | Dose (nmol per animal) | | | Volume administered | |
| | | | ApoA1 | Cho-CpG | Tumor Antigens (M27:M30:TRP2) | | |
| 1 | 8 | Vehicle | 0 | 0 | 0 | 0.1 mL | No |
| 2 | 8 | TAs + Chol-CpG | 0 | 2.5 | 15 | 0.1 mL | No |
| 3 | 8 | TAs + Chol-CpG | 0 | 2.5 | 15 | 0.1 mL | Yes |
| 4 | 8 | Cargomers 1:2¹ | 35 | 2.5 | 15 | 0.1 mL | No |
| 5 | 8 | Cargomers 1:4² | 30 | 2.5 | 15 | 0.1 mL | No |
| Table 3: Treatment groups | | | | | | | |
| ¹ mixture of ApoA-I:chol-CpG (10:1) Cargomers and ApoA-I:TA (1:2) Cargomers | | | | | | | |
| ² mixture of ApoA-I:chol-CpG (10:1) Cargomers and ApoA-I:TA (1:4) Cargomers | | | | | | | |

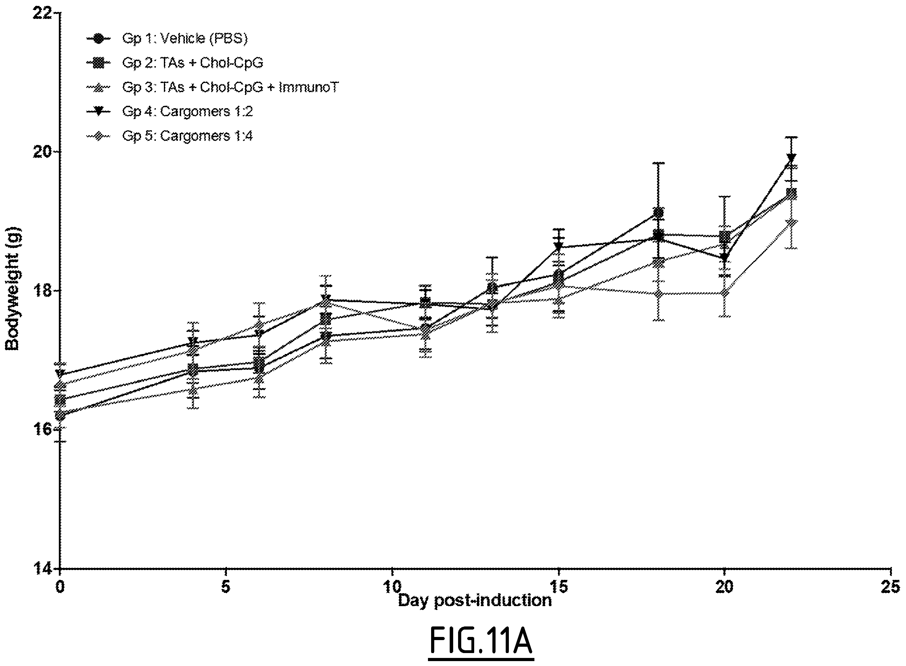

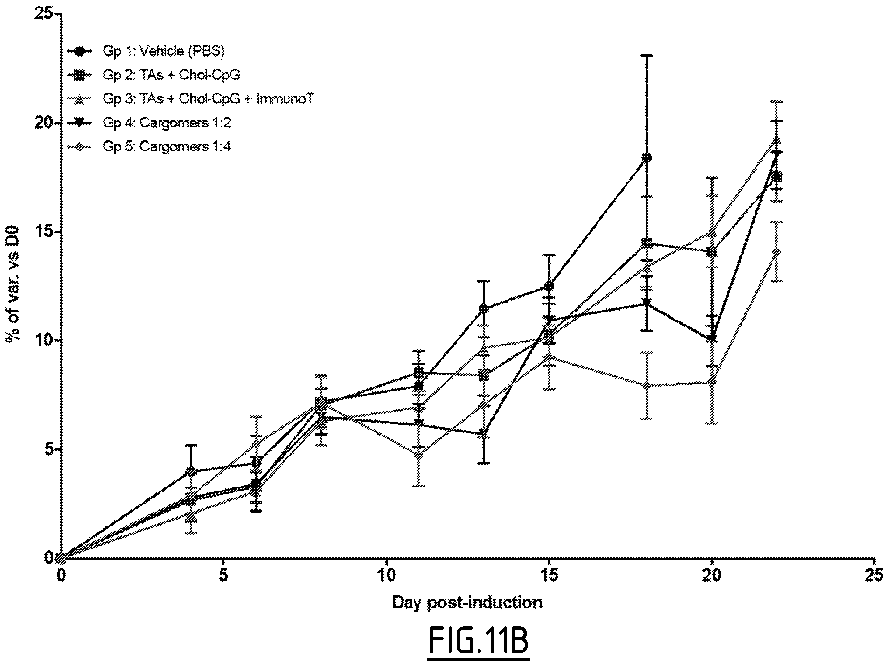

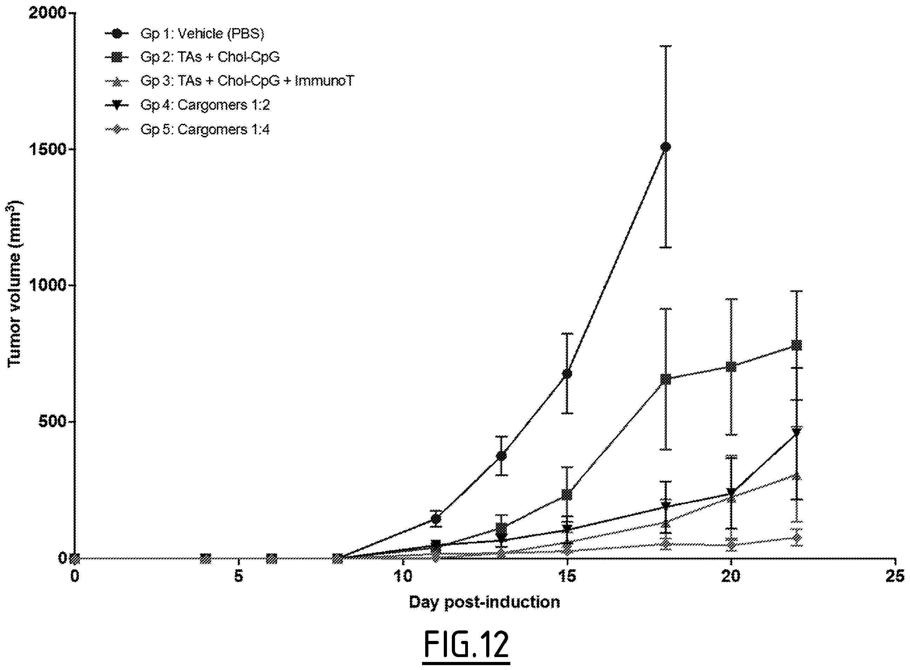

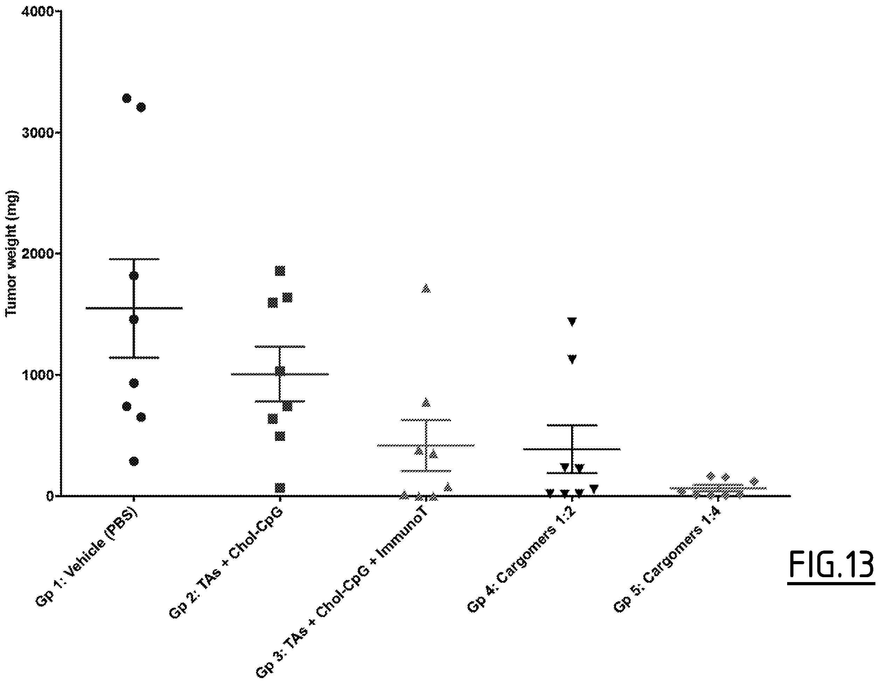

Fig. 11A and Fig. 11B shows the bodyweights and baseline-corrected bodyweights for the animals of Example 4 over the course of the study. Fig. 12 shows tumor volume for the different treatment groups and Fig. 13 shows tumor weights for the animals of each group.

Example 4 confirms the that Cargomers can induce an immune response preventing tumor growth. Cargomer 1:4 was found to be the most potent and and was found to not require check point inhibition for efficacy.

### 8.5. Example 5: Study of Cargomers loaded with tumor antigen peptides and cholesterol-CpG oligonucleotides in a syngeneic mouse cancer model III

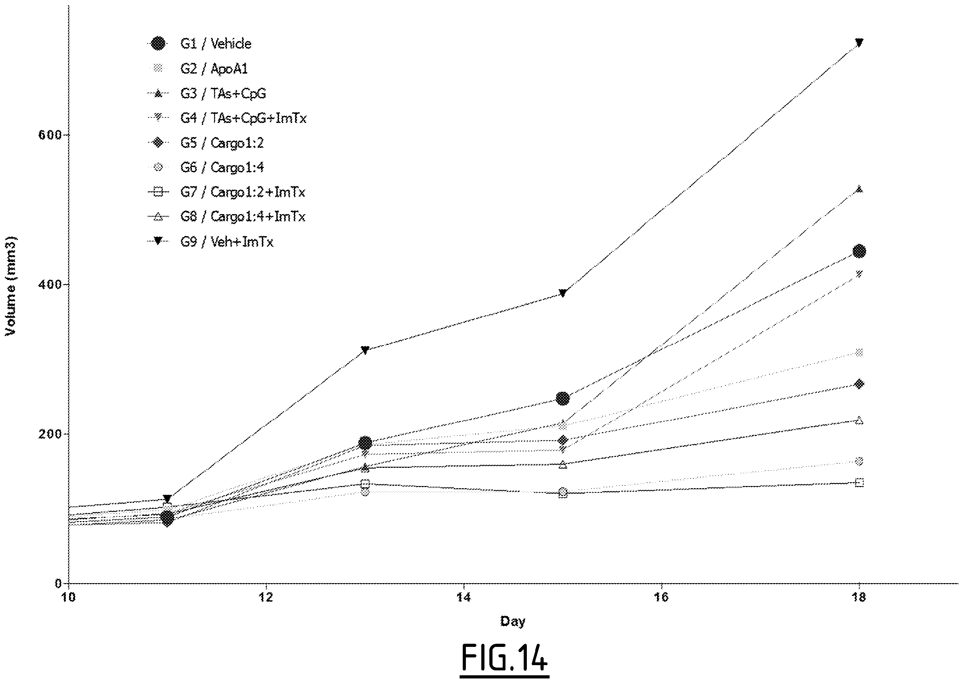

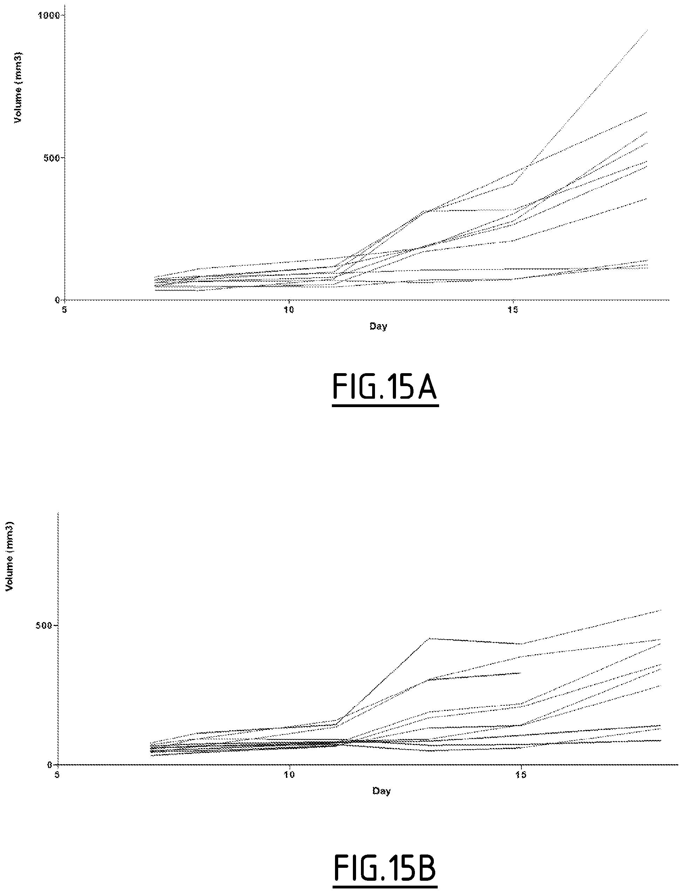

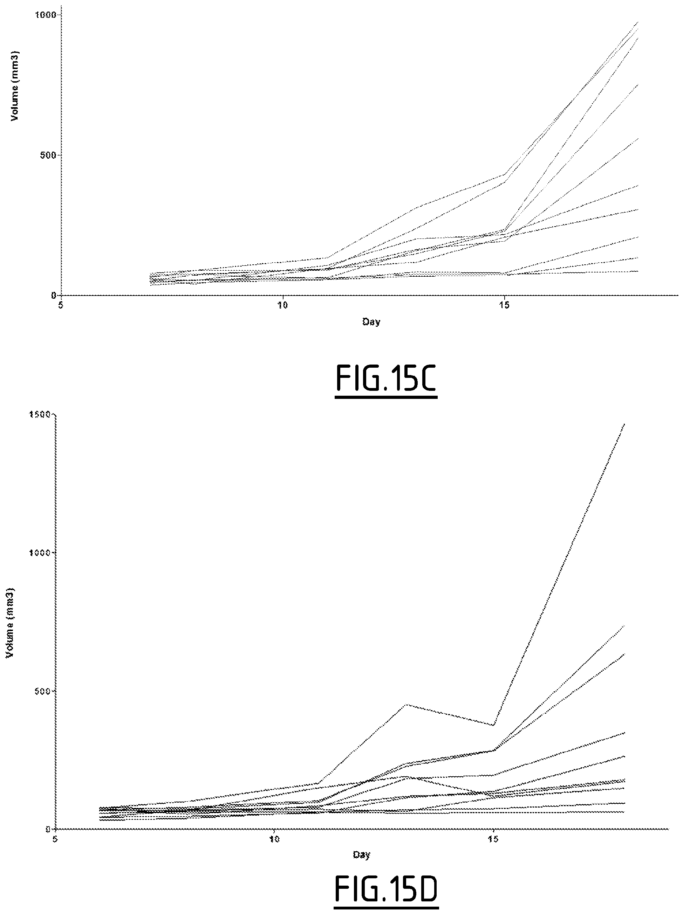

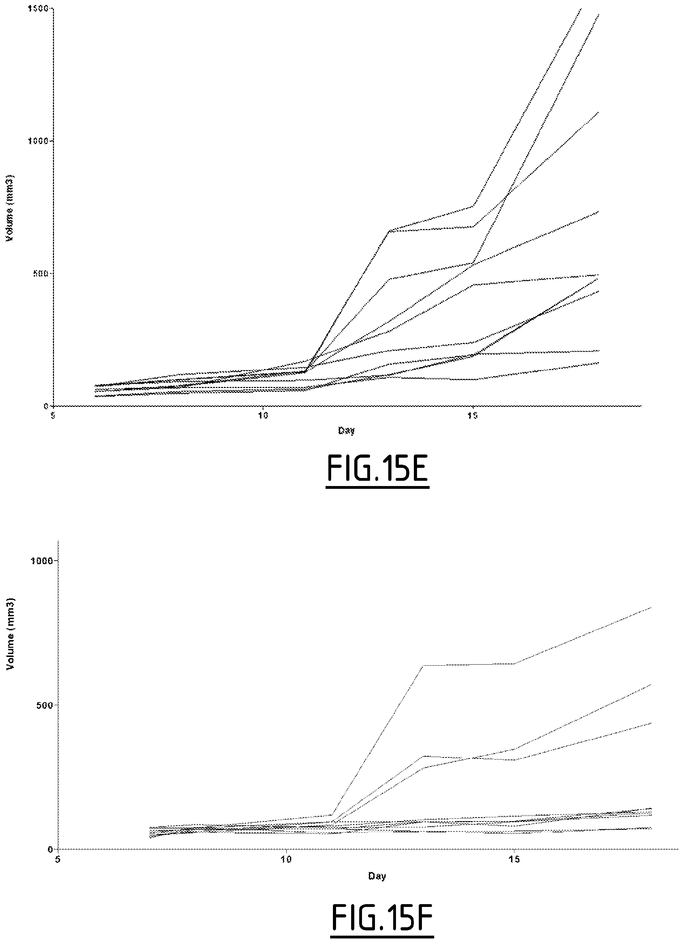

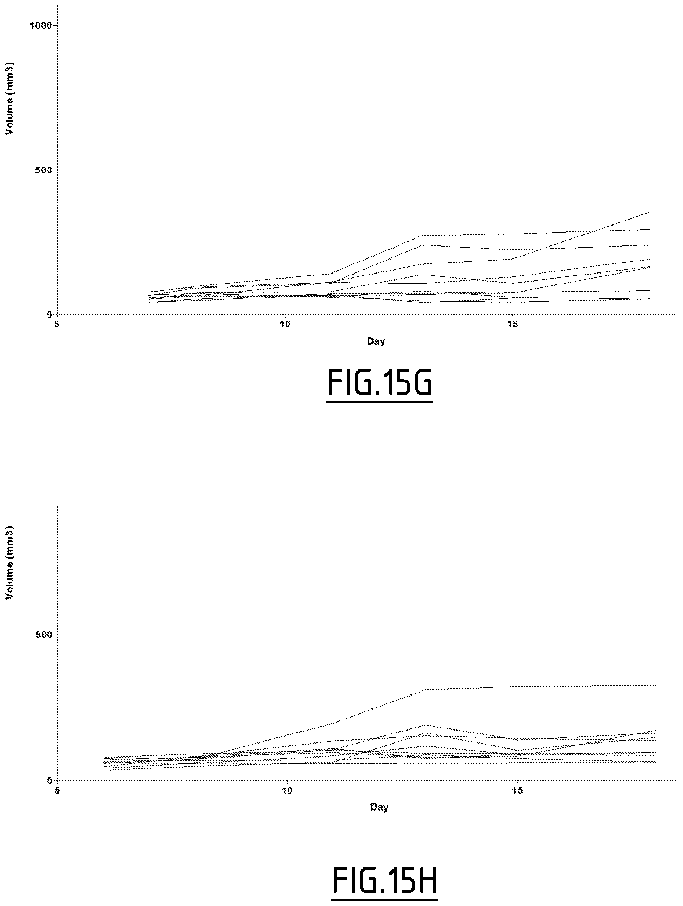

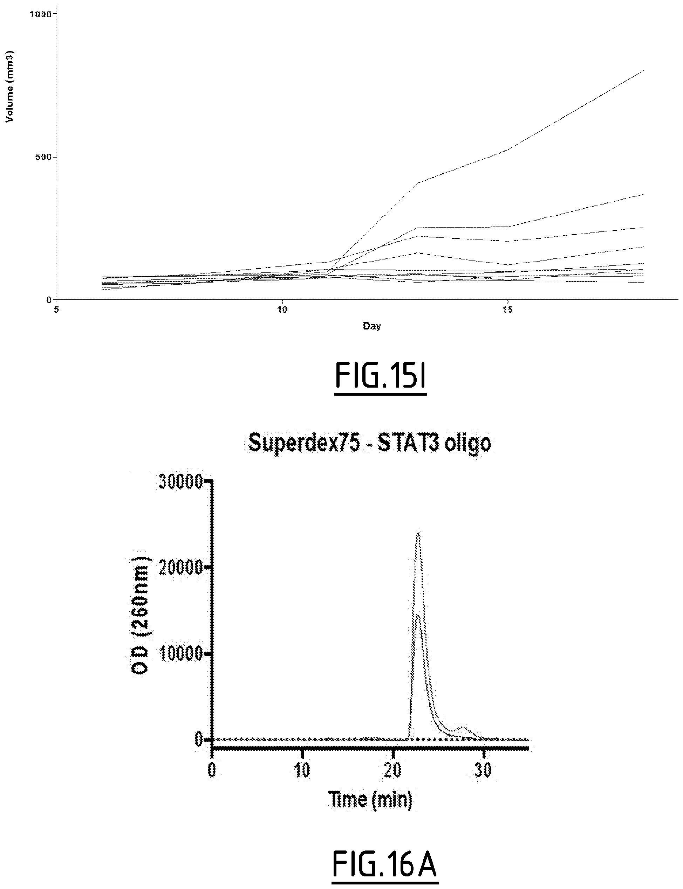

A study similar to the study of Example 2 is being performed with new preparations of Cargomers. Tumor volume for the different treatment groups through day 18 of the study is shown in Fig. 14 and Fig. 15. The results of the study through day 18 are consistent with Example 2.

### 8.6. Example 6: Cargomers with nucleic acid cargo

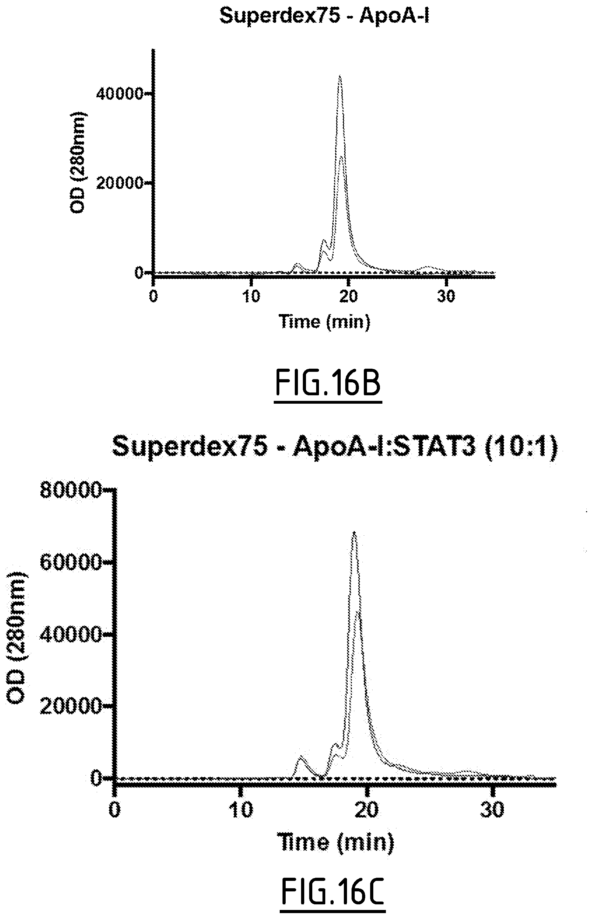

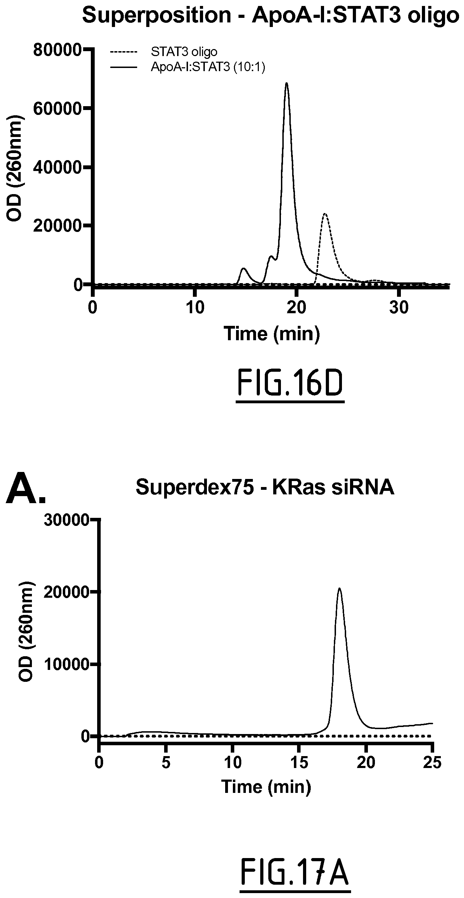

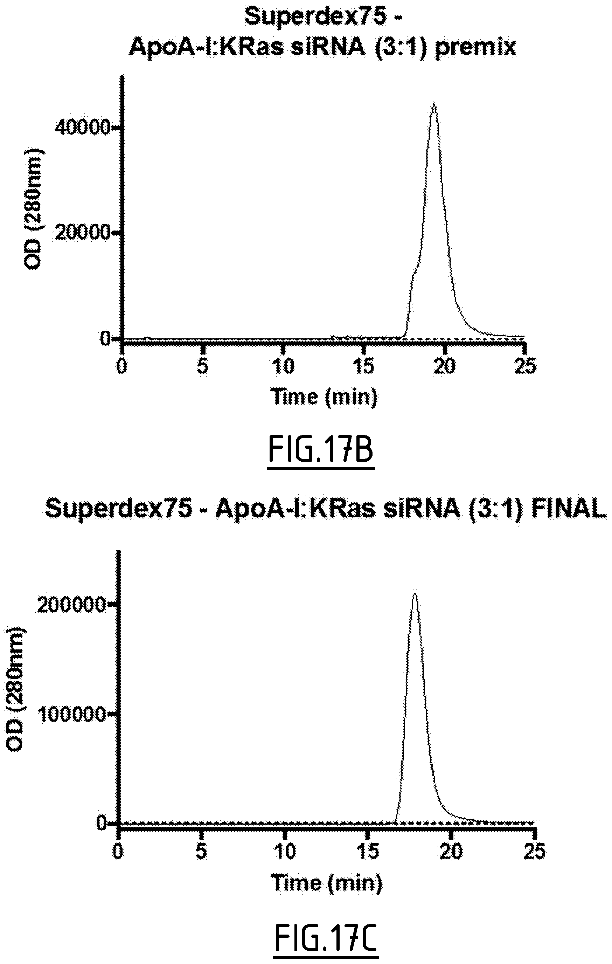

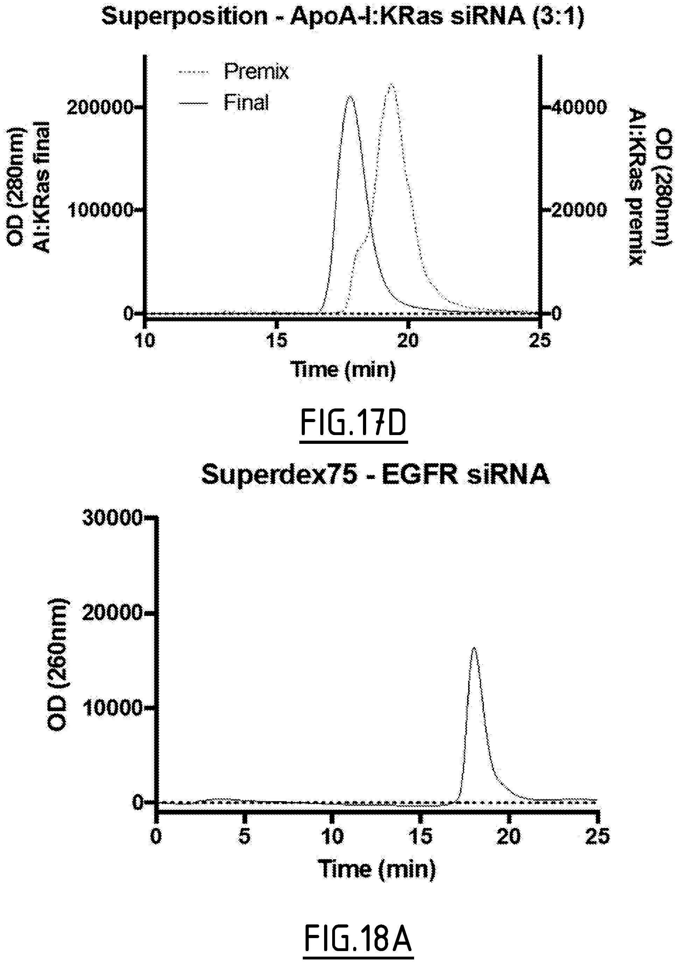

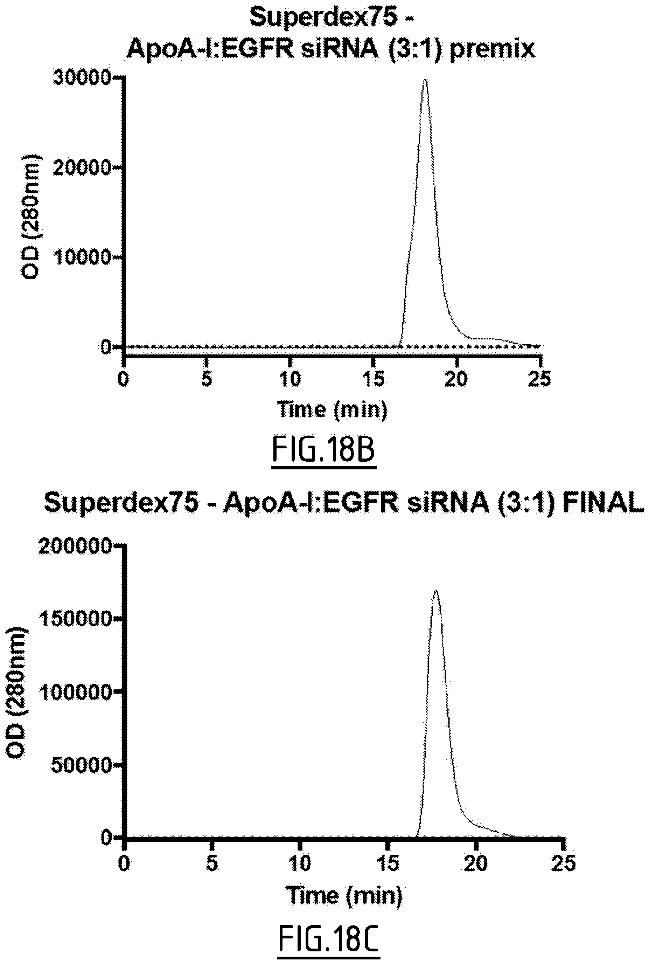

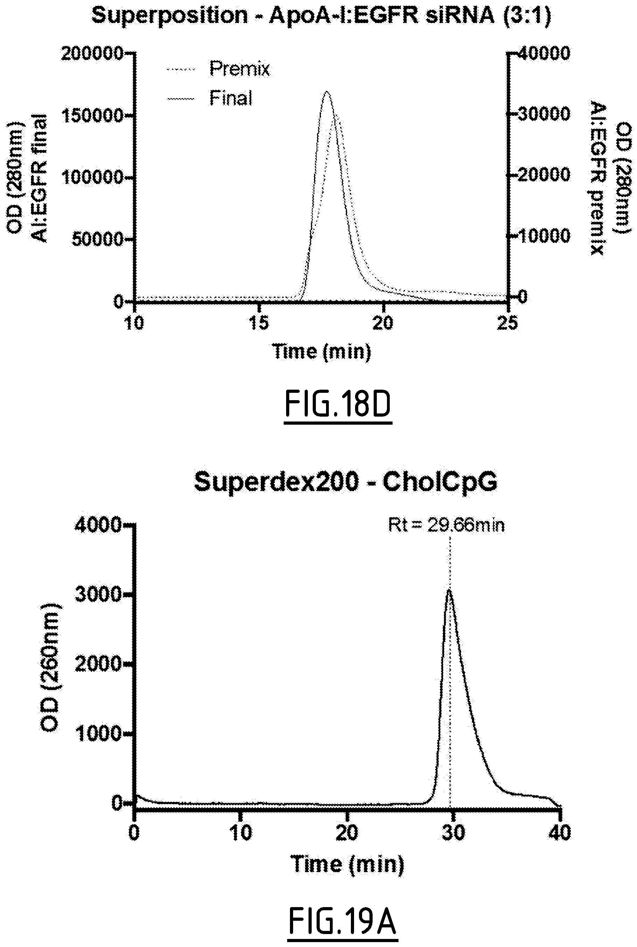

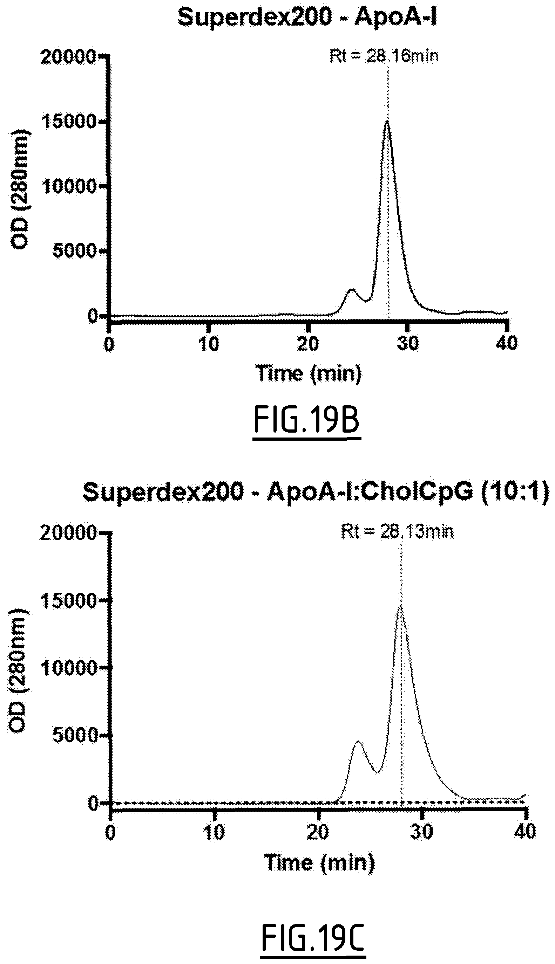

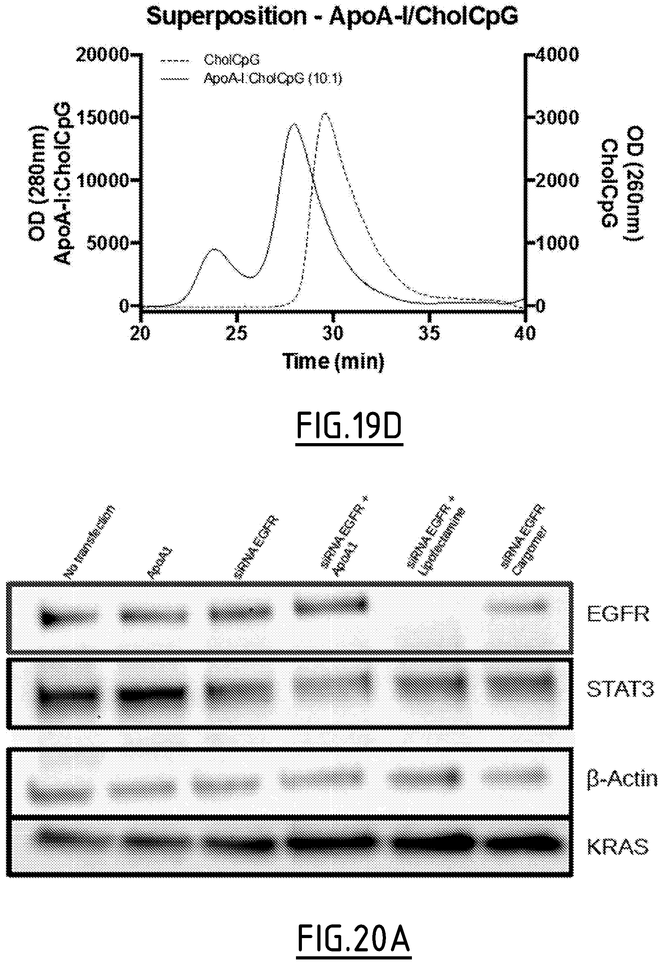

Cargomers were made with the following nucleic acid cargo moieties: an anti-STAT3 antisense oligonucleotide; an anti-KRAS siRNA; an anti-EGFR siRNA; and CpG, an adjuvant. Size exclusion chromatograms showing the Cargomers and components of the Cargomers containing anti-STAT3 antisense oligonucleotide are shown in Fig. 16. Size exclusion chromatograms showing the Cargomers and components of the Cargomers containing anti-KRAS siRNA are shown in Fig. 17. Size exclusion chromatograms showing the Cargomers and components of the Cargomers containing anti-EGFR siRNA are shown in Fig. 18. Size exclusion chromatograms showing the Cargomers and components of the Cargomers containing CpG are shown in Fig. 19.

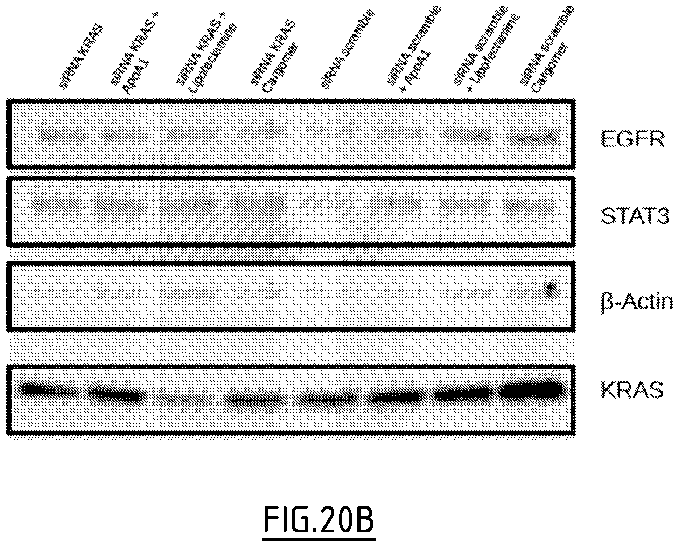

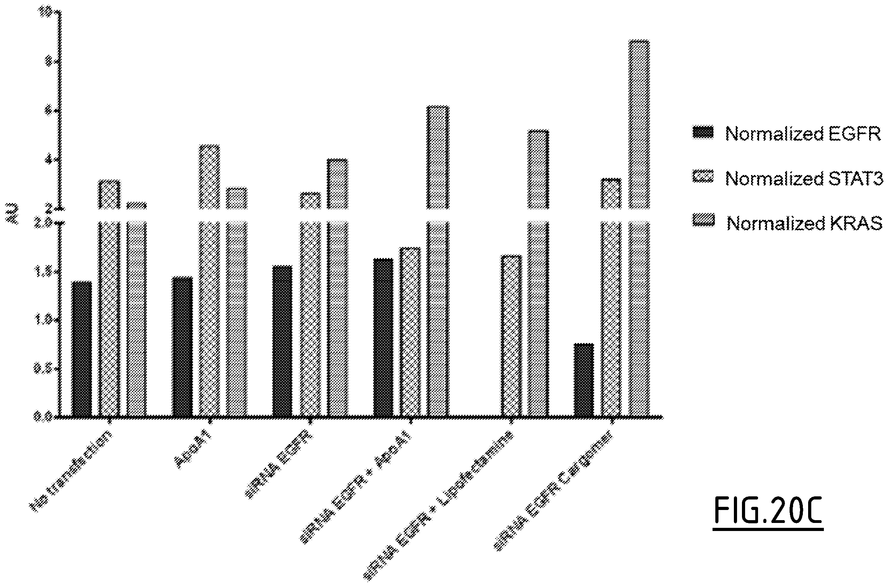

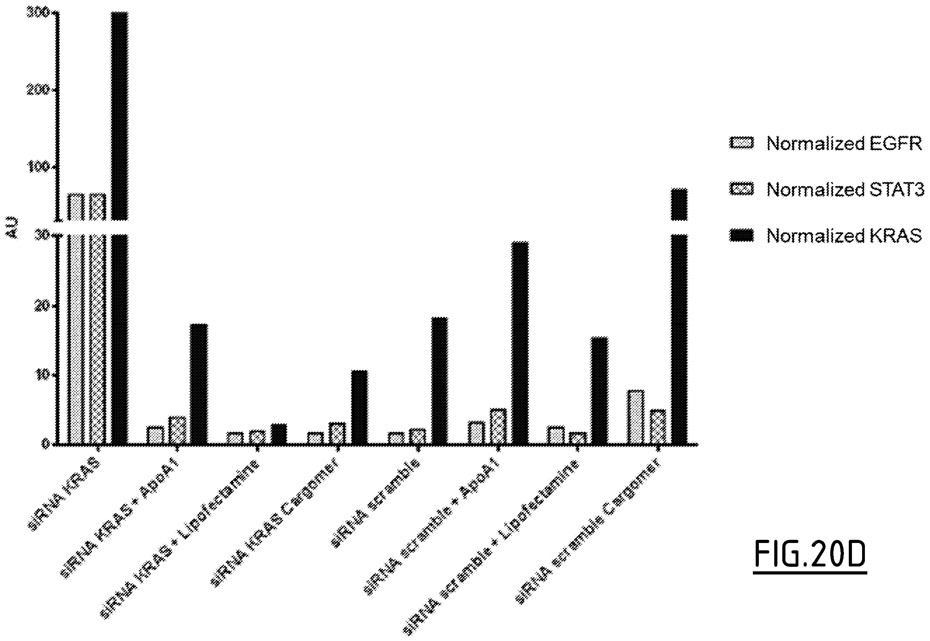

The efficacy of the Cargomers containing an anti-STAT3 antisense oligonucleotide, the Cargomers containing an anti-KRAS siRNA, and the Cargomers containing an anti-EGFR siRNA to silence their respective targets was tested in an *in vitro* assay using PANC-1 cells. Western blots from the assay are shown in Fig. 20. Cargomers containing siRNA were observed to reduce target protein levels.

### 8.7. Example 7: Cargomers with peptide cargo

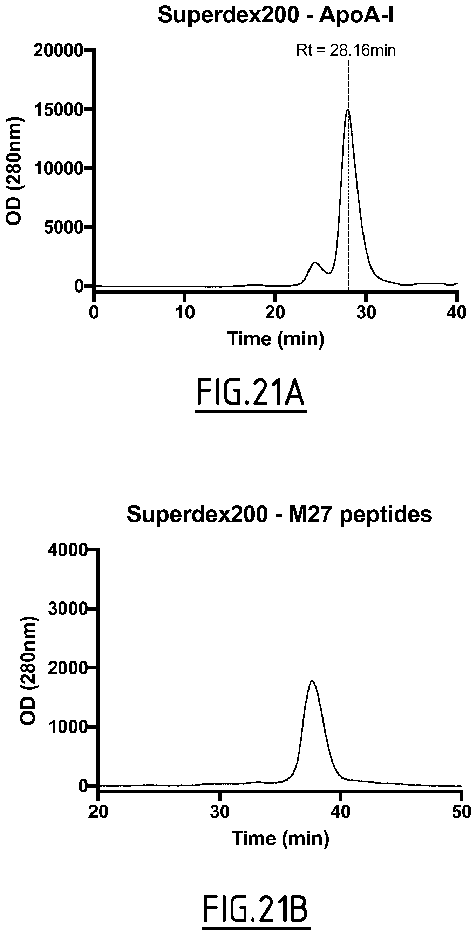

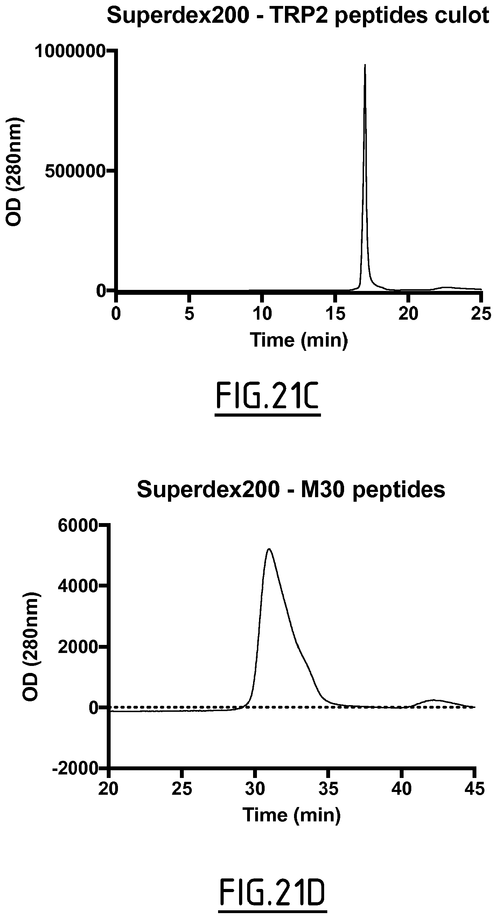

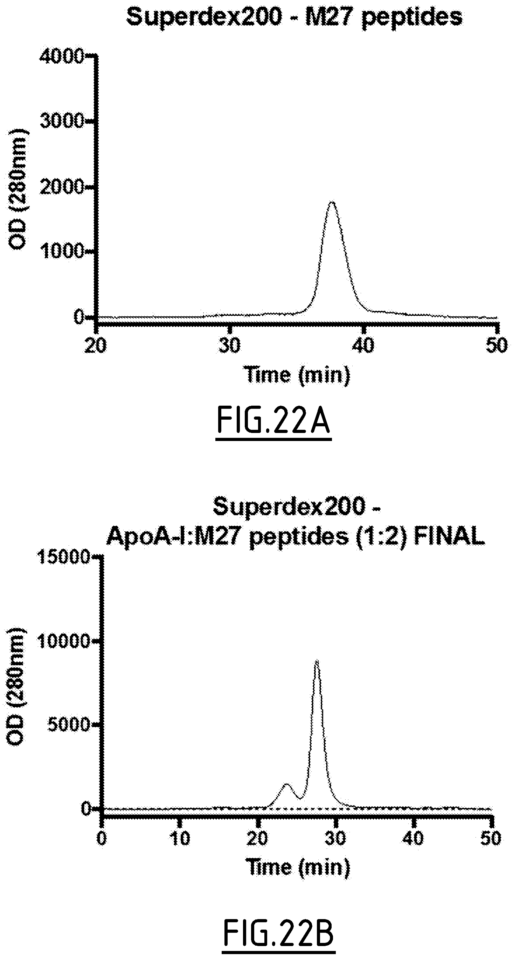

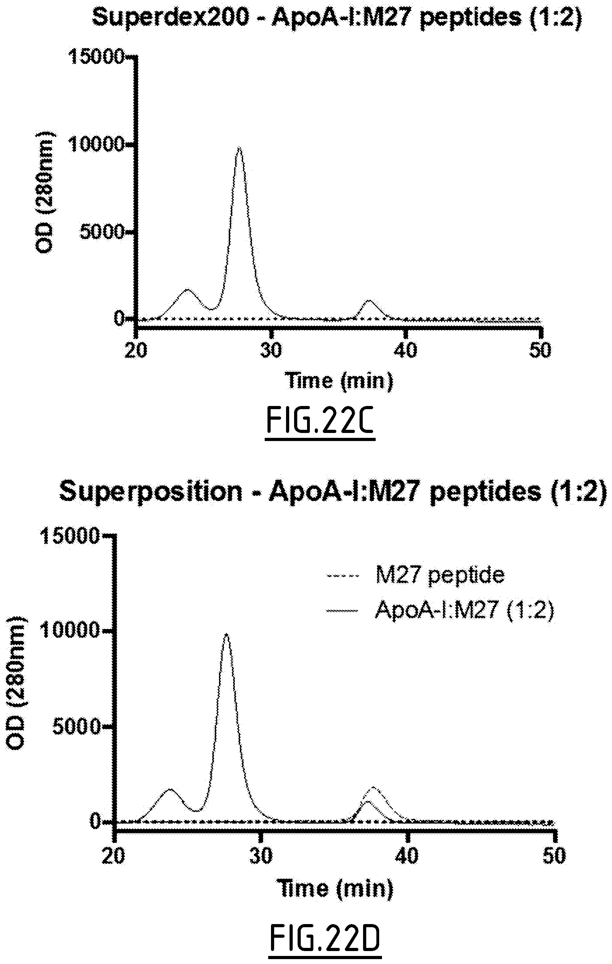

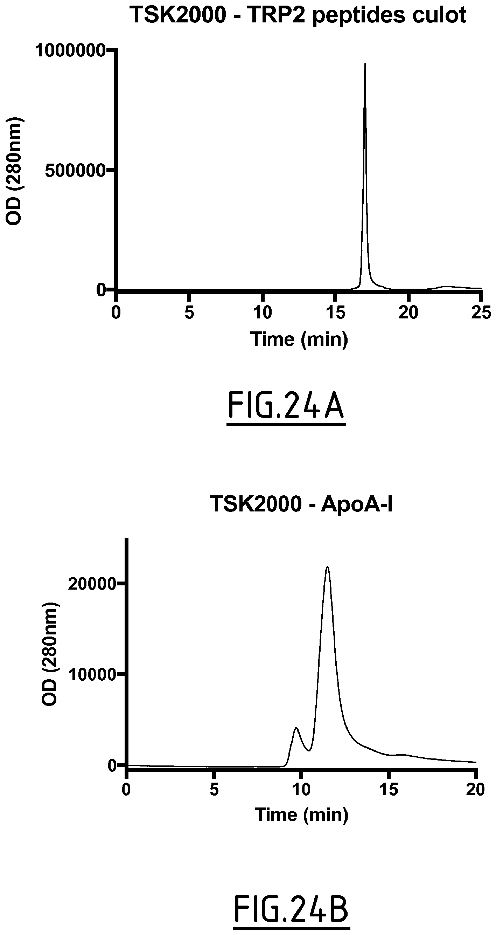

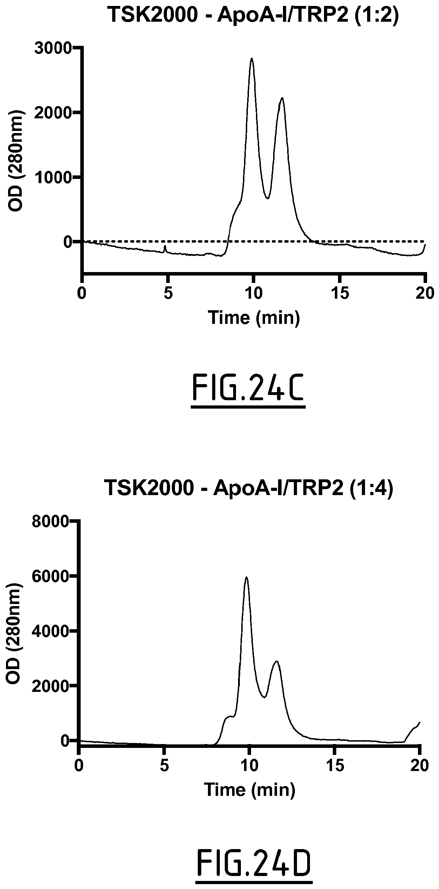

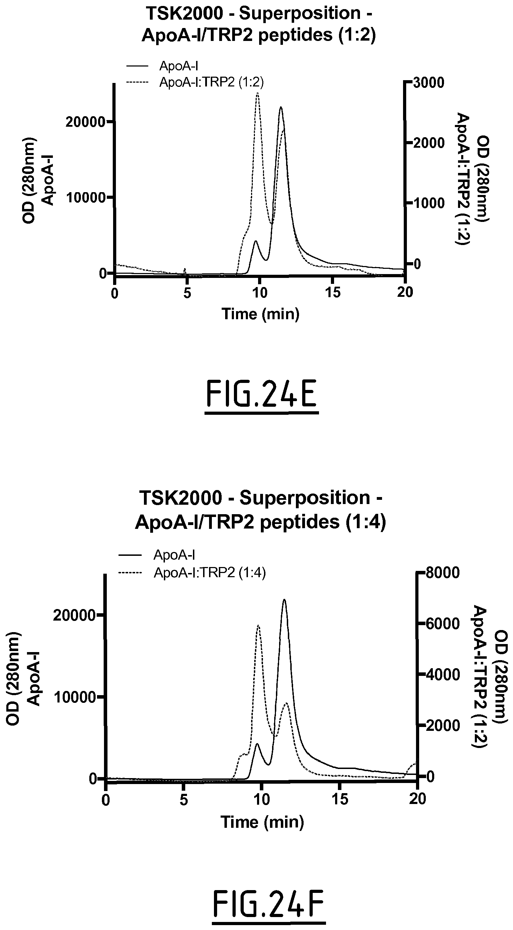

Cargomers of the type used in Examples 2 through 4 were made with the following peptide cargo moieties: M27, M30, and TRP2. Size exclusion chromatograms showing the Cargomers and components of the Cargomers containing the peptides are shown in Fig. 21, Fig. 22, Fig. 23, and Fig. 24.

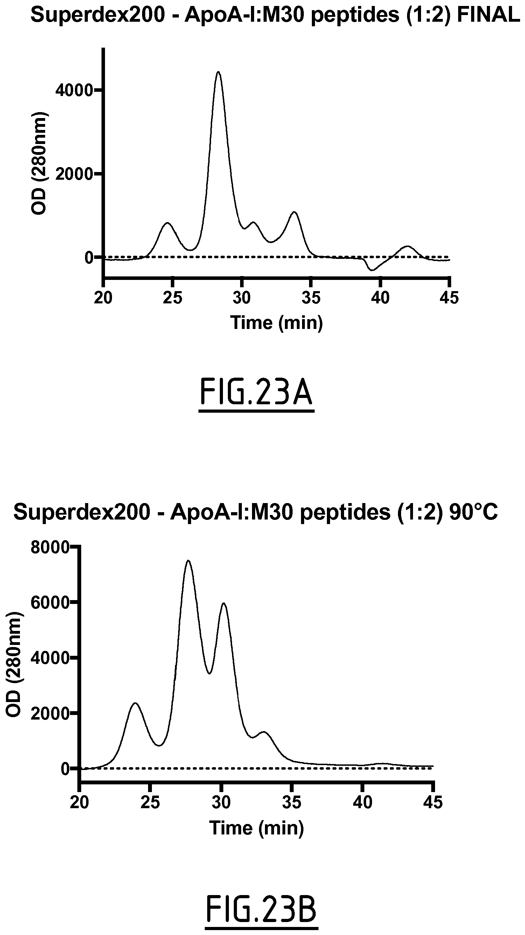

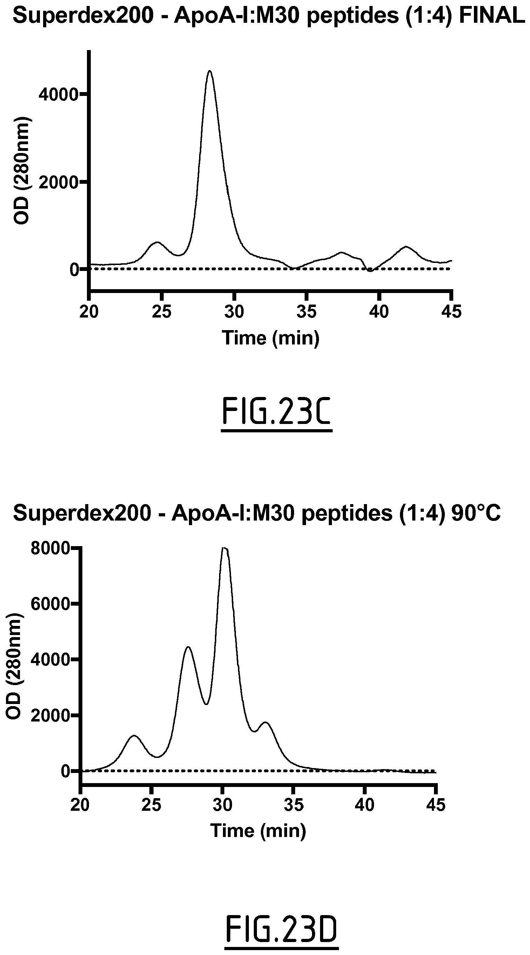

An elegant and simple way to assess binding of a peptide to a Cargomer is to increase the temperature to observe the dissociation of the components because as the temperature is increased, the dissociation-association equilibrium is displaced in favor of the monomeric form of ApoA-I, as shown in Fig. 23.

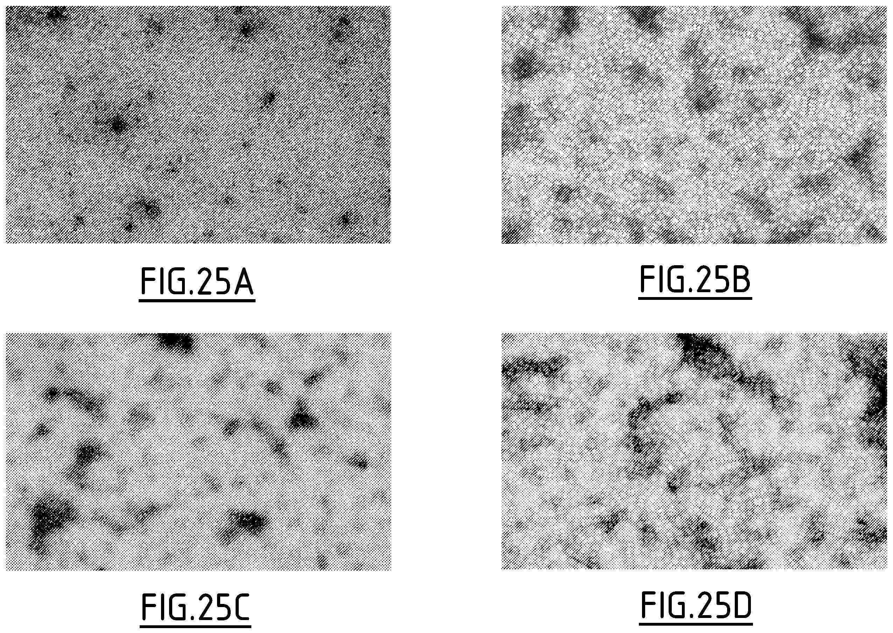

Fig. 25 shows electron micrographs of ApoA-I (Fig. 25A), CER-001 (Fig. 25B), ApoA-I:M27 (1:2) Cargomers (Fig. 2C), and ApoA-I:M27 (1:2) Cargomers and CER-001 (Fig. 25D). CER-001 is a discoidal lipoprotein complex comprising ApoA-I, sphingomyelin (SM) and DPPG in a 1:2.7 lipoprotein wt:total phospholipid wt ratio with a SM:DPPG wt:wt ratio of 97:3. See, Example 4 of WO 2012/109162. Fig. 25 shows that the exemplary Cargomers are small and not discoidal.

### 8.8. Example 8: Study of Cargomers loaded with siRNA or antisense oligonucleotides in a pancreatic cancer model

The purpose of this study is to evaluate the efficacy of Cargomers loaded with siRNA or antisense oligonucleotide in a pancreatic cancer model.

Tumor induction is performed by implanting 2 × 10⁶ PANC1 human pancreatic adenocarcinoma cells by subcutaneous injection on the right flank of female BALB/c nude mice.

Cargomers loaded with siRNA targeting KRAS^{G12D}, siRNA targeting EGFR or antisense oligonucleotide (ASO) targeting STAT3 are administered to the animals according to the following protocol:

| Group | Number of animals | Formulation ID | Dose (nmol per animal) | | Route of administration | Dose (mg/kg) | Frequency | Volume administered |
|---|---|---|---|---|---|---|---|---|
| | | | ApoA1 | siRNA/ASO | | | | |
| 1 | 10 | Cargomers-siRNA scramble (negative control) | 36 | 3.6 | SC | 50 | every other day | 0.1 mL |
| 2 | 10 | siRNA KRAS^{G12D} + siRNA EGFR + ASO STATS | - | 10.8 | SC | - | every other day | 0.3 mL |
| 3 | 10 | Cargomers-siRNA KRAS^{G12D} | 36 | 3.6 | SC | 50 | every other day | 0.1 mL |
| 4 | 10 | Cargomers-ASO STATS | 36 | 3.6 | SC | 50 | every other day | 0.1 mL |
| 5 | 10 | Cargomers-siRNA EGFR | 36 | 3.6 | SC | 50 | every other day | 0.1 mL |
| 6 | 10 | Pool Cargomers-siRNA/ASO | 108 | 10.8 | SC | 150 | every other day | 0.3 mL |
| 7 | 10 | Gemcitabine | - | - | IV | 50 | twice a week | 0.1 mL |
| Table 4: Example 8 protocol | | | | | | | | |

Cargomers loaded with siRNA targeting KRAS^{G12D}, siRNA targeting EGFR or antisense oligonucleotide (ASO) targeting STAT3 reduce tumor growth and prolong survival of tumor bearing mice.

### 8.9. Example 9: Analysis of chol-CpG Cargomers

ApoA-I and sphingomyelin were mixed together to provide a mixture of ApoA-I and SM at 120 µM and 240 µM, respectively, in PBS, pH 7.4 ApoA-I is multimeric under these conditions. Cholesterol-CpG (see Example 2) was added to the mixture to provide a final concentration of 12 µM and an ApoA-I:SM:Chol-CpG molar ratio of 1:2:0.1. The mixture was incubated between 2-8 hours at 37°C to allow the formation of Cargomers. Separately, a sample of CER-001 was incubated with chol-CpG at a 1:0.5 molar ratio.

Analysis by HPLC was performed on the Cargomers, the components used to make the Cargomers, CER-001, and CER-001:CpG using a Superdex^{®} 200 10/300 GL column (L × I.D. 30cm × 10mm, 13 µm average particle size) (GE Healthcare, Ref.17-5175-01). The buffer used for the HPLC was a 10 mM phosphate buffer, pH 7.4. The chromatograms are shown in Fig. 26A-G, with the exception of the chromatogram for the 10 mM phosphate buffer, which showed no significant absorbance at 260 nm or 280 nm.

As expected, the smaller multimeric ApoA-I (ApoA-I alone) eluted later than CER-001 (Fig. 26A and Fig. 26B, respectively). The particular sample of CER-001 used in this study showed a main peak eluting at approximately 22 minutes, and a smaller secondary peak eluting at approximately 14 minutes. It is possible that the secondary peak corresponded to proApoA-I or aggregated material. The observed A280 peak for the ApoA-I:SM:chol-CpG Cargomer sample (Fig. 26D) is approximately equal to the sum of the A280 peaks observed in the ApoA-I (Fig. 26A) and chol-CpG (Fig. 26C) chromatograms, indicating a high degree of cargo loading into the Carogmers. In the absence of SM, an additional A260 peak was observed (Fig. 26E), suggesting incomplete complexing of the ApoA-I and chol-CpG. In the absence of ApoA-I, SM and chol-CpG together eluted at a different time (Fig. 26F) compared to the ApoA-I:SM:chol-CpG Cargomers. Under the conditions of this study, CER-001 appeared to poorly bind chol-CpG (Fig. 26G). Accordingly, in this study, Cargomers were found to be superior to CER-001 for binding chol-CpG.

### 9. SEQUENCE LISTING

| SEQ ID NO | Sequence |
|---|---|
| 1 | |
| 2 | DEPPQSPWDR VKDLATVYVD VLKDSGRDYV SQFEGSALGK QLNLKLLDNW DSVTSTFSKL REQLGPVTQE FWDNLEKETE GLRQEMSKDL EEVKAKVQPY LDDFQKKWQE EMELYRQKVE PLRAELQEGA RQKLHELQEK LSPLGEEMRD RARAHVDALR THLAPYSDEL RQRLAARLEA LKENGGARLA EYHAKATEHL STLSEKAKPA LEDLRQGLLP VLESFKVSFL SALEEYTKKL NTQ |
| 3 | 5'-GGGGGACGA:TCGTCGGGGGG-3' (20 mer) |
| 4 | 5'- GGGGACGAC:GTCGTGGGGGGG -3' (21 mer) |
| 5 | 5'-TCGTCGTTTTGTCGTTTTGTCGTT-3' (24 mer) |
| 6 | 5'-TCGACGTTCGTCGTTCGTCGTTC-3' (23 mer) |
| 7 | 5'-TCGCGACGTTCGCCCGACGTTCGGTA-3' (26 mer) |
| 8 | 5'-TCGTCGTTTTCGGCGC:GCGCCG-3' (22 mer) |
| 9 | 5'-TCGTCGTCGTTC:GAACGACGTTGAT-3' (25 mer) |
| 10 | 5'-TCGCGAACGTTCGCCGCGTTCGAACGCGG-3' (29 mer) |
| 11 | 5'-T*C*C*A*T*G*A*C*G*T*T*C*C*T*G*A*C*G*T*T-3' |
| 12 | LCPGNKYEM |
| 13 | CSSVDWENVSPELNSTDQ |
| 14 | CSVYDFFVWL |
| 15 | CYKLVVVGADGVGKSAL |
| 16 | CYKLVVVGAVGVGKSAL |
| 17 | CYKLVVVGACGVGKSAL |
| 18 | CYKLVVVGAGGVGKSAL |
| 19 | CYKLVVVGADGVGKSALTIQLIQ |
| 20 | CGGMTEYKLVVVGAGG |
| 21 | CGGEGFLCVFAINNTKS |
| 22 | CGGKSALTIQLIQNH |
| 23 | KLVVVGADGVGKSAL |
| 24 | KLVVVGAVGVGKSAL |
| 25 | KLVVVGACGVGKSAL |
| 26 | KLVVVGAGGVGKSAL |
| 27 | YKLVVVGADGVGKSALTIQLI |
| 28 | YKLVVVGAGGVGKSALTIQLI |

## Claims

1. A Cargomer comprising:
(a) 1-8 apolipoprotein molecules;
(b) one or more cargo moieties;
(c) an amount of amphipathic molecules sufficient to solubilize the apolipoprotein molecules, wherein one or more of the cargo moieties of (b) and one or more of the amphipathic molecules of (c) can be be the same molecule(s) in the Cargomer;
(d) optionally, one or more anchors non-covalently coupling one or more cargo moieties to the apolipoprotein molecules; and
(e) optionally, one or more linkers covalently coupling one or more cargo moieties to one or more apolipoprotein molecules, one or more amphipathic molecules or one or more anchors,
wherein (i) the amphipathic molecules, the cargo moieties and, if present, the anchors and/or linkers together contribute a net charge of at least +1 or -1 per apolipoprotein molecule in the Cargomer; and (ii) the apolipoprotein to amphipathic molecule molar ratio ranges from 8:1 to 1:15.

2. The Cargomer of claim 1, which is not a discoidal particle.

3. The Cargomer of claim 1 or claim 2, which comprises 1 to 25 cargo moieties.

4. The Cargomer of any one of claims 1 to 3, wherein at least one amphipathic molecule is also a cargo moiety.

5. The Cargomer of any one of claims 1 to 4, wherein at least one cargo moiety is coupled to an anchor, optionally wherein the anchor is an amphipathic molecule, further optionally wherein the anchor comprises:
(a) a phospholipid, which is optionally a negatively charged phospholipid; or
(b) cholesterol.

6. The Cargomer of any one of claims 1 to 5, wherein the cargo moiety is coupled to the anchor by a direct bond, or wherein the cargo moiety is coupled to the anchor by a linker, optionally wherein at least one cargo moiety is coupled to an apolipoprotein molecule.

7. The Cargomer of any one of claims 1 to 6, wherein the amphipathic molecules comprise a phospholipid, a detergent, a fatty acid, an apolar moiety or sterol covalently attached to a sugar, or a combination thereof, optionally wherein the amphipathic molecules comprise or consist of phospholipid molecules, which are optionally negatively charged phospholipids, neutral phospholipids or a combination thereof.

8. The Cargomer of any one of claims 1 to 7 which comprises 1, 2, 4 or 8 apolipoprotein molecules.

9. The Cargomer of any one of claims 1 to 8, wherein the apolipoprotein molecules comprise or consist of apolipoprotein A-I (ApoA-I) molecules.

10. The Cargomer of any one of claims 1 to 9, wherein one or more cargo moieties comprise a therapeutic agent, a diagnostic agent or an immunogen, optionally wherein:
(a) the one or more cargo moieties comprise a therapeutic agent, which is optionally an immunoinhibitory agent, an immunostimulatory agent, an anti-cancer agent, an anti-infective agent, a nucleic acid drug, an anti-inflammatory agent, an agent for treating cardiovascular disorders, a caspase inhibitor, or a bioactive agent,
(b) the one or more cargo moieties comprise an immunogen, which is optionally an antigen or an antigen-encoding nucleic acid, optionally wherein the antigen is an allergic antigen, an autoantigen, or a tumor antigen, optionally wherein the the tumor antigen is a neoantigen or a shared antigen; or
(c) the one or more cargo moieties comprises a diagnostic agent, which is optionally an imaging agent.

11. A pharmaceutical composition comprising an effective amount of the Cargomers of claim 10(a) and one or more pharmaceutically acceptable carriers, diluents, and/or excipients, or a vaccine composition comprising an effective amount of the Cargomers of claim 10(b) and one or more pharmaceutically acceptable carriers, diluents, excipients, and/or adjuvants, or a diagnostic composition comprising an effective amount of the Cargomers of claim 10(c) and one or more carriers, diluents, and/or excipients suitable for diagnostic use.

12. The composition of claim 11, in which no more than 20%, no more than 10%, no more than 5%, or no more than 2% of the amphipathic molecules are in uncomplexed form, optionally wherein the Cargomers in the composition are at least 75%, at least 85%, at least 90%, at least 95%, or at least 98% homogeneous.

13. The Cargomer of claim 10(a) or claim 10(b) for use as a medicament, optionally wherein the Cargomer is in the form of a pharmaceutical composition comprising one or more pharmaceutically acceptable carriers, diluents, and/or excipients.

14. The Cargomer of claim 10(c) for use *in vivo* as an diagnostic agent, which is optionally in the form of a diagnostic composition comprising one or more carriers, diluents, and/or excipients suitable for diagnostic use.

15. The Cargomer of claim 10(a) or claim 10(b) for use in the treatment of cancer.

## Patentansprüche

1. Cargomer umfassend:
(a) 1-8 Apolipoproteinmoleküle;
(b) eine oder mehrere Frachteinheiten;
(c) eine Menge an amphipathischen Molekülen, die ausreicht, um die Apolipoproteinmolekülezu solubilisieren, wobei eine oder mehrere der Frachteinheiten von (b) und eine oder mehrere der amphipathischen Moleküle von (c) das/die gleiche(n) Molekül(e) im Cargomer sein können;
(d) optional einen oder mehrere Anker, die eine oder mehrere Frachteinheiten nicht-kovalent an die Apolipoproteinmoleküle koppeln; und
(e) optional einen oder mehrere Linker, die einen oder mehrere Frachteinheiten kovalent an ein oder mehrere Apolipoproteinmoleküle, ein oder mehrere amphipathische Moleküle oder einen oder mehrere Anker koppeln,
wobei (i) die amphipathischen Moleküle, die Frachteinheiten und, falls vorhanden, die Anker und/oder Linker zusammen eine Nettoladung von mindestens +1 oder -1 pro Apolipoproteinmolekül im Cargomer beitragen; und (ii) das Apolipoprotein zu amphipathischem Molekül-Molverhältnis im Bereich von 8:1 bis 1:15 liegt.

2. Cargomer gemäß Anspruch 1, der kein diskoidaler Partikel ist.

3. Cargomer gemäß Anspruch 1 oder 2, der 1 bis 25 Frachteinheiten umfasst.

4. Cargomer gemäß irgendeinem der Ansprüche 1 bis 3, wobei mindestens ein amphipathisches Molekül auch eine Frachteinheit ist.

5. Cargomer gemäß irgendeinem der Ansprüche 1 bis 4, wobei mindestens eine Frachteinheit an einen Anker gekoppelt ist, optional wobei der Anker ein amphipathisches Molekül ist, ferner optional, wobei der Anker umfasst:
(a) ein Phospholipid, das optional ein negativ geladenes Phospholipid ist; oder
(b) Cholesterin.

6. Cargomer gemäß irgendeinem der Ansprüche 1 bis 5, wobei die Frachteinheit durch eine direkte Bindung an den Anker gekoppelt ist, oder wobei die Frachteinheit durch einen Linker an den Anker gekoppelt ist, optional wobei mindestens eine Frachteinheit an ein Apolipoproteinmolekül gekoppelt ist.

7. Cargomer gemäß irgendeinem der Ansprüche 1 bis 6, wobei die amphipathischen Moleküle ein Phospholipid, ein Detergens, eine Fettsäure, eine apolare Einheit oder ein kovalent an einen Zucker gebundenes Sterol, oder eine Kombination davon umfassen, optional wobei die amphipathischen Moleküle Phospholipidmoleküle, die optional negativ geladene Phospholipide, neutrale Phospholipide oder eine Kombination davon sind, umfassen oder aus solchen bestehen.

8. Cargomer gemäß irgendeinem der Ansprüche 1 bis 7, der 1, 2, 4 oder 8 Apolipoproteinoleküle umfasst.

9. Cargomer gemäß irgendeinem der Ansprüche 1 bis 8, wobei die Apolipoproteinmoleküle Apolipoprotein A-I (ApoA-I)-Moleküle umfassen oder aus solchen bestehen.

10. Cargomer gemäß irgendeinem der Ansprüche 1 bis 9, wobei ein oder mehrere Frachteinheiten ein therapeutisches Mittel, ein diagnostisches Mittel oder ein Immunogen umfassen, optional wobei
(a) die eine oder die mehreren Frachteinheiten ein therapeutisches Mittel umfassen, das optional ein immuninhibitorisches Mittel, ein immunstimulatorisches Mittel, ein Antikrebsmittel, ein anti-infektiöses Mittel, ein Nukleinsäure-Medikament, ein entzündungshemmendes Mittel, ein Mittel zur Behandlung von Herz-Kreislauf-Erkrankungen, ein Caspase-Inhibitor oder ein bioaktives Mittel ist,
(b) die eine oder die mehreren Frachteinheiten ein Immunogen umfassen, das optional ein Antigen oder eine Antigen-kodierende Nukleinsäure ist, wobei das Antigen optional ein allergisches Antigen, ein Autoantigen oder ein Tumorantigen ist, wobei optional das Tumorantigen ein Neoantigen oder ein geteiltes Antigen ist; oder
(c) die eine oder die mehreren Frachteinheiten ein diagnostisches Mittel umfassen, das gegebenenfalls ein bildgebendes Mittel ist.

11. Pharmazeutische Zusammensetzung, umfassend eine effektive Menge der Cargomere gemäß Anspruch 10(a) und einen oder mehrere pharmazeutisch akzeptable Träger, Verdünnungsmittel und/oder Hilfsstoffe,
oder eine Impfstoffzusammensetzung, umfassend eine wirksame Menge der Cargomere gemäß Anspruch 10(b) und einen oder mehrere pharmazeutisch akzeptable Träger, Verdünnungsmittel, Hilfsstoffe, und/oder Adjuvantien, oder eine diagnostische Zusammensetzung, umfassend eine wirksame Menge der Cargomere gemäß Anspruch 10(c) und einen oder mehrere Träger, Verdünnungsmittel und/oder Hilfsstoffe, die für die diagnostische Verwendung geeignet sind.

12. Pharmazeutische Zusammensetzung gemäß Anspruch 11, in der nicht mehr als 20%, nicht mehr als 10%, nicht mehr als 5% oder nicht mehr als 2% der amphipathischen Moleküle in nicht-komplexierter Form vorliegen, wobei optional die Cargomere in der Zusammensetzung mindestens 75 %, mindestens 85 %, mindestens 90%, mindestens 95% oder mindestens 98% homogen sind.

13. Cargomer gemäß Anspruch 10(a) oder Anspruch 10(b) zur Verwendung als Arzneimittel, optional wobei der Cargomer in Form einer pharmazeutischen Zusammensetzung vorliegt, die einen oder mehrere pharmazeutisch akzeptable Träger, Verdünnungsmittel und/oder Hilfsstoffe umfasst.

14. Cargomer gemäß Anspruch 10(c) zur Verwendung *in vivo* als diagnostisches Mittel, das optional in Form einer diagnostischen Zusammensetzung, die einen oder mehrere Träger, Verdünnungsmittel und/oder Hilfsstoffe, die für die diagnostische Verwendung geeignet sind, umfasst.

15. Cargomer gemäß Anspruch 10(a) oder Anspruch 10(b) zur Verwendung bei der Behandlung von Krebs.

## Revendications

1. Cargomère comprenant :
(a) 1-8 molécules d'apolipoprotéine ;
(b) une ou plusieurs fractions cargo ;
(c) une quantité de molécules amphipathiques suffisante pour solubiliser les molécules d'apolipoprotéine, dans lequel une ou plusieurs des fractions cargo de (b) et une ou plusieurs des molécules amphipathiques de (c) peuvent être la ou les même(s) molécule(s) dans le cargomère ;
(d) facultativement, une ou plusieurs ancres couplant de manière non covalente une ou plusieurs fractions cargo aux molécules d'apolipoprotéine ; et
(e) facultativement, un plusieurs lieurs couplant de manière covalente une ou plusieurs fractions cargo aux une ou plusieurs molécules d'apolipoprotéine, une ou plusieurs molécules amphipathiques ou une ou plusieurs ancres,
dans lequel (i) les molécules amphipathiques, les fractions cargo et, si présents, les ancres et/ou les lieurs contribuent ensemble à une charge nette d'au moins +1 ou -1 par molécule d'apolipoprotéine dans le cargomère ; et (ii) le rapport molaire des molécules d'apolipoprotéine aux molécules amphipathiques va de 8:1 à 1:15.

2. Cargomère selon la revendication 1, qui n'est pas une particule discoïde.

3. Cargomère selon la revendication 1 ou la revendication 2, qui comprend 1 à 25 fractions cargo.

4. Cargomère selon l'une quelconque des revendications 1 à 3, dans lequel au moins une molécule amphipathique est également une fraction cargo.

5. Cargomère selon l'une quelconque des revendications 1 à 4, dans lequel au moins une fraction cargo est couplée à une ancre, facultativement dans lequel l'ancre est une molécule amphipathique, en outre facultativement dans lequel l'ancre comprend :
(a) un phospholipide, qui est facultativement un phospholipide chargé négativement ; ou
(b) du cholestérol.

6. Cargomère selon l'une quelconque des revendications 1 à 5, dans lequel la fraction cargo est couplée à l'ancre par une liaison directe, ou dans lequel la fraction cargo est couplée à l'ancre par un lieur, facultativement dans lequel au moins une fraction cargo est couplée à une molécule d'apolipoprotéine.

7. Cargomère selon l'une quelconque des revendications 1 à 6, dans lequel les molécules amphipathiques comprennent un phospholipide, un détergent, un acide gras, une fraction apolaire ou un stérol fixé de manière covalente à un sucre, ou une combinaison de ceux-ci, facultativement dans lequel les molécules amphipathiques comprennent ou consistent en des molécules phospholipidiques, qui sont facultativement des phospholipides chargés négativement, des phospholipides neutres ou une combinaison de ceux-ci.

8. Cargomère selon l'une quelconque des revendications 1 à 7 qui comprend 1, 2, 4 ou 8 molécules d'apolipoprotéine.

9. Cargomère selon l'une quelconque des revendications 1 à 8, dans lequel les molécules d'apolipoprotéine comprennent ou consistent en des molécules d'apolipoprotéine A-I (ApoA-I).

10. Cargomère selon l'une quelconque des revendications 1 à 9, dans lequel une ou plusieurs fractions cargo comprennent un agent thérapeutique, un agent de diagnostic ou un immunogène, facultativement dans lequel :
(a) les une ou plusieurs fractions cargo comprennent un agent thérapeutique, qui est facultativement un agent immuno-inhibiteur, un agent immunostimulant, un agent anticancéreux, un agent anti-infectieux, un médicament à base d'acide nucléique, un agent anti-inflammatoire, un agent pour traiter des troubles cardiovasculaires, un inhibiteur de caspase, ou un agent bioactif,
(b) les une ou plusieurs fractions cargo comprennent un immunogène, qui est facultativement un antigène ou un acide nucléique codant pour un antigène, facultativement dans lequel l'antigène est un antigène allergique, un auto-antigène, ou un antigène tumoral, facultativement dans lequel l'antigène tumoral est un néoantigène ou un antigène commun ; ou
(c) les une ou plusieurs fractions cargo comprennent un agent de diagnostic, qui est facultativement un agent d'imagerie.

11. Composition pharmaceutique comprenant une quantité efficace des cargomères de la revendication 10(a) et un ou plusieurs supports, diluants et/ou excipients pharmaceutiquement acceptables, ou composition vaccinale comprenant une quantité efficace des cargomères de la revendication 10(b) et un ou plusieurs supports, diluants, excipients et/ou adjuvants pharmaceutiquement acceptables, ou composition de diagnostic comprenant une quantité efficace des cargomères de la revendication 10(c) et un ou plusieurs supports, diluants et/ou excipients appropriés pour une utilisation diagnostique.

12. Composition selon la revendication 11, dans laquelle au plus 20 %, au plus 10 %, au plus 5 % ou au plus 2 % des molécules amphipathiques sont sous forme non complexée, facultativement dans laquelle les cargomères dans la composition sont homogènes à au moins 75 %, au moins 85 %, au moins 90 %, au moins 95 % ou au moins 98 %.

13. Cargomère selon la revendication 10(a) ou la revendication 10(b) pour utilisation en tant que médicament, facultativement dans lequel le cargomère est sous forme de composition pharmaceutique comprenant un ou plusieurs supports, diluants et/ou excipients pharmaceutiquement acceptables.

14. Cargomère selon la revendication 10(c) pour utilisation *in vivo* en tant qu'agent de diagnostic, qui est facultativement sous forme de composition de diagnostic comprenant un ou plusieurs supports, diluants et/ou excipients appropriés pour une utilisation diagnostique.

15. Cargomère selon la revendication 10(a) ou la revendication 10(b) pour utilisation dans le traitement du cancer.
